# EUROPEAN PATENT SPECIFICATION

(11) **EP 4 422 572 B1**
(45) Date of publication and mention of the grant of the patent: **25.03.2026**
(21) Application number: 22886251.2
(22) Date of filing: 17.10.2022
(51) Int. Cl.: A61F 13/512, A61F 13/513

(54) **ABSORBENT WEB AND ARTICLE**
ABSORBIERENDE BAHN UND ARTIKEL
BANDE ABSORBANTE ET ARTICLE

(30) Priority: 29.10.2021 US 202163273207 P
(43) Date of publication of application: 04.09.2024
(73) Proprietor: Solventum Intellectual Properties Company, Maplewood, MN 55144 (US)
(72) Inventor: HOLM, David R., Saint Paul, Minnesota 55133-3427 (US); JACOBSON, Richard L., Saint Paul, Minnesota 55133-3427 (US)
(74) Representative: Simmons & Simmons
(86) International application number: PCT/IB2022/059955
(87) International publication number: WO 2023/073497

(56) References cited:
- WO-A1-03/071019
- WO-A1-2017/135022
- US-A- 5 873 868
- US-A1- 2010 004 615
- US-A1- 2016 129 626
- US-A1- 2020 188 183
- US-B1- 6 222 092
- US-B2- 10 039 673
- US-B2- 7 854 716
- US-B2- 7 854 716

## Description

### Technical Field

The present disclosure relates to an absorbent web, an absorbent article formed from the absorbent web, a wound dressing including the absorbent article, and a method of manufacturing the absorbent article from the absorbent web.

### Background

Generally, absorbent articles of various shapes and sizes are cut from an absorbent web. Such an absorbent article forms a part of a wound dressing to cover different types of skin traumas for preventing infections and promoting healing. The absorbent web typically includes a number of slits to improve a conformability and elongation of the absorbent article. The conformability and elongation of the absorbent article may be needed when the wound dressings are worn at a contoured sacrum and coccyx area, or at an articulating joint, such as, knee, elbow, and the like.

Further, long injuries, such as, surgical incisions, may be difficult to cover because they may not always be linear, and may often require multiple wound dressings, partly because wound dressings of a large size may not be easy to handle or easily available. Accordingly, non-linear skin traumas or long injuries may require multiple wound dressings because conventional wound dressings may not flex, stretch, or bend adequately during application. In addition, skin traumas that traverse non-planar surfaces, such as, long and/or non-linear skin traumas extending over bendable surfaces, may suffer from a lack of adequate wound dressing conformability. In addition to the use of multiple wound dressings, such applications may further require cutting and customizing of the wound dressings which may be time consuming, may increase complexity of application of the wound dressing, and may also result in poor coverage and sealing of the injury from contaminants.

In conventional absorbent webs, the slits do not include a particular orientation and a spatial arrangement to improve conformability and elongation of the wound dressing. Further, when the absorbent article is being cut from the conventional absorbent web, a closed perimeter of the absorbent article may align with one or more slits of the absorbent web. Specifically, the one or more slits may be disposed such that the one or more slits are tangential to the closed perimeter of the absorbent article that may cause debris formation in the absorbent article, may decrease conformability, and may reduce the elongation of the absorbent article, which are not desirable. US 7854716B2 relates to compression bandage systems, in particular for the use in the treatment and/or management of venous leg ulceration.

Therefore, there remains a need for absorbent articles that may be conformable, resilient, comfortable for a patient to wear, easy to apply, and may also exhibit high elongation for various types of skin traumas.

### Summary

Generally, the present disclosure relates to an absorbent web and an absorbent article cut from the absorbent web for use in a wound dressing. The present disclosure further relates to a method of manufacturing the absorbent article from the absorbent web.

In a first aspect, the present disclosure provides an absorbent web. The absorbent web includes a web body defining a machine direction along a length of the web body and a transverse direction orthogonal to the machine direction. The web body includes a first major surface and a second major surface opposing the first major surface. The web body also includes a first longitudinal edge extending along the machine direction and a second longitudinal edge opposing the first longitudinal edge, such that each of the first and second major surfaces is disposed between the first and second longitudinal edges. The first longitudinal edge and the second longitudinal edge define a total width of the web body therebetween along the transverse direction. The web body further includes a first region extending from the first longitudinal edge and having a first width along the transverse direction less than the total width of the web body, a second region spaced apart from the first region and extending from the second longitudinal edge and having a second width along the transverse direction less than the total width of the web body, and a third region disposed between the first and second regions and having a third width along the transverse direction less than the total width of the web body. The web body further includes a plurality of first slits extending from the first major surface to the second major surface and disposed in the first region of the web body. Each first slit is inclined from about 65 degrees to about 115 degrees relative to the machine direction. The web body also includes a plurality of second slits extending from the first major surface to the second major surface and disposed in the second region of the web body. Each second slit is inclined from about 65 degrees to about 115 degrees relative to the machine direction. The web body further includes a plurality of third slits extending from the first major surface to the second major surface and disposed in the third region of the web body. Further, at least some third slits from the plurality of third slits are substantially parallel to or inclined obliquely to the machine direction. Moreover, at least some third slits from the plurality of third slits are inclined to each first slit and each second slit.

In a second aspect, the present disclosure provides an absorbent article formed from a portion of the absorbent web of the first aspect. The absorbent article extends from the first major surface to the second major surface. The absorbent article includes a closed perimeter disposed along the third region and extending at least partially into each of the first and second regions. The closed perimeter intersects at least some first slits from the plurality of first slits at corresponding first intersection points, at least some second slits from the plurality of second slits at corresponding second intersection points, and at least some third slits from the plurality of third slits at corresponding third intersections points. Further, a tangent to the closed perimeter at each of the first, second, and third intersection points forms an angle of at least about four degrees with the corresponding first, second, or third slits.

In a third aspect, the present disclosure provides a wound dressing including the absorbent article of the second aspect.

In a fourth aspect, the present disclosure provides a method of manufacturing an absorbent article from the absorbent web of the first aspect. The method includes cutting through a portion of the absorbent web from the first major surface to the second major surface along a closed perimeter, such that the closed perimeter is disposed along the third region and extends at least partially into each of the first and second regions. The closed perimeter intersects at least some first slits from the plurality of first slits at corresponding first intersection points, at least some second slits from the plurality of second slits at corresponding second intersection points, and at least some third slits from the plurality of third slits at corresponding third intersections points. Further, a tangent to the closed perimeter at each of the first, second, and third intersection points forms an angle of at least about four degrees with the corresponding first, second, or third slits.

In a fifth aspect, the present disclosure provides an absorbent article. The absorbent article includes an article body. The article body includes a first major surface and a second major surface opposing the first major surface. The article body also includes a closed perimeter bounding the first and second major surfaces. The article body further includes a plurality of slits extending from the first major surface to the second major surface. At least some slits from the plurality of slits intersect the closed perimeter at corresponding intersection points. Further, a tangent to the closed perimeter at each intersection point forms an angle of at least about four degrees with the corresponding slit.

### Brief Description of the Drawings

Exemplary embodiments disclosed herein may be more completely understood in consideration of the following detailed description in connection with the following figures. The figures are not necessarily drawn to scale. Like numbers used in the figures refer to like components. However, it will be understood that the use of a number to refer to a component in a given figure is not intended to limit the component in another figure labeled with the same number.
FIG. 1 illustrates a schematic view of a wound dressing according to an embodiment of the present disclosure;
FIG. 2A illustrates a schematic view of an absorbent web according to a first embodiment of the present disclosure;
FIG. 2B illustrates a schematic view of an absorbent article formed from the first absorbent web of FIG. 2A according to an embodiment of the present disclosure;
FIG. 3 illustrates a schematic view of an absorbent web according to a second embodiment of the present disclosure;
FIG. 4 illustrates a schematic view of an absorbent web according to a third embodiment of the present disclosure;
FIG. 5 illustrates a schematic view of an absorbent web according to a fourth embodiment of the present disclosure;
FIG. 6 illustrates a schematic view of an absorbent web according to a fifth embodiment of the present disclosure;
FIG. 7A illustrates a schematic view of an absorbent web according to a sixth embodiment of the present disclosure;
FIGS. 7B, 7C, and 7D illustrate schematic view of various regions of the absorbent web of FIG. 7A according to the sixth embodiment of the present disclosure;
FIG. 8A illustrates a schematic view of an absorbent web according to a seventh embodiment of the present disclosure;
FIGS. 8B, 8C, and 8D illustrate schematic view of various regions of the absorbent web of FIG. 8A according to the seventh embodiment of the present disclosure;
FIG. 9 illustrates a plot representing elongation properties of the absorbent article with slits and an absorbent article without slits according to an embodiment of the present disclosure; and
FIG. 10 is a flowchart for a method of manufacturing the absorbent article from the absorbent web according to an embodiment of the present disclosure.

### Detailed Description

In the following description, reference is made to the accompanying figures that form a part thereof and in which various embodiments are shown by way of illustration. It is to be understood that other embodiments are contemplated and may be made without departing from the scope of the present disclosure. The following detailed description, therefore, is not to be taken in a limiting sense.

In the following disclosure, the following definitions are adopted.

As recited herein, all numbers should be considered modified by the term "about". As used herein, "a," "an," "the," "at least one," and "one or more" are used interchangeably.

As used herein as a modifier to a property or attribute, the term "generally" or "typically", unless otherwise specifically defined, means that the property or attribute would be readily recognizable by a person of ordinary skill but without requiring absolute precision or a perfect match (e.g., within +/- 20 % for quantifiable properties).

The term "substantially", unless otherwise specifically defined, means to a high degree of approximation (e.g., within +/- 10% for quantifiable properties) but again without requiring absolute precision or a perfect match.

The term "about", unless otherwise specifically defined, means to a high degree of approximation (e.g., within +/- 5% for quantifiable properties) but again without requiring absolute precision or a perfect match.

Terms, such as, same, equal, uniform, constant, strictly, and the like, are understood to be within the usual tolerances or measuring error applicable to the particular circumstance rather than requiring absolute precision or a perfect match.

As used herein, the terms "first" and "second" are used as identifiers. The terms "first" and "second" when used in conjunction with a feature or an element can be interchanged throughout the embodiments of this disclosure.

As used herein, when a first material is termed as "similar" to a second material, at least 90 weight % of the first and second materials are identical and any variation between the first and second materials comprises less than about 10 weight % of each of the first and second materials.

As used herein, "at least one of A and B" should be understood to mean "only A, only B, or both A and B".

As used herein, the terms "layer," "sheet," and "dressing," or variations thereof, are used to describe an article having a thickness that is smaller relative to a length and a width thereof.

As used herein, the term "polymer" refers to both materials prepared from one monomer, such as, a homopolymer or to materials prepared from two or more monomers, such as, a copolymer, terpolymer, or the like. Likewise, the term "polymerize" refers to the process of making a polymeric material that can be a homopolymer, copolymer, terpolymer, or the like. Furthermore, unless otherwise specified, the term "polymer" shall include all possible geometrical configurations of the material. These configurations include, but are not limited to, isotactic, syndiotactic, and random symmetries.

As used herein, the terms "nonwoven material", "nonwoven", or "nonwoven layer" are used in their normal sense and specifically, refers to a web that has a structure of individual fibers or threads which are interlaid, but not in any regular, repeating manner. Nonwoven materials, nonwovens, or nonwoven layers have been, in the past, formed by a variety of processes, such as, for example, melt blowing processes, spun bonding processes and bonded carded web processes.

As used herein, the terms "join", "joined", "joining", "bond", "bonded", "bonding", "attach", "attached", or "attaching" encompass configurations whereby an element is directly secured to another element by affixing the element directly to the other element, and configurations whereby an element is indirectly secured to another element by affixing the element to intermediate member(s) which in turn are affixed to the other element.

As used herein, the term "absorbent article", refers to devices which absorb and contain bodily exudates (e.g., urine or blood), and, more specifically, refers to devices which are placed against or in proximity to a body of the wearer to absorb and contain the various bodily exudates discharged from the body. Such absorbent articles may form a part of a wound dressing to absorb blood or other bodily secretions. The term absorbent article includes, but is not limited to, diapers, pants, training pants, adult incontinence products, sanitary napkins, tampons, wipes, and liners. The term "absorbent article" may also encompass cleaning or dusting pads or substrates that have some absorbency.

The term "machine direction" as used herein may refer to a primary movement direction of a material, a strip of material or substrate, or an article moving through a process.

It should be noted that each angle defined herein is measured from the machine direction in a counterclockwise sense.

The present disclosure relates to an absorbent web and an absorbent article formed from the absorbent web for use in a wound dressing. The present disclosure further relates to a method of manufacturing the absorbent article from the absorbent web. The absorbent web includes a web body defining a machine direction along a length of the web body and a transverse direction orthogonal to the machine direction. The web body includes a first major surface and a second major surface opposing the first major surface. The web body also includes a first longitudinal edge extending along the machine direction and a second longitudinal edge opposing the first longitudinal edge, such that each of the first and second major surfaces is disposed between the first and second longitudinal edges. The first longitudinal edge and the second longitudinal edge define a total width of the web body therebetween along the transverse direction. The web body further includes a first region extending from the first longitudinal edge and having a first width along the transverse direction less than the total width of the web body, a second region spaced apart from the first region and extending from the second longitudinal edge and having a second width along the transverse direction less than the total width of the web body, and a third region disposed between the first and second regions and having a third width along the transverse direction less than the total width of the web body. The web body further includes a plurality of first slits extending from the first major surface to the second major surface and disposed in the first region of the web body. Each first slit is inclined from about 65 degrees to about 115 degrees relative to the machine direction. The web body also includes a plurality of second slits extending from the first major surface to the second major surface and disposed in the second region of the web body. Each second slit is inclined from about 65 degrees to about 115 degrees relative to the machine direction. The web body further includes a plurality of third slits extending from the first major surface to the second major surface and disposed in the third region of the web body. At least some third slits from the plurality of third slits are substantially parallel to or inclined obliquely to the machine direction. At least some third slits from the plurality of third slits are inclined to each first slit and each second slit.

A conventional absorbent web typically includes a number of slits extending through the absorbent web. Such slits may not have an orientation and/or a spatial arrangement that improves conformability and elongation of an absorbent article that is cut from the conventional absorbent web. Further, when the absorbent article is being cut from the absorbent web, a closed perimeter of the absorbent article may align with one or more slits of the absorbent web. Specifically, the one or more slits are disposed such that the one or more slits are tangential to the closed perimeter of the absorbent article which may lead to debris formation in the absorbent article, may decrease conformability, and may reduce elongation of the absorbent article.

The wound dressing of the present disclosure includes the absorbent article cut from the absorbent web. The absorbent article may be conformable, resilient, and easy to use for various skin traumas including, but not limited to, long or non-linear types of skin traumas. Further, the wound dressings may be comfortable to wear and may exhibit improved elongation properties. Furthermore, the slits of the absorbent web may be oriented in such a way that, when the absorbent article is cut from the absorbent web, the closed perimeter of the absorbent article does not align with any of the slits. Specifically, the slits of the absorbent web are disposed such that none of the slits are tangential to the closed perimeter of the absorbent article which may eliminate debris formation in the absorbent article, may improve conformability, and may increase elongation of the absorbent article in all directions.

The wound dressing of the present disclosure may be used at a contoured sacrum and coccyx area, or at an articulating joint, such as, knee, elbow, and the like. The wound dressing may be used for long injuries, such as, surgical incisions or for non-linear skin traumas as the wound dressing can flex, stretch, or bend adequately during application. When used on skin traumas that traverse non-planar surfaces, such as, long and/or nonlinear skin traumas over bendable surfaces, the wound dressing including the improved absorbent article may not suffer from a lack of wound dressing conformability. Further, the wound dressing including the improved absorbent article may eliminate a requirement of multiple wound dressings or a customized wound dressing as the absorbent article may exhibit improved stretchability and elongation. Furthermore, the absorbent article may provide improved coverage and sealing of the injury from contaminants.

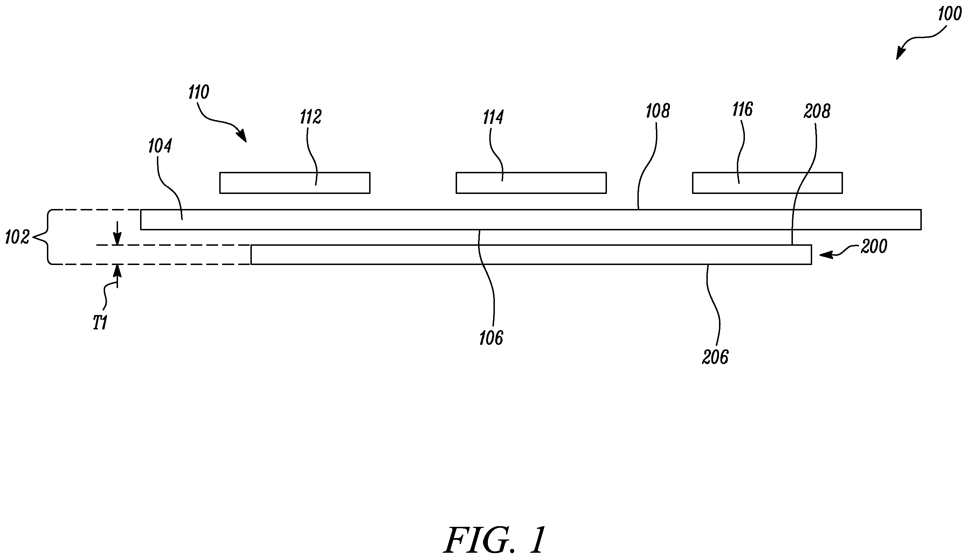

Referring now to Figures, FIG. 1 illustrates a schematic view of a wound dressing 100 including an absorbent article 200, according to an embodiment of the present disclosure. The wound dressing 100 may be disposed on a user (not shown) to treat various types of skin traumas (not shown) for preventing infections and promoting healing. The wound dressing 100 may be worn at a contoured sacrum and coccyx area, or at an articulating joint, such as, knee, elbow, and the like. Further, the wound dressing 100 may be used for long injuries, such as, surgical incisions which may be linear or non-linear. Moreover, the wound dressing 100 may be used to treat skin traumas that traverse non-planar surfaces, such as, long and/or non-linear skin traumas over bendable surfaces.

The wound dressing 100 includes an elongated composite 102. The elongated composite 102 includes a flexible substrate 104. The flexible substrate 104 may be made of nonwoven fibrous webs, woven fibrous webs, knits, films, and the like. In some embodiments, the flexible substrate 104 may include elastomeric polyurethane, co-polyester, and/or polyether block amide films. Such films may exhibit a combination of desirable properties in the flexible substrate 104, such as, conformability, high moisture vapor permeability, and/or transparency. The flexible substrate 104 defines a first surface 106 facing a skin of the user and a second surface 108 opposite to the first surface 106. The flexible substrate 104 may include a rectangular shape. Alternatively, the flexible substrate 104 may have any other shape.

The flexible substrate 104 may be coated with a pressure-sensitive adhesive (not shown) on the first surface 106 thereof. The pressure-sensitive adhesive may include one or more of an acrylate copolymer adhesive, a rubber-based polymer, such as, natural rubbers, synthetic rubbers, styrene block copolymers, and the like, a polyurethane polymer, and a silicone-based polymer. In some embodiments, the pressure-sensitive adhesive may include an acrylic adhesive which may include a copolymer of at least one C4-C12 alkyl (meth)acrylate, such as, isooctyl acrylate or 2-ethylhexylacrylate and at least one high Tg (e.g., polar) co-monomer, such as, (meth)acrylamide, N-vinyl pyrrolidone, polyethylene oxide acrylate, or a mixture thereof. In other embodiments, the acrylic adhesive may include at least 90 weight percentage (wt. %) C4-C12 alkyl (meth)acrylate(s). Suitable examples include a 90:10 isooctyl acrylate to acrylic acid copolymer, a 70:15:15 isooctyl acrylate to ethylene oxide acrylate to acrylic acid terpolymer, and/or a 25:69:6 2-ethylhexylacrylate to butyl acrylate to acrylic acid terpolymer.

The wound dressing 100 also includes a delivery system 110. The delivery system 110 may assist a caregiver to apply the wound dressing 100 to a user. The delivery system 110 may provide stability to the wound dressing 100 for proper application and may enable high conformability and resiliency of the dressing. The delivery system 110 may include a first support network 112, a second support network 114, and a third support network 116 releasably attached to the second surface 108 of the flexible substrate 104. The support networks 112, 114, 116 allow for coverage of long wounds, especially non-planar wounds that require dressing conformability and resiliency. The first, second, and third support networks 112, 114, 116 may include one or more materials selected from polyethylene/vinyl acetate copolymer, polyvinyl acetate-coated paper, polyester film, and polyethylene film. The first, second, and third support networks 112, 114, 116 may further include materials, such as, nonwoven materials, polymer films, and papers. One example of a support network material may include a polyethylene/vinyl acetate copolymer coated super calendared kraft paper. Other examples of suitable support network materials may include polyethylene/vinyl acetate copolymer coated polyester film, polyvinyl acetate coated polyester film, or polyethylene film. In some embodiments, the first, second, and third support networks 112, 114, 116 may further include nonwoven materials, polymer films, or papers attached to the aforementioned polymer films at distinct locations.

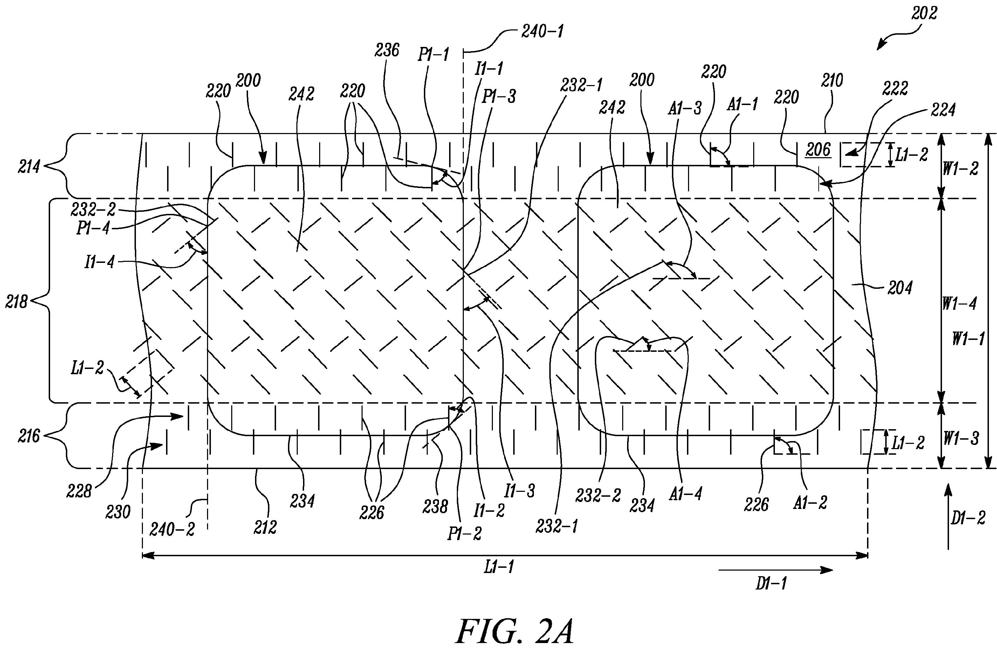

The wound dressing 100 further includes the absorbent article 200 disposed on the first surface 106 of the flexible substrate 104. The absorbent article 200 may be typically cut from an absorbent web 202 (shown in FIG. 2A). The absorbent article 200 defines a thickness "T1".

FIG. 2A illustrates a schematic view of the exemplary absorbent web 202. The absorbent web 202 may include one or more layers. Each layer may embody a layer of absorbent material. In some embodiments, the absorbent web 202 may be flexible. In some embodiments, the absorbent web 202 may be translucent or transparent, such that the absorbent article 200 formed from the absorbent web 202 may allow visual inspection of a wound without removal of the wound dressing 100. In some embodiments, the absorbent web 202 may include synthetic or natural materials including, but not limited to, woven or nonwoven materials (e.g., woven or nonwoven cotton or rayon), hydrocolloids (e.g., pectin, gelatin, carboxymethylcellulose (CMC), cross-linked carboxymethylcellulose (X-link CMC), cross-linked polyacrylic acid (PAA)), polymer gels (e.g., hydrogels), open cell foams, absorbent gels, nonwoven fibrous webs, woven fibrous webs, knits, films, collagens, hydro fibers, alginates, and/or combinations thereof. In some embodiments, the absorbent web 202 may include a polymeric fabric, a polymeric foam, and/or combinations thereof.

In some embodiments, the absorbent web 202 may include an absorbent hydrogel. The absorbent hydrogel may include a swellable, crosslinked poly (N-vinyl lactam), such as, crosslinked polyvinylpyrrolidone, a swelling agent, such as, poly glycerol-3, and an optional modifying polymer, such as, hydroxypropyl guar present in an amount sufficient to form a cohesive, absorbent hydrogel composition. The swellable poly (N-vinyl lactam) may be present in an amount from about 10 to about 50 wt. % of the composition. When the poly (N-vinyl lactam) is poly (N-vinyl pyrrolidone), poly (N-vinyl pyrrolidone) may be present in an amount from about 15 to about 45 wt. % of the composition. In some embodiments, the poly (N-vinyl pyrrolidone) may be present in an amount from about 18 to about 35 wt. % of the composition. In some embodiments, the amount of swelling agent may be present in an amount from about 50 to about 90 wt. % of the composition. In some embodiments, the optional modifying polymer may be present in an amount of less than about 5 wt. % of the composition. In some embodiments, the absorbent hydrogel may include less than about 25 wt. % water with respect to the composition.

In some embodiments, the absorbent web 202 may include one or more active agents. For example, the absorbent web 202 may include growth factors, antibacterial agents (e.g., penicillin, neomycin sulfate, sulfonamides, sulfadiazine, silver sulfadiazine, trimethoprim, povidone iodine, iodine, silver, silver chloride, chlorhexidine (e.g., chlorhexidine gluconate)), antifungal agents (e.g., griseofulvin, chlormidazole hydrochloride, clotrimazole, ketoconazole, miconazole, miconazole nitrate, nistatin, tolnaftate, or the like), disinfectants and antiseptics (e.g., benzalkonium chloride, cetalkonium chloride, chlorhexidine gluconate, ethanol, iodine, methyl benzethonium, povidone iodine, isopropanol, silver, silver oxide, silver salts, such as, silver lactate and silver chloride, triclosan, and the like), local anesthetics (e.g., tetracaine, benzocaine, prilocaine, procaine, and the like), debriding agents, antiinflammatory agents (e.g., indomethacin, ketoprofen, diclofenac, ibuprofen, and the like), astringents, enzymes, nutrients (e.g., vitamins, minerals, oxygen, or the like), and odor absorbing agents (e.g., zeolites, silicates, chitosans, cyclodextrins, and the like). In some embodiments, the absorbent web 202 may include chlorhexidine gluconate.

The absorbent web 202 includes a web body 204 defining a machine direction "D1-1" along a length "L1-1" of the web body 204 and a transverse direction "D1-2" orthogonal to the machine direction "D1-1". The length "L1-1" depicted herein includes a partial length of the web body 204. Further, the web body 204 defines the thickness "T1" (see FIG. 1). It should be noted that, as the absorbent article 200 is cut from the web body 204, the thickness "T1" is common for the absorbent article 200 and the web body 204. The web body 204 is embodied as a generally long, rectangular shaped strip. In some examples, the web body 204 may be wound to form a spool. The web body 204 includes a first major surface 206 and a second major surface 208 (shown in FIG. 1) opposing the first major surface 206. The first major surface 206 and the second major surface 208 have a substantially planar profile. The web body 204 also includes a first longitudinal edge 210 extending along the machine direction "D1-1" and a second longitudinal edge 212 opposing the first longitudinal edge 210, such that each of the first and second major surfaces 206, 208 is disposed between the first and second longitudinal edges 210, 212. The first longitudinal edge 210 and the second longitudinal edge 212 define a total width "W1-1" of the web body 204 therebetween along the transverse direction "D1-2".

The web body 204 further includes a first region 214 extending from the first longitudinal edge 210 and having a first width "W1-2" along the transverse direction "D1-2" less than the total width "W1-1" of the web body 204, a second region 216 spaced apart from the first region 214 and extending from the second longitudinal edge 212 and having a second width "W1-3" along the transverse direction "D1-2" less than the total width "W1-1" of the web body 204, and a third region 218 disposed between the first and second regions 214, 216 and having a third width "W1-4" along the transverse direction "D1-2" less than the total width "W1-1" of the web body 204. In the illustrated embodiment of FIG. 2A, each of the first, second, and third regions 214, 216, 218 is a substantially rectangular region of the web body 204 extending from the first major surface 206 to the second major surface 208. Further, in the illustrated embodiment of FIG. 2A, the first and second widths "W1-2", "W1-3" may be substantially equal to each other. In other embodiments, the first and second widths "W1-2", "W1-3" may be different from each other. Further, in the illustrated embodiment of FIG. 2A, the third width "W1-4" may be greater than each of the first and second widths "W1-2", "W1-3" by a factor of at least two. In other embodiments, the third width "W1-4" may be greater than each of the first and second widths "W1-2", "W1-3" by a factor of at least 1.25, 1.5, 2.5, 3, 3.5, 4, 5, and the like, without any limitations. In some embodiments, the third width "W1-4" may be equal to the first and/or second widths "W1-2", "W1-3". In other embodiments, the third width "W1-4" may be less than the first and/or second widths "W1-2", "W1-3".

The web body 204 includes a plurality of first slits 220 extending from the first major surface 206 to the second major surface 208 and disposed in the first region 214 of the web body 204. More particularly, the first slits 220 extend along the thickness "T1" of the web body 204. Therefore, each first slit 220 is a through slit. Each first slit 220 is inclined from about 65 degrees to about 115 degrees relative to the machine direction "D1-1". Specifically, a first angle "A1-1" may be defined between each first slit 220 and the machine direction "D1-1", such that the first angle "A1-1" may lie between 65 degrees and 115 degrees. In some embodiments, at least some of the first slits 220 may be orthogonal to the machine direction "D1-1". In the illustrated embodiment of FIG. 2A, the first angle "A1-1" may be substantially equal to 90 degrees, i.e., each first slit 220 may be orthogonal to the machine direction "D1-1". Further, in the illustrated embodiment of FIG. 2A, each first slit 220 may be substantially parallel to the transverse direction "D1-2". Moreover, the first slits 220 may be substantially parallel to each other. The first region 214 may define a first row of slits 222 including multiple first slits 220 and a second row of slits 224 including multiple first slits 220, such that each first slit 220 in the first row of slits 222 may be misaligned from each first slit 220 in the second row of slits 224. Specifically, the first row of slits 222 is offset from the second row of slits 224 relative to the machine direction "D1-1".

The web body 204 also includes a plurality of second slits 226 extending from the first major surface 206 to the second major surface 208 and disposed in the second region 216 of the web body 204. More particularly, the second slits 226 extend along the thickness "T1" of the web body 204. Therefore, each second slit 226 is a through slit. Each second slit 226 is inclined from about 65 degrees to about 115 degrees relative to the machine direction "D1-1". Specifically, a second angle "A1-2" may be defined between the each second slit 226 and the machine direction "D1-1", such that the second angle "A1-2" may lie between 65 degrees and 115 degrees. In some embodiments, at least some of the second slits 226 may be orthogonal to the machine direction "D1-1". In the illustrated embodiment of FIG. 2A, the second angle "A1-2" may be substantially equal to 90 degrees, i.e., each second slit 226 may be orthogonal to the machine direction "D1-1". Further, in the illustrated embodiment of FIG. 2A, each second slit 226 may be substantially parallel to the transverse direction "D1-2". Moreover, the second slits 226 may be substantially parallel to each other. The second region 216 may define a first row of slits 228 including multiple second slits 226 and a second row of slits 230 including multiple second slits 226, such that each second slit 226 in the first row of slits 228 may be misaligned from each second slit 226 in the second row of slits 230. Specifically, the first row of slits 228 is offset from the second row of slits 230 relative to the machine direction "D1-1".

The web body 204 further includes a plurality of third slits 232-1, 232-2 extending from the first major surface 206 to the second major surface 208 and disposed in the third region 218 of the web body 204. More particularly, the third slits 232-1, 232-2 extend along the thickness "T1" of the web body 204. Therefore, each third slit 232-1, 232-2 is a through slit. In the illustrated embodiment of FIG. 2A, the third region 218 includes the multiple third slits 232-1 and the multiple third slits 232-2. At least some third slits 232-1, 232-2 from the plurality of third slits 232-1, 232-2 are substantially parallel to or inclined obliquely to the machine direction "D1-1". In some embodiments, each third slit 232-1, 232-2 may be substantially parallel to or inclined obliquely to the machine direction "D1-1". In the illustrated embodiment of FIG. 2A, each third slit 232-1, 232-2 may be inclined obliquely to the machine direction "D1-1". Moreover, in the illustrated embodiment of FIG. 2A, each third slit 232-1, 232-2 may be inclined obliquely to the transverse direction "D1-2".

Further, in the illustrated embodiment of FIG. 2A, at least some third slits 232-1 from the plurality of third slits 232-1, 232-2 may be inclined from about 85 degrees to about 265 degrees relative to the machine direction "D1-1". Specifically, a third angle "A1-3" may be defined between each third slit 232-1 and the machine direction "D1-1", such that the third angle "A1-3" may lie between 85 degrees and 265 degrees. Further, at least some third slits 232-1 from the plurality of third slits 232-1, 232-2 may be inclined at an obtuse angle relative to the machine direction "D1-1". Specifically, the third slits 232-1 may be inclined relative to the machine direction "D1-1", such that the third angle "A1-3" may be an obtuse angle.

Moreover, in the illustrated embodiment of FIG. 2A, at least some third slits 232-2 from the plurality of third slits 232-1, 232-2 may be inclined from about 20 degrees to about 115 degrees relative to the machine direction "D1-1". Specifically, a fourth angle "A1-4" may be defined between the each third slit 232-2 and the machine direction "D1-1", such that the fourth angle "A1-4" may lie between 20 degrees and 115 degrees. Further, at least some third slits 232-2 from the plurality of third slits 232-1, 232-2 may be inclined at an acute angle relative to the machine direction "D1-1". Specifically, the third slits 232-2 may be inclined relative to the machine direction "D1-1", such that the fourth angle "A1-4" may be an acute angle. In other embodiments, at least some third slits 232-1, 232-2 from the plurality of third slits 232-1, 232-2 may be substantially parallel to the machine direction "D1-1". In some embodiments, at least some third slits 232-1, 232-2 from the plurality of third slits 232-1, 232-2 may be orthogonal to the machine direction "D1-1".

Further, the plurality of third slits 232-1 may be parallel to each other. Additionally, the plurality of third slits 232-2 may be parallel to each other. Furthermore, at least some third slits 232-1, 232-2 from the plurality of third slits 232-1, 232-2 may be substantially orthogonal to at least some other third slits 232-1, 232-2 from the plurality of third slits 232-1, 232-2. In the illustrated embodiment of FIG. 2A, each third slit 232-1 may be substantially orthogonal to each third slit 232-2. In other examples, at least some third slits 232-1, 232-2 from the plurality of third slits 232-1, 232-2 may be inclined obliquely to at least some other third slits 232-1, 232-2 from the plurality of third slits 232-1, 232-2. For example, each third slit 232-1 may be inclined obliquely to each third slit 232-2. Further, at least some third slits 232-1, 232-2 from the plurality of third slits 232-1, 232-2 are inclined to each first slit 220 and each second slit 226. In the illustrated embodiment of FIG. 2A, each third slit 232-1, 232-2 may be inclined to each first slit 220 and each second slit 226. In some embodiments, at least some other third slits 232-1, 232-2 from the plurality of third slits 232-1, 232-2 may be substantially parallel to each first slit 220 and each second slit 226.

The first slits 220, the second slits 226, and the third slits 232-1, 232-2 may be spatially arranged in at least three different directions. Further, the first slits 220, the second slits 226, and the third slits 232-1, 232-2 may include different angular orientations with respect to each other. The angular orientation may differ by 90 degrees or more. Each of the pluralities of first, second, and third slits 220, 226, 232-1, 232-2 may be substantially linear. In some embodiments, each of the pluralities of first, second, and third slits 220, 226, 232-1, 232-2 may include a non-linear profile. In some embodiments, each of the pluralities of first, second, and third slits 220, 226, 232-1, 232-2 may include a combination of a linear profile and a non-linear profile. Each of the plurality of first, second, and third slits 220, 226, 232-1, 232-2 may include a slit length "L1-2" from about 3 mm to about 25 mm. In some embodiments, the slit length "L1-2" of the first, second, and third slits 220, 226, 232-1, 232-2 may be similar to each other. Alternatively, the slit length "L1-2" of the first, second, and third slits 220, 226, 232-1, 232-2 may be different from each other, without any limitations.

Further, multiple absorbent articles 200 may be cut from the absorbent web 202. For exemplary purposes, only two absorbent articles 200 are illustrated herein. Furthermore, the absorbent article 200 extends from the first major surface 206 to the second major surface 208. Any type of machine may be used to cut the absorbent articles 200 from the absorbent web 202. For example, the machine may include a shearing machine, without limiting the scope of the present disclosure.

The absorbent article 200 includes a closed perimeter 234 disposed along the third region 218 and extending at least partially into each of the first and second regions 214, 216. The closed perimeter 234 intersects at least some first slits 220 from the plurality of first slits 220 at corresponding first intersection points "P1-1", at least some second slits 226 from the plurality of second slits 226 at corresponding second intersection points "P1-2", and at least some third slits 232-1, 232-2 from the plurality of third slits 232-1, 232-2 at corresponding third intersection points "P1-3", "P1-4". Therefore, the closed perimeter 234 may cut through at least some of the first slits 220, at least some of the second slits 226, and at least some of the third slits 232-1, 232-2, such that the at least some first slits 220, at least some second slits 226, and at least some third slits 232-1, 232-2 are at partially bounded by the closed perimeter 234. A tangent 236, 238, 240-1, 240-2 to the closed perimeter 234 at each of the first, second, and third intersection points "P1-1", "P1-2", "P1-3", "P1-4" forms an angle "I1-1", "I1-2", "I1-3", "I1-4" of at least about four degrees with the corresponding first, second, or third slits 220, 226, 232-1, 232-2. Specifically, the tangents 236, 238, 240-1, 240-2 may include a first tangent 236 at the first intersection point "P1-1", a second tangent 238 at the second intersection point "P1-2", and third tangents 240-1, 240-2 at the respective third intersection points "P1-3", "P1-4". The angles "I1-1", "I1-2", "I1-3", "I1-4" may include a first intersection angle "I1-1", a second intersection angle "I1-2", and third intersection angles "I1-3", "I1-4". Further, the first intersection angle "I1-1" may be defined between each first slit 220 and the first tangent 236, the second intersection angle "I1-2" may be defined between each second slit 226 and the second tangent 238, the third intersection angle "I1-3" may be defined between each third slit 232-1 and the third tangent 240-1, and the third intersection angle "I1-4" may be defined between each third slit 232-2 and the third tangent 240-2. Each of the first, second, and third intersection angles "I1-1", "I1-2", "I1-3", "I1-4" may be greater than four degrees.

In some embodiments, the first intersection angle "I1-1" is at least 10 degrees, at least 20 degrees, at least 30 degrees, or at least 45 degrees, or at least 60 degrees. In some embodiments, the second intersection angle "I1-2" is at least 10 degrees, at least 20 degrees, at least 30 degrees, at least 45 degrees, or at least 60 degrees. In some embodiments, the third intersection angle "I1-3" is at least 10 degrees, at least 20 degrees, at least 30 degrees, at least 45 degrees, or at least 60 degrees. In some embodiments, the third intersection angle "I1-4" is at least 10 degrees, at least 20 degrees, at least 30 degrees, at least 45 degrees, or at least 60 degrees.

Further, the first slits 220, the second slits 226, and the third slits 232-1, 232-2 may be disposed in such a way relative to the closed perimeter 234, such that none of the first, second, and third slits 220, 226, 232-1, 232-2 are tangential to or aligned with the closed perimeter 234 at the first intersection points "P1-1", the second intersection points "P1-2", and the third intersection points "P1-3", "P1-4", respectively. Furthermore, the shape of the closed perimeter 234 may be one of circular, rectangular with rounded corners, square with rounded corners, kidney-shape, and double lobe-shape. In the illustrated embodiment of FIG. 2A, the shape of the closed perimeter 234 of the absorbent article 200 is square with rounded corners.

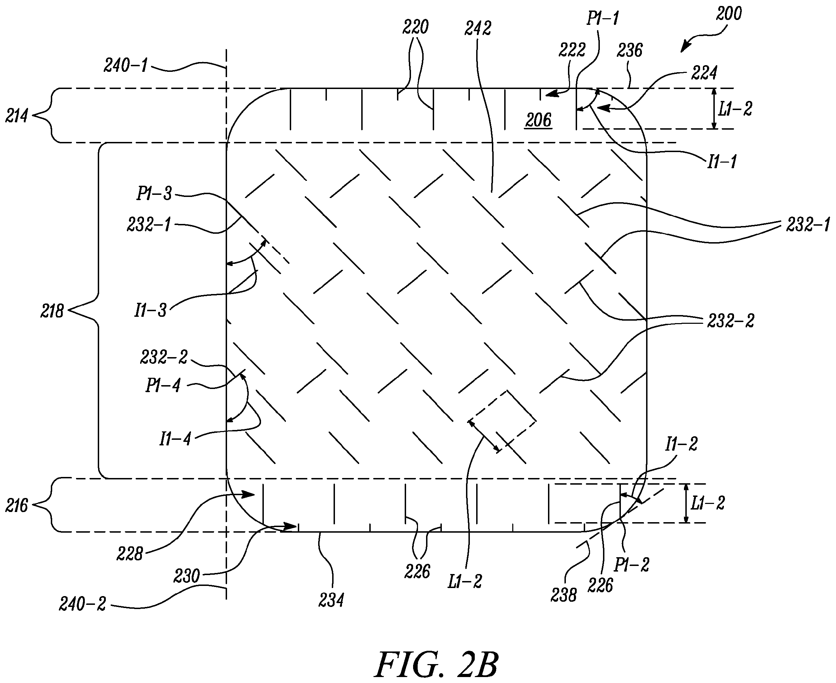

Referring to FIG. 2B, the single absorbent article 200 is illustrated. The absorbent article 200 is formed from a portion of the absorbent web 202 of FIG. 2A. Further, the absorbent article 200 may be placed on an adhesive film backing (not shown) or other layers during the construction of the wound dressing 100 (see FIG. 1). The absorbent article 200 includes an article body 242. The article body 242 includes the first major surface 206 and the second major surface 208 (see FIG. 1) opposing the first major surface 206. The article body 242 also includes the closed perimeter 234 bounding the first and second major surfaces 206, 208. The article body 242 further includes the plurality of slits 220, 226, 232-1, 232-2 extending from the first major surface 206 to the second major surface 208. At least some slits 220, 226, 232-1, 232-2 from the plurality of slits 220, 226, 232-1, 232-2 intersect the closed perimeter 234 at corresponding intersection points "P1-1", "P1-2", "P1-3", "P1-4". The tangent 236, 238, 240-1, 240-2 to the closed perimeter 234 at each intersection point "P1-1", "P1-2", "P1-3", "P1-4" forms the angle "I1-1", "I1-2", "I1-3", "I1-4" of at least about four degrees with the corresponding slit 220, 226, 232-1, 232-2.

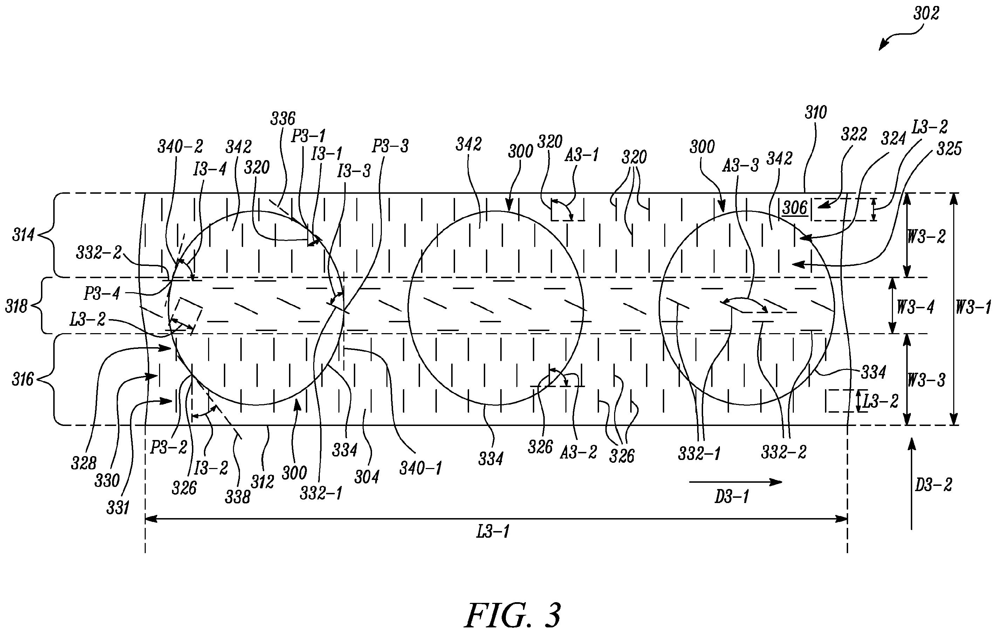

FIG. 3 illustrates an absorbent web 302, according to another embodiment of the present disclosure. The absorbent web 302 defines a web body 304, a machine direction "D3-1", a transverse direction "D3-2", and a length "L3-1" similar to the web body 204, the machine direction "D1-1", the transverse direction "D1-2", and the length "L1-1" of the absorbent web 202 of FIG. 2A. Further, the absorbent web 302 defines a first major surface 306, a second major surface (not shown), a first longitudinal edge 310, and a second longitudinal edge 312 similar to the first major surface 206, the second major surface 208, the first longitudinal edge 210, and the second longitudinal edge 212 of the absorbent web 202 of FIG. 2A, respectively. The first longitudinal edge 310 and the second longitudinal edge 312 define a total width "W3-1" of the web body 304 therebetween along the transverse direction "D3-2".

The absorbent web 302 includes a first region 314 extending from the first longitudinal edge 310 and having a first width "W3-2" along the transverse direction "D3-2" less than the total width "W3-1" of the web body 304, a second region 316 spaced apart from the first region 314 and extending from the second longitudinal edge 312 and having a second width "W3-3" along the transverse direction "D3-2" less than the total width "W3-1" of the web body 304, and a third region 318 disposed between the first and second regions 314, 316 and having a third width "W3-4" along the transverse direction "D3-2" less than the total width "W3-1" of the web body 304. In the illustrated embodiment of FIG. 3, each of the first, second, and third regions 314, 316, 318 is a substantially rectangular region of the web body 304 extending from the first major surface 306 to the second major surface. Further, in the illustrated embodiment of FIG. 3, the first and second widths "W3-2", "W3-3" may be substantially equal to each other. In other embodiments, the first and second widths "W3-2", "W3-3" may be different from each other. Moreover, in the illustrated embodiment of FIG. 3, the third width "W3-4" may be less than each of the first and second widths "W3-2", "W3-3". Alternatively, the third width "W3-4" may be greater than each of the first and second widths "W3-2", "W3-3", or the third width "W3-4" may be equal to the first and/or second widths "W3-2", "W3-3", without any limitations.

The web body 304 includes a plurality of first slits 320 extending from the first major surface 306 to the second major surface and disposed in the first region 314 of the web body 304. More particularly, the first slits 320 extend along a thickness (not shown) of the web body 304. Therefore, each first slit 320 is a through slit. Each first slit 320 is inclined from about 65 degrees to about 115 degrees relative to the machine direction "D3-1". Specifically, a first angle "A3-1" may be defined between the each first slit 320 and the machine direction "D3-1", such that the first angle "A3-1" may lie between 65 degrees and 115 degrees. In some embodiments, at least some of the first slits 320 may be orthogonal to the machine direction "D3-1". In the illustrated embodiment of FIG. 3, the first angle "A3-1" may be substantially equal to 90 degrees, i.e., each first slit 320 may be orthogonal to the machine direction "D3-1". In the illustrated embodiment of FIG. 3, each first slit 320 may be substantially parallel to the transverse direction "D3-2". Moreover, the first slits 320 may be substantially parallel to each other. The first region 314 may define a first row of slits 322 including multiple first slits 320, a second row of slits 324 including multiple first slits 320, and a third row of slits 325 including multiple first slits 320, such that each first slit 320 in the first and third rows of slits 322, 325 may be misaligned from each first slit 320 in the second row of slits 324, and each first slit 320 in the first row of slits 322 may be aligned with a corresponding first slit 320 in the third row of slits 325. Specifically, the first and second rows of slits 322, 325 is offset from the second row of slits 324 relative to the machine direction "D3-1".

The web body 304 also includes a plurality of second slits 326 extending from the first major surface 306 to the second major surface and disposed in the second region 316 of the web body 304. More particularly, the second slits 326 extend along the thickness of the web body 304. Therefore, each second slit 326 is a through slit. Each second slit 326 is inclined from about 65 degrees to about 115 degrees relative to the machine direction "D3-1". Specifically, a second angle "A3-2" may be defined between the each second slit 326 and the machine direction "D3-1", such that the second angle "A3-2" may lie between 65 degrees and 115 degrees. In some embodiments, at least some of the second slits 326 may be orthogonal to the machine direction "D3-1". In the illustrated embodiment of FIG. 3, the second angle "A3-2" may be substantially equal to 90 degrees, i.e., each second slit 326 may be orthogonal to the machine direction "D3-1". Further, in the illustrated embodiment of FIG. 3, each second slit 326 may be substantially parallel to the transverse direction "D3-2". Moreover, the second slits 326 may be substantially parallel to each other. Moreover, the second region 316 may define a first row of slits 328 including multiple second slits 326, a second row of slits 330 including multiple second slits 326, and a third row of slits 331 including multiple second slits 326, such that each second slit 326 in the first and third rows of slits 331 may be misaligned from each second slit 326 in the second row of slits 330, and each second slit 326 in the first row of slits 328 may align with a corresponding second slit 326 in the third row of slits 331. Specifically, the first and third rows of slits 328, 331 is offset from the second row of slits 330 relative to the machine direction "D3-1".

The web body 304 further includes a plurality of third slits 332-1, 332-2 extending from the first major surface 306 to the second major surface and disposed in the third region 318 of the web body 304. More particularly, the third slits 332-1, 332-2 extend along the thickness of the web body 304. Therefore, each third slit 332-1, 332-2 is a through slit. In the illustrated embodiment of FIG. 3, the third region 318 includes the multiple third slits 332-1 and the multiple third slits 332-2. At least some third slits 332-1, 332-2 from the plurality of third slits 332-1, 332-2 are substantially parallel to or inclined obliquely to the machine direction "D3-1". In some embodiments, each third slit 332-1, 332-2 may be substantially parallel to or inclined obliquely to the machine direction "D3-1". Specifically, in the illustrated embodiment of FIG. 3, at least some third slits 332-1 may be inclined obliquely to the machine direction "D3-1". In the illustrated embodiment of FIG. 3, the third slits 332-1 may also be inclined obliquely to the transverse direction "D3-2". Moreover, in the illustrated embodiment of FIG. 3, at least some third slits 332-1 from the plurality of third slits 332-1, 332-2 may be inclined from about 85 degrees to about 265 degrees relative to the machine direction "D3-1". Specifically, a third angle "A3-3" may be defined between each third slit 332-1 and the machine direction "D3-1", such that the third angle "A3-3" may lie between 85 degrees and 265 degrees. Further, at least some third slits 332-1 from the plurality of third slits 332-1, 332-2 may be inclined at an obtuse angle relative to the machine direction "D3-1". In the illustrated embodiment of FIG. 3, the third slits 332-1 may be inclined relative to the machine direction "D3-1", such that the third angle "A3-3" may be an obtuse angle. Alternatively, at least some third slits 332-1, 332-2 from the plurality of third slits 332-1, 332-2 may be inclined at an acute angle relative to the machine direction "D3-1". For example, some of the third slits 332-1, 332-2 may be inclined relative to the machine direction "D3-1", such that the third angle "A3-3" may be an acute angle. Further, in the illustrated embodiment of FIG. 3, at least some third slits 332-2 may be parallel to the machine direction "D3-1". Moreover, the third slits 332-2 may be orthogonal to the transverse direction "D3-2".

Further, the plurality of third slits 332-1 may be parallel to each other and the plurality of third slits 332-2 may be parallel to each other. Moreover, at least some third slits 332-1, 332-2 from the plurality of third slits 332-1, 332-2 may be inclined obliquely to at least some other third slits 332-1, 332-2 from the plurality of third slits 332-1, 332-2. In the illustrated embodiment of FIG. 3, each third slit 332-1 may be inclined obliquely to each third slit 332-2. In other embodiments, at least some third slits 332-1, 332-2 from the plurality of third slits 332-1, 332-2 may be substantially orthogonal to at least some other third slits 332-1, 332-2 from the plurality of third slits 332-1, 332-2. For example, the third slits 332-1 may be substantially orthogonal to the third slits 332-2.

Further, at least some third slits 332-1, 332-2 from the plurality of third slits 332-1, 332-2 are inclined to each first slit 320 and each second slit 326. In the illustrated embodiment of FIG. 3, each third slit 332-1, 332-2 may be inclined to each first slit 320 and each second slit 326. In some embodiments, at least some other third slits 332-1, 332-2 from the plurality of third slits 332-1, 332-2 may be substantially parallel to each first slit 320 and each second slit 326. In the illustrated embodiment of FIG. 3, each third slit 332-2 may be substantially orthogonal to each first slit 320 and each second slit 326.

The first slits 320, the second slits 326, and the third slits 332-1, 332-2 may be spatially arranged in at least three different directions. Further, the first slits 320, the second slits 326, and the third slits 332-1, 332-2 may include different angular orientations with respect to each other. The angular orientation may differ by 90 degrees or more. Each of the pluralities of first, second, and third slits 320, 326, 332-1, 332-2 may be substantially linear. In some embodiments, each of the pluralities of first, second, and third slits 320, 326, 332-1, 332-2 may include a non-linear profile. In some embodiments, each of the pluralities of first, second, and third slits 320, 326, 332-1, 332-2 may include a combination of a linear profile and a non-linear profile. Each of the plurality of first, second, and third slits 320, 326, 332-1, 332-2 may include a slit length "L3-2" from about 3 mm to about 25 mm. In some embodiments, the slit length "L3-2" of the first, second, and third slits 320, 326, 332-1, 332-2 may be similar to each other. Alternatively, the slit length "L3-2" of the first, second, and third slits 320, 326, 332-1, 332-2 may be different from each other, without any limitations.

Further, multiple absorbent articles 300 may be cut from the absorbent web 302. For exemplary purposes, three absorbent articles 300 are illustrated herein. The absorbent articles 300 include a closed perimeter 334 disposed along the third region 318 and extending at least partially into each of the first and second regions 314, 316. The closed perimeter 334 intersects at least some first slits 320 from the plurality of first slits 320 at corresponding first intersection points "P3-1", at least some second slits 326 from the plurality of second slits 326 at corresponding second intersection points "P3-2", and at least some third slits 332-1, 332-2 from the plurality of third slits 332-1, 332-2 at corresponding third intersection points "P3-3", "P3-4". Therefore, the closed perimeter 334 may cut through at least some of the first slits 320, at least some of the second slits 326, and at least some of the third slits 332-1, 332-2, such that the at least some first slits 320, at least some second slits 326, and at least some third slits 332-1, 332-2 are at partially bounded by the closed perimeter 334. A tangent 336, 338, 340-1, 340-2 to the closed perimeter 334 at each of the first, second, and third intersection points "P3-1", "P3-2", "P3-3", "P3-4" forms an angle "I3-1", "I3-2", "I3-3", "I3-4" of at least about four degrees with the corresponding first, second, or third slits 320, 326, 332-1, 332-2. Specifically, the tangents 336, 338, 340-1, 340-2 may include a first tangent 336 at the first intersection point "P3-1", a second tangent 338 at the second intersection point "P3-2", and third tangents 340-1, 340-2 at the respective third intersection points "P3-3", "P3-4". The angles "I3-1", "I3-2", "I3-3", "I3-4" may include a first intersection angle "I3-1", a second intersection angle "I3-2", and third intersection angles "I3-3", "I3-4". Further, the first intersection angle "I3-1" may be defined between each first slit 320 and the first tangent 336, the second intersection angle "I3-2" may be defined between each second slit 326 and the second tangent 338, the third intersection angle "I3-3" may be defined between each third slit 332-1 and the third tangent 340-1, and the third intersection angle "I3-4" may be defined between each third slit 332-2 and the third tangent 340-2. Each of the first, second, and third intersection angles "I3-1", "I3-2", "I3-3", "I3-4" may be greater than four degrees.

In some embodiments, the first intersection angle "I3-1" is at least 10 degrees, at least 20 degrees, at least 30 degrees, or at least 45 degrees, or at least 60 degrees. In some embodiments, the second intersection angle "I3-2" is at least 10 degrees, at least 20 degrees, at least 30 degrees, at least 45 degrees, or at least 60 degrees. In some embodiments, the third intersection angle "I3-3" is at least 10 degrees, at least 20 degrees, at least 30 degrees, at least 45 degrees, or at least 60 degrees. In some embodiments, the third intersection angle "I3-4" is at least 10 degrees, at least 20 degrees, at least 30 degrees, at least 45 degrees, or at least 60 degrees.

Further, the first slits 320, the second slits 326, and the third slits 332-1, 332-2 may be disposed in such a way relative to the closed perimeter 334, such that none of the first, second, and third slits 320, 326, 332-1, 332-2 are tangential to or aligned with the closed perimeter 334 at the first intersection points "P3-1", the second intersection points "P3-2", and the third intersection points "P3-3", "P3-4", respectively. In the illustrated embodiment of FIG. 3, the shape of the closed perimeter 334 of the absorbent articles 300 is circular. The absorbent articles 300 are formed from a portion of the absorbent web 302. The absorbent articles 300 includes an article body 342. The article body 342 includes the first major surface 306 and the second major surface (not shown) opposing the first major surface 306. The article body 342 also includes the closed perimeter 334 bounding the first major surface 306 and the second major surface. The article body 342 further includes the plurality of slits 320, 326, 332-1, 332-2 extending from the first major surface 306 to the second major surface. At least some slits 320, 326, 332-1, 332-2 from the plurality of slits 320, 326, 332-1, 332-2 intersect the closed perimeter 334 at corresponding intersection points "P3-1", "P3-2", "P3-3", "P3-4". The tangent 336, 338, 340-1, 340-2 to the closed perimeter 334 at each intersection point "P3-1", "P3-2", "P3-3", "P3-4" forms the angle "I3-1", "I3-2", "I3-3", "I3-4" of at least about four degrees with the corresponding slit 320, 326, 332-1, 332-2.

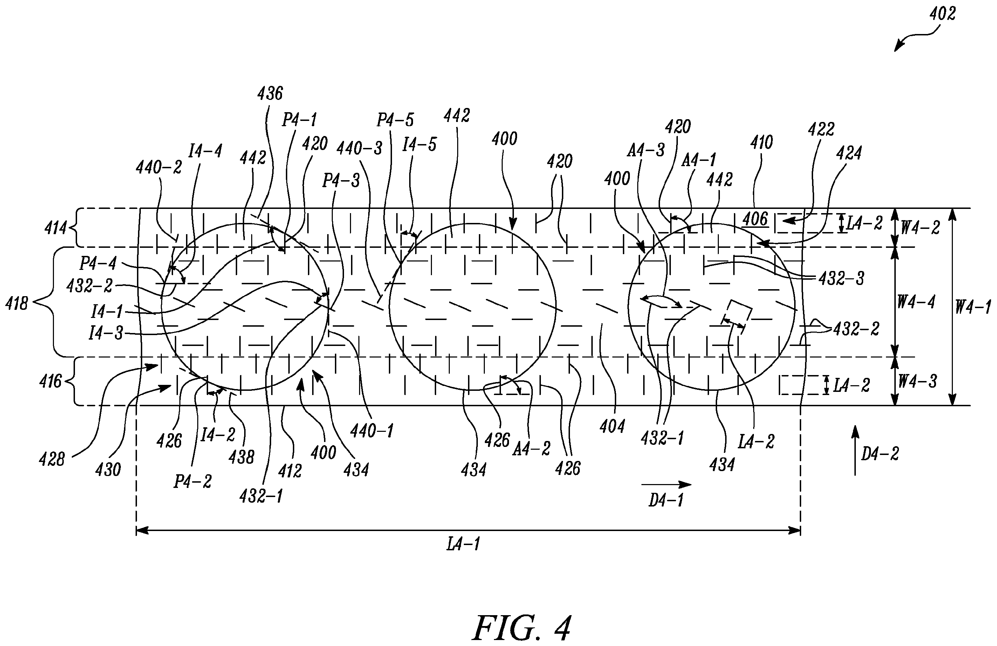

FIG. 4 illustrates an absorbent web 402, according to another embodiment of the present disclosure. The absorbent web 402 defines a web body 404, a machine direction "D4-1", a transverse direction "D4-2", and a length "L4-1" similar to the web body 204, the machine direction "D1-1", the transverse direction "D1-2", and the length "L1-1" of the absorbent web 402 of FIG. 2A, respectively. Further, the absorbent web 402 defines a first major surface 406, a second major surface (not shown), a first longitudinal edge 410, and a second longitudinal edge 412 similar to the first major surface 206, the second major surface 208, the first longitudinal edge 210, and the second longitudinal edge 212 of the absorbent web 202 of FIG. 2A, respectively. The first longitudinal edge 410 and the second longitudinal edge 412 define a total width "W4-1" of the web body 404 therebetween along the transverse direction "D4-2".

The absorbent web 402 includes a first region 414 extending from the first longitudinal edge 410 and having a first width "W4-2" along the transverse direction "D4-2" less than the total width "W4-1" of the web body 404, a second region 416 spaced apart from the first region 414 and extending from the second longitudinal edge 412 and having a second width "W4-3" along the transverse direction "D4-2" less than the total width "W4-1" of the web body 404, and a third region 418 disposed between the first and second regions 414, 416 and having a third width "W4-4" along the transverse direction "D4-2" less than the total width "W4-1" of the web body 404. In the illustrated embodiment of FIG. 4, each of the first, second, and third regions 414, 416, 418 is a substantially rectangular region of the web body 404 extending from the first major surface 406 to the second major surface. Further, in the illustrated embodiment of FIG. 4, the first and second widths "W4-2", "W4-3" may be substantially equal to each other. In other embodiments, the first and second widths "W4-2", "W4-3" may be different from each other. Moreover, in the illustrated embodiment of FIG. 4, the third width "W4-4" may be greater than each of the first and second widths "W4-2", "W4-3". Further, in the illustrated embodiment of FIG. 4, the third width "W4-4" may be greater than each of the first and second widths "W4-2", "W4-3" by a factor of at least two. In other embodiments, the third width "W4-4" may be greater than each of the first and second widths "W4-2", "W4-3" by a factor of at least 1.25, 1.5, 2.5, 3, 3.5, 4, 5, and the like, without any limitations. Alternatively, the third width "W4-4" may be lesser than each of the first and second widths "W4-2", "W4-3". In another embodiment, the third width "W4-4" may be equal to the first and/or second widths "W4-2", "W4-3".

The web body 404 includes a plurality of first slits 420 extending from the first major surface 406 to the second major surface and disposed in the first region 414 of the web body 404. More particularly, the first slits 420 extend along a thickness (not shown) of the web body 404. Therefore, each first slit 420 is a through slit. Each first slit 420 is inclined from about 65 degrees to about 115 degrees relative to the machine direction "D4-1". Specifically, a first angle "A4-1" may be defined between each first slit 420 and the machine direction "D4-1", such that the first angle "A4-1" may lie between 65 degrees and 115 degrees. Further, in some embodiments, at least some of the first slits 420 may be orthogonal to the machine direction "D4-1". In the illustrated embodiment of FIG. 4, the first angle "A4-1" may be substantially equal to 90 degrees, i.e., each first slit 420 may be orthogonal to the machine direction "D4-1". Further, in the illustrated embodiment of FIG. 4, each first slit 420 may be substantially parallel to the transverse direction "D4-2". Moreover, the first slits 420 may be substantially parallel to each other. The first region 414 may define a first row of slits 422 including multiple first slits 420 and a second row of slits 424 including multiple first slits 420, such that each first slit 420 in the first row of slits 422 may be misaligned from each first slit 420 in the second row of slits 424. Specifically, the first row of slits 422 is offset from the second row of slits 424 relative to the machine direction "D4-1".

The web body 404 also includes a plurality of second slits 426 extending from the first major surface 406 to the second major surface and disposed in the second region 416 of the web body 404. More particularly, the second slits 426 extend along the thickness of the web body 404. Therefore, each second slit 426 is a through slit. Each second slit 426 is inclined from about 65 degrees to about 115 degrees relative to the machine direction "D4-1". Specifically, a second angle "A4-2" may be defined between the each second slit 426 and the machine direction "D4-1", such that the second angle "A4-2" may lie between 65 degrees and 115 degrees. Further, in some embodiments, at least some of the second slits 426 may be orthogonal to the machine direction "D4-1". In the illustrated embodiment of FIG. 4, the second angle "A4-2" may be substantially equal to 90 degrees, i.e., each second slit 426 may be orthogonal to the machine direction "D4-1". Further, in the illustrated embodiment of FIG. 4, each second slit 426 may be substantially parallel to the transverse direction "D4-2". Moreover, the second slits 426 may be substantially parallel to each other. The second region 416 may define a first row of slits 428 including multiple second slits 426 and a second row of slits 430 including multiple second slits 426, such that each second slit 426 in the first row of slits 428 may be misaligned from each second slit 426 in the second row of slits 430. Specifically, the first row of slits 428 is offset from the second row of slits 430 relative to the machine direction "D4-1".

The web body 404 further includes a plurality of third slits 432-1, 432-2, 432-3 extending from the first major surface 406 to the second major surface and disposed in the third region 418 of the web body 404. More particularly, the third slits 432-1, 432-2, 432-3 extend along the thickness of the web body 404. Therefore, each third slit 432-1, 432-2, 432-3 is a through slit. In the illustrated embodiment of FIG. 4, the third region 418 includes the multiple third slits 432-1, the multiple third slits 432-2, and the multiple third slits 432-3. At least some third slits 432-1, 432-2, 432-3 from the plurality of third slits 432-1, 432-2, 432-3 are substantially parallel to or inclined obliquely to the machine direction "D4-1". In some embodiments, each third slit 432-1, 432-2, 432-3 may be substantially parallel to or inclined obliquely to the machine direction "D4-1". Specifically, in the illustrated embodiment of FIG. 4, at least some third slits 432-1 may be inclined obliquely to the machine direction "D4-1". In the illustrated embodiment of FIG. 4, at least some third slits 432-1 may be inclined obliquely to the transverse direction "D4-2".

Further, in the illustrated embodiment of FIG. 4, at least some third slits 432-1, 432-3 from the plurality of third slits 432-1, 432-2, 432-3 may be inclined from about 85 degrees to about 265 degrees relative to the machine direction "D4-1". Specifically, a third angle "A4-3" may be defined between each third slit 432-1 and the machine direction "D4-1", such that the third angle "A4-3" may lie between 85 degrees and 265 degrees. Further, at least some third slits 432-1 from the plurality of third slits 432-1, 432-2, 432-3 may be inclined at an obtuse angle relative to the machine direction "D4-1". Specifically, the third slits 432-1 may be inclined relative to the machine direction "D4-1", such that the third angle "A4-3" may be an obtuse angle. In other embodiments, at least some third slits 432-1, 432-2, 432-3 from the plurality of third slits 432-1, 432-2, 432-3 may be inclined at an acute angle relative to the machine direction "D4-1". For example, some of the third slits 432-1, 432-2, 432-3 may be inclined relative to the machine direction "D4-1", such that the third angle "A4-3" may be an acute angle.

Moreover, in some embodiments, at least some third slits 432-2 from the plurality of third slits 432-1, 432-2, 432-3 may be substantially parallel to the machine direction "D4-1". In the illustrated embodiment of FIG. 4, at least some third slits 432-2 may be orthogonal to the transverse direction "D4-2". In some embodiments, at least some third slits 432-3 from the plurality of third slits 432-1, 432-2, 432-3 may be orthogonal to the machine direction "D4-1". Specifically, the third slits 432-3 may be orthogonal to the machine direction "D4-1". Further, the third slits 432-3 may be parallel to the transverse direction "D4-2".

The plurality of third slits 432-1 may be parallel to each other, the plurality of third slits 432-2 may be parallel to each other, and the plurality of third slits 432-3 may be parallel to each other. Further, at least some third slits 432-1, 432-2, 432-3 from the plurality of third slits 432-1, 432-2, 432-3 may be inclined obliquely to at least some other third slits 432-1, 432-2, 432-3 from the plurality of third slits 432-1, 432-2, 432-3. In the illustrated embodiment of FIG. 4, each third slit 432-1 may be inclined obliquely to each third slit 432-2, 432-3. Further, at least some third slits 432-2 from the plurality of third slits 432-1, 432-2, 432-3 may be substantially orthogonal to at least some other third slits 432-3 from the plurality of third slits 432-1, 432-2, 432-3. Moreover, at least some third slits 432-2 may be disposed between adjacent third slits 432-3.

Further, at least some third slits 432-1, 432-2 from the plurality of third slits 432-1, 432-2, 432-3 are inclined to each first slit 420 and each second slit 426. In the illustrated embodiment of FIG. 4, each third slit 432-1, 432-2 may be inclined to each first slit 420 and each second slit 426. Further, at least some other third slits 432-2 from the plurality of third slits 432-1, 432-2, 432-3 may be substantially orthogonal to each first slit 420 and each second slit 426. In the illustrated embodiment of FIG. 4, each third slit 432-2 may be substantially orthogonal to each first slit 420 and each second slit 426. Further, at least some other third slits 432-3 from the plurality of third slits 432-1, 432-2, 432-3 may be substantially parallel to each first slit 420 and each second slit 426. In the illustrated embodiment of FIG. 4, each third slit 432-3 may be substantially parallel to each first slit 420 and each second slit 426.

The first slits 420, the second slits 426, and the third slits 432-1, 432-2, 432-3 may be spatially arranged in at least three different directions. Further, the first slits 420, the second slits 426, and the third slits 432-1, 432-2, 432-3 may include different angular orientations with respect to each other. The angular orientation may differ by 90 degrees or more. Each of the pluralities of first, second, and third slits 420, 426, 432-1, 432-2, 432-3 may be substantially linear. In some embodiments, each of the pluralities of first, second, and third slits 420, 426, 432-1, 432-2, 432-3 may include a non-linear profile. In some embodiments, each of the pluralities of first, second, and third slits 420, 426, 432-1, 432-2, 432-3 may include a combination of a linear profile and a non-linear profile. Each of the plurality of first, second, and third slits 420, 426, 432-1, 432-2, 432-3 may include a slit length "L4-2" from about 3 mm to about 25 mm. In some embodiments, the slit length "L4-2" of the first, second, and third slits 420, 426, 432-1, 432-2, 432-3 may be similar to each other. Alternatively, the slit length "L4-2" of the first, second, and third slits 420, 426, 432-1, 432-2, 432-3 may be different from each other, without any limitations.

Further, multiple absorbent articles 400 may be cut from the absorbent web 402. For exemplary purposes, only three absorbent articles 400 are illustrated herein. The absorbent article 400 includes a closed perimeter 434 disposed along the third region 418 and extending at least partially into each of the first and second regions 414, 416. The closed perimeter 434 intersects at least some first slits 420 from the plurality of first slits 420 at corresponding first intersection points "P4-1", at least some second slits 426 from the plurality of second slits 426 at corresponding second intersection points "P4-2", and at least some third slits 432-1, 432-2, 432-3 from the plurality of third slits 432-1, 432-2, 432-3 at corresponding third intersection points "P4-3", "P4-4", "P4-5". Therefore, the closed perimeter 434 may cut through at least some of the first slits 420, at least some of the second slits 426, and at least some of the third slits 432-1, 432-2, 432-3 such that the at least some first slits 420, at least some second slits 426, and at least some third slits 432-1, 432-2 are at partially bounded by the closed perimeter 434. A tangent 436, 438, 440-1, 440-2, 440-3 to the closed perimeter 434 at each of the first, second, and third intersection points "P4-1", "P4-2", "P4-3", "P4-4", "P4-5" forms an angle "I4-1", "I4-2", "I4-3", "I4-4", "I4-5" of at least about four degrees with the corresponding first, second, or third slits 420, 426, 432-1, 432-2, 432-3. Specifically, the tangents 436, 438, 440-1, 440-2, 440-3 may include a first tangent 436 at the first intersection point "P4-1", a second tangent 438 at the second intersection point "P4-2", and third tangents 440-1, 440-2, 440-3 at the respective third intersection points "P4-3", "P4-4", "P4-5". The angles "I4-1", "I4-2", "I4-3", "I4-4", "I4-5" may include a first intersection angle "I4-1", a second intersection angle "I4-2", and third intersection angles "I4-3", "I4-4", "I4-5". Further, the first intersection angle "I4-1" may be defined between each first slit 420 and the first tangent 436, the second intersection angle "I4-2" may be defined between each second slit 426 and the second tangent 438, the third intersection angle "I4-3" may be defined between each third slit 432-1 and the third tangent 440-1, the third intersection angle "I4-4" may be defined between each third slit 432-2 and the third tangent 440-2, and the third intersection angle "I4-5" may be defined between each third slit 432-3 and the third tangent 440-3. Each of the first, second, and third intersection angles "I4-1", "I4-2", "I4-3", "I4-4", "I4-5" may be greater than four degrees.

In some embodiments, the first intersection angle "I4-1" is at least 10 degrees, at least 20 degrees, at least 30 degrees, or at least 45 degrees, or at least 60 degrees. In some embodiments, the second intersection angle "I4-2" is at least 10 degrees, at least 20 degrees, at least 30 degrees, at least 45 degrees, or at least 60 degrees. In some embodiments, the third intersection angle "I4-3" is at least 10 degrees, at least 20 degrees, at least 30 degrees, at least 45 degrees, or at least 60 degrees. In some embodiments, the third intersection angle "I4-4" is at least 10 degrees, at least 20 degrees, at least 30 degrees, at least 45 degrees, or at least 60 degrees. In some embodiments, the third intersection angle "I4-5" is at least 10 degrees, at least 20 degrees, at least 30 degrees, at least 45 degrees, or at least 60 degrees.

Further, the first slits 420, the second slits 426, and the third slits 432-1, 432-2, 432-3 may be disposed in such a way relative to the closed perimeter 434, such that none of the first, second, and third slits 420, 426, 432-1, 432-2, 432-3 are tangential to or aligned with the closed perimeter 434 at the first intersection points "P4-1", the second intersection points "P4-2", and the third intersection points "P4-3", "P4-4", "P4-5", respectively. In the illustrated embodiment of FIG. 4, the shape of the closed perimeter 434 of the absorbent article 400 is circular. The absorbent article 400 is formed from a portion of the absorbent web 402. The absorbent article 400 includes an article body 442. The article body 442 includes the first major surface 406 and the second major surface (not shown) opposing the first major surface 406. The article body 442 also includes the closed perimeter 434 bounding the first major surface 406 and the second major surface. The article body 442 further includes the plurality of slits 420, 426, 432-1, 432-2, 432-3 extending from the first major surface 406 to the second major surface. At least some slits 420, 426, 432-1, 432-2, 432-3 from the plurality of slits 420, 426, 432-1, 432-2, 432-3 intersect the closed perimeter 434 at corresponding intersection points "P4-1", "P4-2", "P4-3", "P4-4", "P4-5". The tangent 436, 438, 440-1, 440-2, 440-3 to the closed perimeter 434 at each intersection point "P4-1", "P4-2", "P4-3", "P4-4", "P4-5" forms the angle "I4-1", "I4-2", "I4-3", "I4-4", "I4-5" of at least about four degrees with the corresponding slit 420, 426, 432-1, 432-2, 432-3.

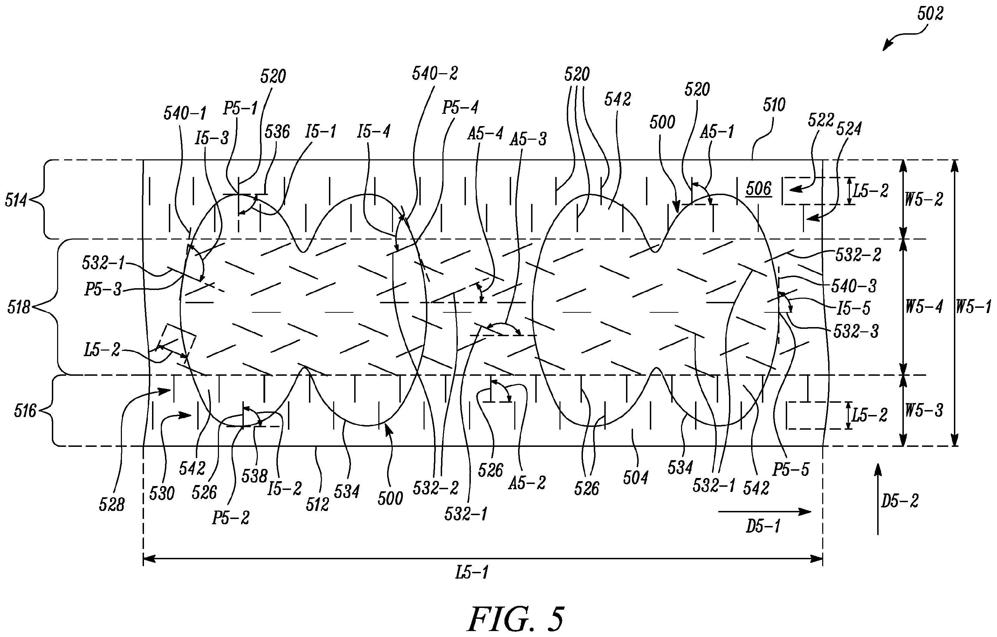

FIG. 5 illustrates an absorbent web 502, according to another embodiment of the present disclosure. The absorbent web 502 defines a web body 504, a machine direction "D5-1", a transverse direction "D5-2", and a length "L5-1" similar to the web body 204, the machine direction "D1-1", the transverse direction "D1-2", and the length "L1-1" of the absorbent web 202 of FIG. 2A, respectively. Further, the absorbent web 502 defines a first major surface 506, a second major surface (not shown), a first longitudinal edge 510, and a second longitudinal edge 512 similar to the first major surface 206, the second major surface 208, the first longitudinal edge 210, and the second longitudinal edge 212 of the absorbent web 202 of FIG. 2A, respectively. The first longitudinal edge 510 and the second longitudinal edge 512 define a total width "W5-1" of the web body 504 therebetween along the transverse direction "D5-2".

The absorbent web 502 includes a first region 514 extending from the first longitudinal edge 510 and having a first width "W5-2" along the transverse direction "D5-2" less than the total width "W5-1" of the web body 504, a second region 516 spaced apart from the first region 514 and extending from the second longitudinal edge 512 and having a second width "W5-3" along the transverse direction "D5-2" less than the total width "W5-1" of the web body 504, and a third region 518 disposed between the first and second regions 514, 516 and having a third width "W5-4" along the transverse direction "D5-2" less than the total width "W5-1" of the web body 504. In the illustrated embodiment of FIG. 5, each of the first, second, and third regions 514, 516, 518 is a substantially rectangular region of the web body 504 extending from the first major surface 506 to the second major surface. Further, in the illustrated embodiment of FIG. 5, the first and second widths "W5-2", "W5-3" may be substantially equal to each other. In other embodiments, the first and second widths "W5-2", "W5-3" may be different from each other. Moreover, in the illustrated embodiment of FIG. 5, the third width "W5-4" may be greater than each of the first and second widths "W5-2", "W5-3". Further, in the illustrated embodiment of FIG. 5, the third width "W5-4" may be greater than each of the first and second widths "W5-2", "W5-3" by a factor of at least two. In other embodiments, the third width "W5-4" may be greater than each of the first and second widths "W5-2", "W5-3" by a factor of at least 1.25, 1.5, 2.5, 3, 3.5, 4, 5, and the like, without any limitations. Alternatively, the third width "W5-4" may be lesser than each of the first and second widths "W5-2", "W5-3". In another embodiment, the third width "W5-4" may be equal to the first and/or second widths "W5-2", "W5-3".

The web body 504 includes a plurality of first slits 520 extending from the first major surface 506 to the second major surface and disposed in the first region 514 of the web body 504. More particularly, the first slits 520 extend along a thickness (not shown) of the web body 504. Therefore, each first slit 520 is a through slit. Each first slit 520 is inclined from about 65 degrees to about 115 degrees relative to the machine direction "D5-1". Specifically, a first angle "A5-1" may be defined between the each first slit 520 and the machine direction "D5-1", such that the first angle "A5-1" may lie between 65 degrees and 115 degrees. Further, in some embodiments, at least some of the first slits 520 may be orthogonal to the machine direction "D5-1". In the illustrated embodiment of FIG. 5, the first angle "A5-1" may be substantially equal to 90 degrees, i.e., each first slit 520 may be orthogonal to the machine direction "D51-1". Further, in the illustrated embodiment of FIG. 5, each first slit 520 may be substantially parallel to the transverse direction "D5-2". Further, the first slits 520 may be substantially parallel to each other. Moreover, the first region 514 may define a first row of slits 522 including multiple first slits 520 and a second row of slits 524 including multiple first slits 520, such that each first slit 520 in the first row of slits 522 may be misaligned from the each first slit 520 in the second row of slits 524.

The web body 504 also includes a plurality of second slits 526 extending from the first major surface 506 to the second major surface and disposed in the second region 516 of the web body 504. More particularly, the second slits 526 extend along the thickness of the web body 504. Therefore, each second slit 526 is a through slit. Each second slit 526 is inclined from about 65 degrees to about 115 degrees relative to the machine direction "D5-1". Specifically, a second angle "A5-2" may be defined between each second slit 526 and the machine direction "D5-1", such that the second angle "A5-2" may lie between 65 degrees and 115 degrees. Further, in some embodiments, at least some of the second slits 526 may be orthogonal to the machine direction "D5-1". In the illustrated embodiment of FIG. 5, the second angle "A5-2" may be substantially equal to 90 degrees, i.e., each second slit 526 may be orthogonal to the machine direction "D51-1". Further, in the illustrated embodiment of FIG. 5, each second slit 526 may be substantially parallel to the transverse direction "D5-2". Moreover, the second slits 526 may be substantially parallel to each other. The second region 516 may define a first row of slits 528 including multiple second slits 526 and a second row of slits 530 including multiple second slits 526, such that each second slit 526 in the first row of slits 528 is misaligned from each second slit 526 in the second row of slits 530. Specifically, the first row of slits 528 is offset from the second row of slits 530 relative to the machine direction "D51-1".

The web body 504 further includes a plurality of third slits 532-1, 532-2, 532-3 extending from the first major surface 506 to the second major surface and disposed in the third region 518 of the web body 504. More particularly, the third slits 532-1, 532-2, 532-3 extend along the thickness of the web body 504. Therefore, each third slit 532-1, 532-2, 532-3 is a through slit. In the illustrated embodiment of FIG. 5, the third region 518 includes the multiple third slits 532-1, the multiple third slits 532-2, and the multiple third slits 532-3. At least some third slits 532-1, 532-2, 532-3 from the plurality of third slits 532-1, 532-2, 532-3 are substantially parallel to or inclined obliquely to the machine direction "D5-1". In some embodiments, each third slit 532-1, 532-2, 532-3 may be substantially parallel to or inclined obliquely to the machine direction "D5-1". Specifically, in the illustrated embodiment of FIG. 5, at least some third slits 532-1, 532-2 may be inclined obliquely to the machine direction "D5-1". Moreover, in the illustrated embodiment of FIG. 5, at least some third slits 532-1, 532-2 may be inclined obliquely to the transverse direction "D5-2".

Further, in the illustrated embodiment of FIG. 5, at least some third slits 532-1 from the plurality of third slits 532-1, 532-2, 532-3 may be inclined from about 85 degrees to about 265 degrees relative to the machine direction "D5-1". Specifically, a third angle "A5-3" may be defined between each third slit 532-1 and the machine direction "D5-1", such that the third angle "A5-3" may lie between 85 degrees and 265 degrees. Further, at least some third slits 532-1 from the plurality of third slits 532-1, 532-2, 532-3 may be inclined at an obtuse angle relative to the machine direction "D5-1". For example, the third slits 532-1 may be inclined relative to the machine direction "D5-1", such that the third angle "A5-3" may be an obtuse angle.

Moreover, in the illustrated embodiment of FIG. 5, at least some third slits 532-2 from the plurality of third slits 532-1, 532-2, 532-3 may be inclined from about 10 degrees to about 90 degrees relative to the machine direction "D5-1". Specifically, a fourth angle "A5-4" may be defined between each third slit 532-2 and the machine direction "D5-1", such that the fourth angle "A5-4" may lie between 10 degrees and 90 degrees. Further, at least some third slits 532-2 from the plurality of third slits 532-1, 532-2, 532-3 may be inclined at an acute angle relative to the machine direction "D5-1". Specifically, the third slits 532-2 may be inclined relative to the machine direction "D5-1", such that the fourth angle "A5-4" may be an acute angle.

Moreover, in the illustrated embodiment of FIG. 5, at least some third slits 532-3 from the plurality of third slits 532-1, 532-2, 532-3 may be substantially parallel to the machine direction "D5-1". Specifically, the third slits 532-3 may be parallel to the machine direction "D5-1". In the illustrated embodiment of FIG. 5, the third slits 532-3 may be orthogonal to the transverse direction "D5-2". In various embodiments, at least some third slits 532-1, 532-2, 532-3 from the plurality of third slits 532-1, 532-2, 532-3 may be orthogonal to the machine direction "D5-1".

Further, the plurality of third slits 532-1 may be parallel to each other, the plurality of third slits 532-2 may be parallel to each other, and the plurality of third slits 532-3 may be parallel to each other. Furthermore, at least some third slits 532-1, 532-2, 532-3 from the plurality of third slits 532-1, 532-2, 532-3 may be inclined obliquely to at least some other third slits 532-1, 532-2, 532-3 from the plurality of third slits 532-1, 532-2, 532-3. In the illustrated embodiment of FIG. 5, the third slits 532-1, 532-2, 532-3 may be inclined obliquely to each other. Alternatively, at least some third slits 532-1, 532-2, 532-3 from the plurality of third slits 532-1, 532-2, 532-3 may be substantially orthogonal to at least some other third slits 532-1, 532-2, 532-3 from the plurality of third slits 532-1, 532-2, 532-3.

Further, at least some third slits 532-1, 532-2, 532-3 from the plurality of third slits 532-1, 532-2, 532-3 are inclined to each first slit 520 and each second slit 526. In the illustrated embodiment of FIG. 5, each third slit 532-1, 532-2, 532-3 may be inclined to each first slit 520 and each second slit 526. Further, the third slits 532-3 may be orthogonal to each first slit 520 and each second slit 526. In some embodiments, at least some other third slits 532-1, 532-2, 532-3 from the plurality of third slits 532-1, 532-2, 532-3 may be substantially parallel to each first slit 520 and each second slit 526.

The first slits 520, the second slits 526, and the third slits 532-1, 532-2, 532-3 may be spatially arranged in at least three different directions. Further, the first slits 520, the second slits 526, and the third slits 532-1, 532-2, 532-3 may include different angular orientations with respect to each other. The angular orientation may differ by 90 degrees or more. Each of the pluralities of first, second, and third slits 520, 526, 532-1, 532-2, 532-3 may be substantially linear. In some embodiments, each of the pluralities of first, second, and third slits 520, 526, 532-1, 532-2, 532-3 may include a non-linear profile. In some embodiments, each of the pluralities of first, second, and third slits 520, 526, 532-1, 532-2, 532-3 may include a combination of a linear profile and a non-linear profile. Each of the plurality of first, second, and third slits 520, 526, 532-1, 532-2, 532-3 may include a slit length "L5-2" from about 3 mm to about 25 mm. In some embodiments, the slit length "L5-2" of the first, second, and third slits 520, 526, 532-1, 532-2, 532-3 may be similar to each other. Alternatively, the slit length "L5-2" of the first, second, and third slits 520, 526, 532-1, 532-2, 532-3 may be different from each other, without any limitations.

Further, multiple absorbent articles 500 may be cut from the absorbent web 502. For exemplary purposes, only two absorbent articles 500 are illustrated herein. The absorbent article 500 includes a closed perimeter 534 disposed along the third region 518 and extending at least partially into each of the first and second regions 514, 516. The closed perimeter 534 intersects at least some first slits 520 from the plurality of first slits 520 at corresponding first intersection points "P5-1", at least some second slits 526 from the plurality of second slits 526 at corresponding second intersection points "P5-2", and at least some third slits 532-1, 532-2, 532-3 from the plurality of third slits 532-1, 532-2, 532-3 at corresponding third intersection points "P5-3", "P5-4", "P5-5". Therefore, the closed perimeter 534 may cut through at least some of the first slits 520, at least some of the second slits 526, and at least some of the third slits 532-1, 532-2, such that the at least some first slits 520, at least some second slits 526, and at least some third slits 532-1, 532-2 are at partially bounded by the closed perimeter 534. A tangent 536, 538, 540-1, 540-2, 540-3 to the closed perimeter 534 at each of the first, second, and third intersection points "P5-1", "P5-2", "P5-3", "P5-4", "P5-5" forms an angle "I5-1", "I5-2", "I5-3", "I5-4", "I5-5" of at least about four degrees with the corresponding first, second, or third slits 520, 526, 532-1, 532-2, 532-3. Specifically, the tangents 536, 538, 540-1, 540-2, 540-3 may include a first tangent 536 at the first intersection point "P5-1", a second tangent 538 at the second intersection point "P5-2", and third tangents 540-1, 540-2, 540-3 at the respective third intersection points "P5-3", "P5-4", "P5-5". The angles "I5-1", "I5-2", "I5-3", "I5-4", "I5-5" may include a first intersection angle "I5-1", a second intersection angle "I5-2", and third intersection angles "I5-3", "I5-4", "I5-5". Further, the first intersection angle "I5-1" may be defined between each first slit 520 and the first tangent 536, the second intersection angle "I5-2" may be defined between each second slit 526 and the second tangent 538, the third intersection angle "I5-3" may be defined between each third slit 532-1 and the third tangent 540-1, the third intersection angle "I5-4" may be defined between each third slit 532-2 and the third tangent 540-2, and the third intersection angle "I5-5" may be defined between each third slit 532-3 and the third tangent 540-3. Each of the first, second, and third intersection angles "I5-1", "I5-2", "I5-3", "I5-4", "I5-5" may be greater than four degrees.

In some embodiments, the first intersection angle "I5-1" is at least 10 degrees, at least 20 degrees, at least 30 degrees, or at least 45 degrees, or at least 60 degrees. In some embodiments, the second intersection angle "I5-2" is at least 10 degrees, at least 20 degrees, at least 30 degrees, at least 45 degrees, or at least 60 degrees. In some embodiments, the third intersection angle "I5-3" is at least 10 degrees, at least 20 degrees, at least 30 degrees, at least 45 degrees, or at least 60 degrees. In some embodiments, the third intersection angle "I5-4" is at least 10 degrees, at least 20 degrees, at least 30 degrees, at least 45 degrees, or at least 60 degrees. In some embodiments, the third intersection angle "I5-5" is at least 10 degrees, at least 20 degrees, at least 30 degrees, at least 45 degrees, or at least 60 degrees.

Further, the first slits 520, the second slits 526, and the third slits 532-1, 532-2, 532-3 may be disposed in such a way relative to the closed perimeter 534, such that none of the first, second, and third slits 520, 526, 532-1, 532-2, 532-3 are tangential to or aligned with the closed perimeter 534 at the first intersection points "P5-1", the second intersection points "P5-2", and the third intersection points "P5-3", "P5-4", "P5-5", respectively. In the illustrated embodiment of FIG. 5, a shape of the closed perimeter 534 of the absorbent article 500 is double lobe-shape. The absorbent article 500 is formed from a portion of the absorbent web 502. The absorbent article 500 includes an article body 542. The article body 542 includes the first major surface 506 and the second major surface opposing the first major surface 506. The article body 542 also includes the closed perimeter 534 bounding the first major surface 506 and the second major surface. The article body 542 further includes the plurality of slits 520, 526, 532-1, 532-2, 532-3 extending from the first major surface 506 to the second major surface. At least some slits 520, 526, 532-1, 532-2, 532-3 from the plurality of slits 520, 526, 532-1, 532-2, 532-3 intersect the closed perimeter 534 at corresponding intersection points "P5-1", "P5-2", "P5-3", "P5-4", "P5-5". The tangent 536, 538, 540-1, 540-2, 540-3 to the closed perimeter 534 at each intersection point "P5-1", "P5-2", "P5-3", "P5-4", "P5-5" forms the angle "I5-1", "I5-2", "I5-3", "I5-4", "I5-5" of at least about four degrees with the corresponding slit 520, 526, 532-1, 532-2, 532-3.

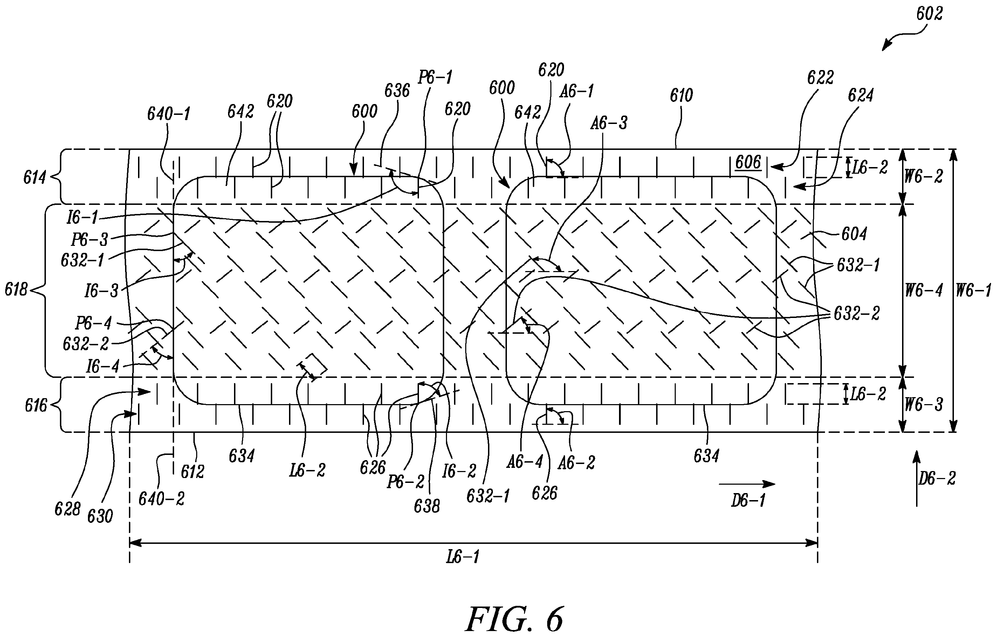

FIG. 6 illustrates an absorbent web 602, according to another embodiment of the present disclosure. The absorbent web 602 defines a web body 604, a machine direction "D6-1", a transverse direction "D6-2", and a length "L6-1" similar to the web body 204, the machine direction "D1-1", the transverse direction "D1-2", and the length "L1-1" of the first absorbent web 202 of FIG. 2A, respectively. Further, the absorbent web 602 defines a first major surface 606, a second major surface (not shown), a first longitudinal edge 610, and a second longitudinal edge 612 similar to the first major surface 206, the second major surface 208, the first longitudinal edge 210, and the second longitudinal edge 212 of the first absorbent web 202 of FIG. 2A, respectively. The first longitudinal edge 610 and the second longitudinal edge 612 define a total width "W6-1" of the web body 604 therebetween along the transverse direction "D6-2".

The absorbent web 602 includes a first region 614 extending from the first longitudinal edge 610 and having a first width "W6-2" along the transverse direction "D6-2" less than the total width "W6-1" of the web body 604, a second region 616 spaced apart from the first region 614 and extending from the second longitudinal edge 612 and having a second width "W6-3" along the transverse direction "D6-2" less than the total width "W6-1" of the web body 604, and a third region 618 disposed between the first and second regions 614, 616 and having a third width "W6-4" along the transverse direction "D6-2" less than the total width "W6-1" of the web body 604. In the illustrated embodiment of FIG. 6, each of the first, second, and third regions 614, 616, 618 is a substantially rectangular region of the web body 604 extending from the first major surface 606 to the second major surface. Further, in the illustrated embodiment of FIG. 6, the first and second widths "W6-2", "W6-3" may be substantially equal to each other. In other embodiments, the first and second widths "W6-2", "W6-3" may be different from each other. Moreover, in the illustrated embodiment of FIG. 6, the third width "W6-4" may be greater than each of the first and second widths "W6-2", "W6-3". Further, in the illustrated embodiment of FIG. 6, the third width "W6-4" may be greater than each of the first and second widths "W6-2", "W6-3" by a factor of at least two. In other embodiments, the third width "W6-4" may be greater than each of the first and second widths "W6-2", "W6-3" by a factor of at least 1.25, 1.5, 2.5, 3, 3.5, 4, 5, and the like, without any limitations. Alternatively, the third width "W6-4" may be lesser than each of the first and second widths "W6-2", "W6-3". In another embodiment, the third width "W6-4" may be equal to the first and/or second widths "W6-2", "W6-3".

The web body 604 includes a plurality of first slits 620 extending from the first major surface 606 to the second major surface and disposed in the first region 614 of the web body 604. More particularly, the first slits 620 extend along a thickness (not shown) of the web body 604. Therefore, each first slit 620 is a through slit. Each first slit 620 is inclined from about 65 degrees to about 115 degrees relative to the machine direction "D6-1". Specifically, a first angle "A6-1" may be defined between the each first slit 620 and the machine direction "D6-1", such that the first angle "A6-1" may lie between 65 degrees and 115 degrees. Further, in some embodiments, at least some of the first slits 620 may be orthogonal to the machine direction "D6-1". In the illustrated embodiment of FIG. 6, the first angle "A6-1" may be substantially equal to 90 degrees, i.e., each first slit 620 may be orthogonal to the machine direction "D6-1". Further, in the illustrated embodiment of FIG. 6, each first slit 620 may be substantially parallel to the transverse direction "D6-2". Moreover, the first slits 620 may be substantially parallel to each other. The first region 614 may define a first row of slits 622 including multiple first slits 620 and a second row of slits 624 including multiple first slits 620, such that each first slit 620 in the first row of slits 622 may be misaligned from each first slit 620 in the second row of slits 624. Specifically, the first row of slits 622 is offset from the second row of slits 624 relative to the machine direction "D6-1".

The web body 604 also includes a plurality of second slits 626 extending from the first major surface 606 to the second major surface and disposed in the second region 616 of the web body 604. More particularly, the second slits 626 extend along the thickness of the web body 604. Therefore, each second slit 626 is a through slit. Each second slit 626 is inclined from about 65 degrees to about 115 degrees relative to the machine direction "D6-1". Specifically, a second angle "A6-2" may be defined between the each second slit 626 and the machine direction "D6-1", such that the second angle "A6-2" may lie between 65 degrees and 115 degrees. Further, in some embodiments, at least some of the second slits 626 may be orthogonal to the machine direction "D6-1". In the illustrated embodiment of FIG. 6, the second angle "A6-2" may be substantially equal to 90 degrees, i.e., each second slit 620 may be orthogonal to the machine direction "D6-1". Further, in the illustrated embodiment of FIG. 6, each second slit 626 may be substantially parallel to the transverse direction "D6-2". Moreover, the second slits 626 may be substantially parallel to each other. The second region 616 may define a first row of slits 628 including multiple second slits 626 and a second row of slits 630 including multiple second slits 626, such that each second slit 626 in the first row of slits 628 may be misaligned from each second slit 626 in the second row of slits 630. Specifically, the first row of slits 628 is offset from the second row of slits 630 relative to the machine direction "D6-1".

The web body 604 further includes a plurality of third slits 632-1, 632-2 extending from the first major surface 606 to the second major surface and disposed in the third region 618 of the web body 604. More particularly, the third slits 632-1, 632-2 extend along the thickness of the web body 604. Therefore, each third slit 632-1, 632-2 is a through slit. In the illustrated embodiment of FIG. 6, the third region 618 includes the multiple third slits 632-1 and the multiple third slits 632-2. At least some third slits 632-1, 632-2 from the plurality of third slits 632-1, 632-2 are substantially parallel to or inclined obliquely to the machine direction "D6-1". In some embodiments, each third slit 632-1, 632-2 may be substantially parallel to or inclined obliquely to the machine direction "D6-1". In the illustrated embodiment of FIG. 6, each third slit 632-1, 632-2 may be inclined obliquely to the machine direction "D6-1". Moreover, in the illustrated embodiment of FIG. 6, each third slit 632-1, 632-2 may be inclined obliquely to the transverse direction "D6-2".

Further, in the illustrated embodiment of FIG. 6, at least some third slits 632-1 from the plurality of third slits 632-1, 632-2 may be inclined from about 85 degrees to about 265 degrees relative to the machine direction "D6-1". Specifically, a third angle "A6-3" may be defined between the each third slit 632-1 and the machine direction "D6-1", such that the third angle "A6-3" may lie between 85 degrees and 265 degrees. Further, at least some third slits 632-1 from the plurality of third slits 632-1, 632-2 may be inclined at an obtuse angle relative to the machine direction "D6-1". Specifically, the third slits 632-1 may be inclined relative to the machine direction "D6-1", such that the third angle "A6-3" may be an obtuse angle.

Moreover, in the illustrated embodiment of FIG. 6, at least some third slits 632-2 from the plurality of third slits 632-1, 632-2 may be inclined from about 20 degrees to about 115 degrees relative to the machine direction "D6-1". Specifically, a fourth angle "A6-4" may be defined between the each third slit 632-2 and the machine direction "D6-1", such that the fourth angle "A6-4" may lie between 20 degrees and 115 degrees. Further, at least some third slits 632-2 from the plurality of third slits 632-1, 632-2 may be inclined at an acute angle relative to the machine direction "D6-1". Specifically, the third slits 632-2 may be inclined relative to the machine direction "D6-1", such that the fourth angle "A6-4" may be an acute angle. In other embodiments, at least some third slits 632-1, 632-2 from the plurality of third slits 632-1, 632-2 may be substantially parallel to the machine direction "D6-1". In other embodiments, at least some third slits 632-1, 632-2 from the plurality of third slits 632-1, 632-2 may be orthogonal to the machine direction "D6-1".

Further, the plurality of third slits 632-1 may be parallel to each other. Additionally, the plurality of third slits 632-2 may be parallel to each other. Furthermore, at least some third slits 632-1, 632-2 from the plurality of third slits 632-1, 632-2 may be substantially orthogonal to at least some other third slits 632-1, 632-2 from the plurality of third slits 632-1, 632-2. In the illustrated embodiment of FIG. 6, each third slit 632-1 may be substantially orthogonal to each third slit 632-2. In some examples, at least some third slits 632-1, 632-2 from the plurality of third slits 632-1, 632-2 may be inclined obliquely to at least some other third slits 632-1, 632-2 from the plurality of third slits 632-1, 632-2. For example, each third slit 632-1 may be inclined obliquely to each slit 632-2. Further, at least some third slits 632-1, 632-2 from the plurality of third slits 632-1, 632-2 are inclined to each first slit 620 and each second slit 626. In the illustrated embodiment of FIG. 6, each third slit 632-1, 632-2 is inclined to each first slit 620 and each second slit 626. In some embodiments, at least some third slits 632-1, 632-2 from the plurality of third slits 632-1, 632-2 may be parallel to each first slit 620 and each second slit 626.

The first slits 620, the second slits 626, and the third slits 632-1, 632-2 may be spatially arranged in at least three different directions. Further, the first slits 620, the second slits 626, and the third slits 632-1, 632-2 may include different angular orientations with respect to each other. The angular orientation may differ by 90 degrees or more. Each of the pluralities of first, second, and third slits 620, 626, 632-1, 632-2 may be substantially linear. In some embodiments, each of the pluralities of first, second, and third slits 620, 626, 632-1, 632-2 may include a non-linear profile. In some embodiments, each of the pluralities of first, second, and third slits 620, 626, 632-1, 632-2 may include a combination of a linear profile and a non-linear profile. Each of the plurality of first, second, and third slits 620, 626, 632-1, 632-2 may include a slit length "L6-2" from about 3 mm to about 65 mm. In some embodiments, the slit length "L6-2" of the first, second, and third slits 620, 626, 632-1, 632-2 may be similar to each other. Alternatively, the slit length "L6-2" of the first, second, and third slits 620, 626, 632-1, 632-2 may be different from each other, without any limitations.

Further, multiple absorbent articles 600 may be cut from the absorbent web 602. For exemplary purposes, only two absorbent articles 600 are illustrated herein. The absorbent article 600 includes a closed perimeter 634 disposed along the third region 618 and extending at least partially into each of the first and second regions 614, 616. The closed perimeter 634 intersects at least some first slits 620 from the plurality of first slits 620 at corresponding first intersection points "P6-1", at least some second slits 626 from the plurality of second slits 626 at corresponding second intersection points "P6-2", and at least some third slits 632-1, 632-2 from the plurality of third slits 632-1, 632-2 at corresponding third intersection points "P6-3", "P6-4". Therefore, the closed perimeter 634 may cut through at least some of the first slits 620, at least some of the second slits 626, and at least some of the third slits 632-1, 632-2, such that the at least some first slits 620, at least some second slits 626, and at least some third slits 632-1, 632-2 are at partially bounded by the closed perimeter 634. A tangent 636, 638, 640-1, 640-2 to the closed perimeter 634 at each of the first, second, and third intersection points "P6-1", "P6-2", "P6-3", "P6-4" forms an angle "16-1", "16-2", "16-3", "I6-4" of at least about four degrees with the corresponding first, second, or third slits 620, 626, 632-1, 632-2. Specifically, the tangents 636, 638, 640-1, 640-2 may include a first tangent 636 at the first intersection point "P6-1", a second tangent 638 at the second intersection point "P6-2", third tangents 640-1, 640-2 at the respective third intersection points "P6-3", "P6-4". The angles "I6-1", "16-2", "16-3", "I6-4" may include a first intersection angle "I6-1", a second intersection angle "I6-2", and third intersection angles "16-3", "16-4". Further, the first intersection angle "I6-1" may be defined between each first slit 620 and the first tangent 636, the second intersection angle "16-2" may be defined between each second slit 626 and the second tangent 638, the third intersection angle "I6-3" may be defined between each third slit 632-1 and the third tangent 640-1, and the third intersection angle "I6-4" may be defined between each third slit 632-2 and the third tangent 640-2. Each of the first, second, and third intersection angles "I6-1", "16-2", "I6-3", "I6-4" may be greater than four degrees.

In some embodiments, the first intersection angle "I6-1" is at least 10 degrees, at least 20 degrees, at least 30 degrees, or at least 45 degrees, or at least 60 degrees. In some embodiments, the second intersection angle "I6-2" is at least 10 degrees, at least 20 degrees, at least 30 degrees, at least 45 degrees, or at least 60 degrees. In some embodiments, the third intersection angle "I6-3" is at least 10 degrees, at least 20 degrees, at least 30 degrees, at least 45 degrees, or at least 60 degrees. In some embodiments, the third intersection angle "16-4" is at least 10 degrees, at least 20 degrees, at least 30 degrees, at least 45 degrees, or at least 60 degrees.

Further, the first slits 620, the second slits 626, and the third slits 632-1, 632-2 may be disposed in such a way relative to the closed perimeter 634, such that none of the first, second, and third slits 620, 626, 632-1, 632-2 are tangential to or aligned with the closed perimeter 634 at the first intersection points "P6-1", the second intersection points "P6-2", and the third intersection points "P6-3", "P6-4", respectively. In the illustrated embodiment of FIG. 6, the shape of the closed perimeter 634 of the absorbent article 600 has a rectangular shape with rounded corners. The absorbent article 600 is formed from a portion of the absorbent web 602. The absorbent article 600 includes an article body 642. The article body 642 includes the first major surface 606 and the second major surface opposing the first major surface 606. The article body 642 also includes the closed perimeter 634 bounding the first major surface 606 and the second major surface. The article body 642 further includes the plurality of slits 620, 626, 632-1, 632-2 extending from the first major surface 606 to the second major surface. At least some slits 620, 626, 632-1, 632-2 from the plurality of slits 620, 626, 632-1, 632-2 intersect the closed perimeter 634 at corresponding intersection points "P6-1", "P6-2", "P6-3", "P6-4". The tangent 636, 638, 640-1, 640-2 to the closed perimeter 634 at each intersection point "P6-1", "P6-2", "P6-3", "P6-4" forms the angle "16-1", "16-2", "16-3", "16-4" of at least about four degrees with the corresponding slit 620, 626, 632-1, 632-2.

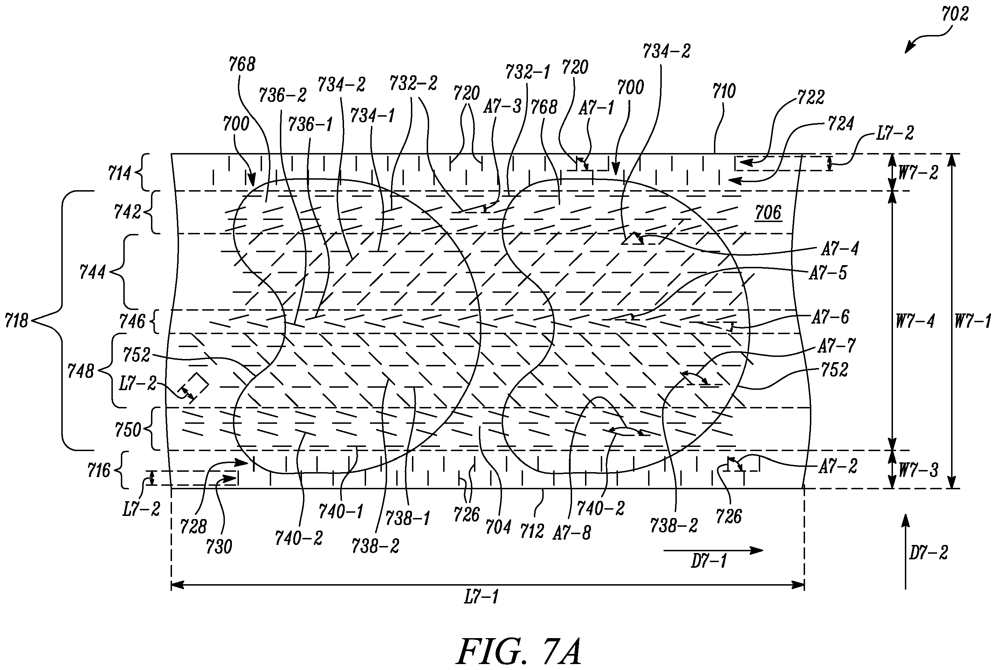

FIG. 7A illustrates an absorbent web 702, according to another embodiment of the present disclosure. The absorbent web 702 defines a web body 704, a machine direction "D7-1", a transverse direction "D7-2", and a length "L7-1" similar to the web body 204, the machine direction "D1-1", the transverse direction "D1-2", and the length "L1-1" of the absorbent web 202 of FIG. 2A, respectively. Further, the absorbent web 702 defines a first major surface 706, a second major surface (not shown), a first longitudinal edge 710, and a second longitudinal edge 712 similar to the first major surface 206, the second major surface 208, the first longitudinal edge 210, and the second longitudinal edge 212 of the absorbent web 202 of FIG. 2A, respectively. The first longitudinal edge 710 and the second longitudinal edge 712 define a total width "W7-1" of the web body 704 therebetween along the transverse direction "D7-2".

The absorbent web 702 includes a first region 714 extending from the first longitudinal edge 710 and having a first width "W7-2" along the transverse direction "D7-2" less than the total width "W7-1" of the web body 704, a second region 716 spaced apart from the first region 714 and extending from the second longitudinal edge 712 and having a second width "W7-3" along the transverse direction "D7-2" less than the total width "W7-1" of the web body 704, and a third region 718 disposed between the first and second regions 714, 716 and having a third width "W7-4" along the transverse direction "D7-2" less than the total width "W7-1" of the web body 704. In the illustrated embodiment of FIG. 7A, each of the first, second, and third regions 714, 716, 718 is a substantially rectangular region of the web body 704 extending from the first major surface 706 to the second major surface. Further, in the illustrated embodiment of FIG. 7A, the first and second widths "W7-2", "W7-3" may be substantially equal to each other. In other embodiments, the first and second widths "W7-2", "W7-3" may be different from each other. Moreover, in the illustrated embodiment of FIG. 7A, the third width "W7-4" may be greater than each of the first and second widths "W7-2", "W7-3". Further, in the illustrated embodiment of FIG. 7A, the third width "W7-4" may be greater than each of the first and second widths "W7-2", "W7-3" by a factor of at least four. In other embodiments, the third width "W7-4" may be greater than each of the first and second widths "W7-2", "W7-3" by a factor of at least 1.25, 1.5, 2.5, 3, 3.5, 4, 5, and the like, without any limitations. Alternatively, the third width "W7-4" may be lesser than each of the first and second widths "W7-2", "W7-3". In another embodiment, the third width "W7-4" may be equal to the first and/or second widths "W7-2", "W7-3".

The web body 704 includes a plurality of first slits 720 extending from the first major surface 706 to the second major surface and disposed in the first region 714 of the web body 704. More particularly, the first slits 720 extend along a thickness (not shown) of the web body 704. Therefore, each first slit 720 is a through slit. Each first slit 720 is inclined from about 65 degrees to about 115 degrees relative to the machine direction "D7-1". Specifically, a first angle "A7-1" may be defined between the each first slit 720 and the machine direction "D7-1", such that the first angle "A7-1" may lie between 65 degrees and 115 degrees. Further, in some embodiments, at least some of the first slits 720 may be orthogonal to the machine direction "D7-1". In the illustrated embodiment of FIG. 7A, the first angle "A7-1" may be substantially equal to 90 degrees, i.e., each first slit 720 may be orthogonal to the machine direction "D7-1". Further, in the illustrated embodiment of FIG. 7A, each first slit 720 may be substantially parallel to the transverse direction "D7-2". Moreover, the first slits 720 may be substantially parallel to each other. The first region 714 may define a first row of slits 722 including multiple first slits 720 and a second row of slits 724 including multiple first slits 720, such that each first slit 720 in the first row of slits 722 may be misaligned from each first slit 720 in the second row of slits 724. Specifically, the first row of slits 722 is offset from the second row of slits 724 relative to the machine direction "D7-1".

The web body 704 also includes a plurality of second slits 726 extending from the first major surface 706 to the second major surface and disposed in the second region 716 of the web body 704. Therefore, each second slit 726 is a through slit. More particularly, the second slits 726 extend along the thickness of the web body 704. Each second slit 726 is inclined from about 65 degrees to about 115 degrees relative to the machine direction "D7-1". Specifically, a second angle "A7-2" may be defined between the each second slit 726 and the machine direction "D7-1", such that the second angle "A7-2" may lie between 65 degrees and 115 degrees. Further, in some embodiments, at least some of the second slits 726 may be orthogonal to the machine direction "D7-1". In the illustrated embodiment of FIG. 7A, the second angle "A7-2" may be substantially equal to 90 degrees, i.e., each first slit 726 may be orthogonal to the machine direction "D7-1". Further, in the illustrated embodiment of FIG. 7A, each second slit 726 may be substantially parallel to the transverse direction "D7-2". Moreover, the second slits 726 may be substantially parallel to each other. The second region 716 may define a first row of slits 728 including multiple second slits 726 and a second row of slits 730 including multiple second slits 726, such that each second slit 726 in the first row of slits 728 may be misaligned from each second slit 726 in the second row of slits 730. Specifically, the first row of slits 728 is offset from the second row of slits 730 relative to the machine direction "D7-1".

The web body 704 further includes a plurality of third slits 732-1, 732-2, 734-1, 734-2, 736-1, 736-2, 738-1, 738-2, 740-1, 740-2 extending from the first major surface 706 to the second major surface and disposed in the third region 718 of the web body 704. More particularly, the third slits 732-1, 732-2, 734-1, 734-2, 736-1, 736-2, 738-1, 738-2, 740-1, 740-2 extend along the thickness of the web body 704. Therefore, each third slit 732-1, 732-2, 734-1, 734-2, 736-1, 736-2, 738-1, 738-2, 740-1, 740-2 is a through slit. Further, at least some third slits 732-1, 732-2, 734-1, 734-2, 736-1, 736-2, 738-1, 738-2, 740-1, 740-2 from the plurality of third slits 732-1, 732-2, 734-1, 734-2, 736-1, 736-2, 738-1, 738-2, 740-1, 740-2 are substantially parallel to or inclined obliquely to the machine direction "D7-1". In the illustrated embodiment of FIG. 7A, the third region 718 includes a first subregion 742 disposed adjacent to the first region 714. At least some third slits 732-1 from the plurality of third slits 732-1, 732-2 disposed in the first subregion 742 may be substantially parallel to the machine direction "D7-1". Further, at least some third slits 732-2 from the plurality of third slits 732-1, 732-2 disposed in the first subregion 742 may be inclined from about 10 degrees to about 20 degrees relative to the machine direction "D7-1". Specifically, a third angle "A7-3" may be defined between each third slit 732-2 and the machine direction "D7-1", such that the third angle "A7-3" may lie between 10 degrees and 20 degrees.

The third region 718 also includes a second subregion 744 disposed adjacent to the first subregion 742 opposite to the first region 714. At least some third slits 734-1 from the plurality of third slits 734-1, 734-2 disposed in the second subregion 744 may be substantially parallel to the machine direction "D7-1". Further, at least some third slits 734-2 from the plurality of third slits 734-1, 734-2 disposed in the second subregion 744 may be inclined from about 40 degrees to about 70 degrees relative to the machine direction "D7-1". Specifically, a fourth angle "A7-4" may be defined between each third slit 734-2 and the machine direction "D7-1", such that the fourth angle "A7-4" may lie between 40 degrees and 70 degrees.

The third region 718 further includes a third subregion 746 disposed adjacent to the second subregion 744 opposite to the first subregion 742. At least some third slits 736-1 from the plurality of third slits 736-1, 736-2 disposed in the third subregion 746 may be inclined from about 10 degrees to about 20 degrees relative to the machine direction "D7-1". Specifically, a fifth angle "A7-5" may be defined between each third slit 736-1 and the machine direction "D7-1", such that the fifth angle "A7-5" may lie between 10 degrees and 20 degrees. Further, at least some third slits 736-2 from the plurality of third slits 736-1, 736-2 disposed in the third subregion 746 may be inclined from about 85 degrees to about 265 degrees relative to the machine direction "D7-1". Specifically, a sixth angle "A7-6" may be defined between each third slit 736-2 and the machine direction "D7-1", such that the sixth angle "A7-6" may lie between 85 degrees and 265 degrees.

The third region 718 includes a fourth subregion 748 disposed adjacent to the third subregion 746 opposite to the second region 716. At least some third slits 738-1 from the plurality of third slits 738-1, 738-2 disposed in the fourth subregion 748 may be substantially parallel to the machine direction "D7-1". Further, at least some third slits 738-2 from the plurality of third slits 738-1, 738-2 disposed in the fourth subregion 748 may be inclined from about 110 degrees to about 140 degrees relative to the machine direction "D7-1". Specifically, a seventh angle "A7-7" may be defined between each third slit 732-2 and the machine direction "D7-1", such that the seventh angle "A7-7" may lie between 110 degrees and 140 degrees.

The third region 718 also includes a fifth subregion 750 disposed between the fourth subregion 748 and the second region 716. At least some third slits 740-1 from the plurality of third slits 740-1, 740-2 disposed in the fifth subregion 750 may be substantially parallel to the machine direction "D7-1". Further, at least some third slits 740-2 from the plurality of third slits 740-1, 740-2 disposed in the fifth subregion 750 may be inclined from about 160 degrees to about 170 degrees relative to the machine direction "D7-1". Specifically, an eighth angle "A7-8" may be defined between each third slit 732-1, 732-2 and the machine direction "D7-1", such that the eighth angle "A7-8" may lie between 160 degrees and 170 degrees.

It should be noted that some third slits 732-1, 732-2, 734-1, 734-2, 736-1, 736-2, 738-1, 738-2, 740-1, 740-2 may be inclined at an acute angle relative to the machine direction "D7-1". It should be further noted that some of third slits 732-1, 732-2, 734-1, 734-2, 736-1, 736-2, 738-1, 738-2, 740-1, 740-2 may be inclined at an obtuse angle relative to the machine direction "D7-1". Further, at least some third slits 732-1, 732-2, 734-1, 734-2, 736-1, 736-2, 738-1, 738-2, 740-1, 740-2 from the plurality of third slits 732-1, 732-2, 734-1, 734-2, 736-1, 736-2, 738-1, 738-2, 740-1, 740-2 are inclined to each first slit 720 and each second slit 726. In the illustrated embodiment of FIG. 7A, each third slit 732-1, 732-2, 734-1, 734-2, 736-1, 736-2, 738-1, 738-2, 740-1, 740-2 may be inclined to each first slit 720 and each second slit 726. Moreover, at least some third slits 732-1, 734-1, 738-1, 740-1 from the plurality of third slits 732-1, 732-2, 734-1, 734-2, 736-1, 736-2, 738-1, 738-2, 740-1, 740-2 may be orthogonal to each first slit 720 and each second slit 726. In the illustrated embodiment of FIG. 7A, each third slit 732-1, 734-1, 738-1, 740-1 may be orthogonal to each first slit 720 and each second slit 726. In some embodiments, at least some third slits 732-1, 732-2, 734-1, 734-2, 736-1, 736-2, 738-1, 738-2, 740-1, 740-2 from the plurality of third slits 732-1, 732-2, 734-1, 734-2, 736-1, 736-2, 738-1, 738-2, 740-1, 740-2 may be parallel to each first slit 720 and each second slit 726.

The first slits 720, the second slits 726, and the third slits 732-1, 732-2, 734-1, 734-2, 736-1, 736-2, 738-1, 738-2, 740-1, 740-2 may be spatially arranged in at least three different directions. Further, the first slits 720, the second slits 726, and the third slits 732-1, 732-2, 734-1, 734-2, 736-1, 736-2, 738-1, 738-2, 740-1, 740-2 may include different angular orientations with respect to each other. The angular orientation may differ by 90 degrees or more. Each of the pluralities of first, second, and third slits 720, 726, 732-1, 732-2, 734-1, 734-2, 736-1, 736-2, 738-1, 738-2, 740-1, 740-2 may be substantially linear. In some embodiments, each of the pluralities of first, second, and third slits 720, 726, 732-1, 732-2, 734-1, 734-2, 736-1, 736-2, 738-1, 738-2, 740-1, 740-2 may include a non-linear profile. In some embodiments, each of the pluralities of first, second, and third slits 720, 726, 732-1, 732-2, 734-1, 734-2, 736-1, 736-2, 738-1, 738-2, 740-1, 740-2 may include a combination of a linear profile and a non-linear profile. Each of the plurality of first, second, and third slits 720, 726, 732-1, 732-2, 734-1, 734-2, 736-1, 736-2, 738-1, 738-2, 740-1, 740-2 may include a slit length "L7-2" from about 3 mm to about 25 mm. In some embodiments, the slit length "L7-2" of the first, second, and third slits 720, 726, 732-1, 732-2, 734-1, 734-2, 736-1, 736-2, 738-1, 738-2, 740-1, 740-2 may be similar to each other. Alternatively, the slit length "L7-2" of the first, second, and third slits 720, 726, 732-1, 732-2, 734-1, 734-2, 736-1, 736-2, 738-1, 738-2, 740-1, 740-2 may be different from each other, without any limitations.

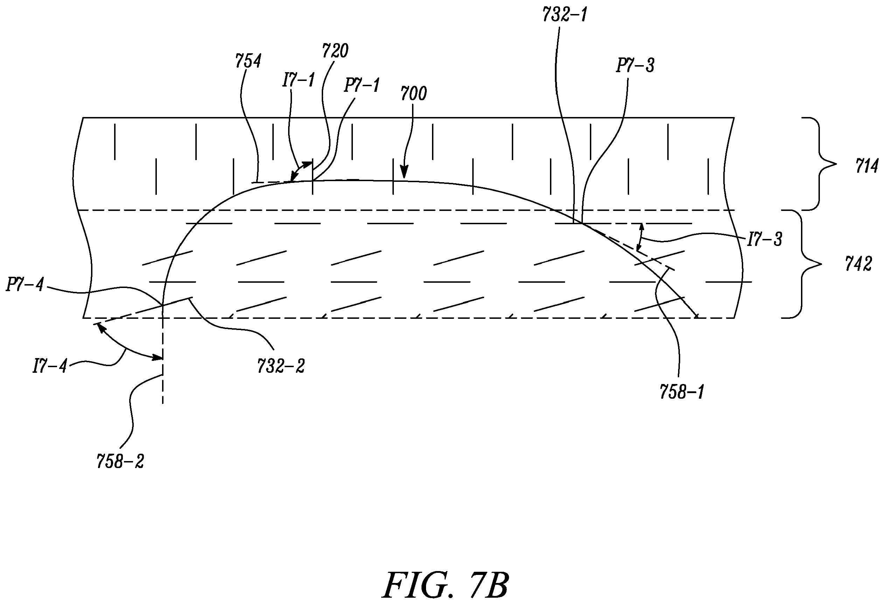

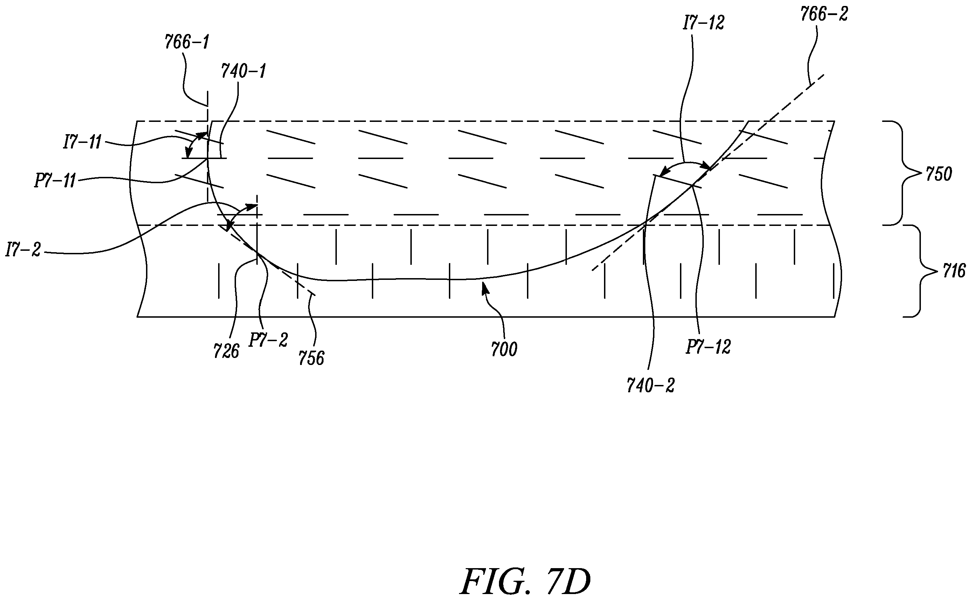

Further, multiple absorbent articles 700 may be cut from the absorbent web 702. For exemplary purposes, only two absorbent articles 700 are illustrated herein. The absorbent article 700 includes a closed perimeter 752 disposed along the third region 718 and extending at least partially into each of the first and second regions 714, 716. The closed perimeter 752 intersects at least some first slits 720 from the plurality of first slits 720 at corresponding first intersection points "P7-1" (shown in FIG. 7B), at least some second slits 726 from the plurality of second slits 726 at corresponding second intersection points "P7-2" (shown in FIG. 7D), and at least some third slits 732-1, 732-2, 734-1, 734-2, 736-1, 736-2, 738-1, 738-2, 740-1, 740-2 from the plurality of third slits 732-1, 732-2, 734-1, 734-2, 736-1, 736-2, 738-1, 738-2, 740-1, 740-2 at corresponding third, fourth, fifth, sixth, and seventh intersection points "P7-3", "P7-4", "P7-5", "P7-6", "P7-7", "P7-8", "P7-9", "P7-10", "P7-11", "P7-12" (shown in FIGS. 7B to 7D). Therefore, the closed perimeter 734 may cut through at least some of the first slits 720, at least some of the second slits 726, and at least some of the third slits 732-1, 732-2, 734-1, 734-2, 736-1, 736-2, 738-1, 738-2, 740-1, 740-2, such that the at least some first slits 720, at least some second slits 726, and at least some third slits 732-1, 732-2, 734-1, 734-2, 736-1, 736-2, 738-1, 738-2, 740-1, 740-2 are at partially bounded by the closed perimeter 734. A tangent 754, 756, 758-1, 758-2, 760-1, 760-2, 762-1, 762-2, 764-1, 764-2, 766-1, 766-2 (shown in FIGS. 7B to 7D) to the closed perimeter 752 at each of the first, second, third, fourth, fifth, sixth, and seventh intersection points "P7-1", "P7-2", "P7-3", "P7-4", "P7-5", "P7-6", "P7-7", "P7-8", "P7-9", "P7-10", "P7-11", "P7-12" forms an angle "17-1", "I7-2", "I7-3", "I7-4", "I7-5", "I7-6", "I7-7", "I7-8", "I7-9", "I7-10", "I7-11", "I7-12" of at least about four degrees with the corresponding first, second, or third slits 720, 726, 732-1, 732-2, 734-1, 734-2, 736-1, 736-2, 738-1, 738-2, 740-1, 740-2. Specifically, the tangents 754, 756, 758-1, 758-2, 760-1, 760-2, 762-1, 762-2, 764-1, 764-2, 766-1, 766-2 may include a first tangent 754 at the first intersection point "P7-1", a second tangent 756 at the second intersection point "P7-2", third tangents 758-1, 758-2 at the respective third intersection points "P7-3", "P7-4", fourth tangents 760-1, 760-2 at the respective fourth intersection points "P7-5", "P7-6", fifth tangents 762-1, 762-2 at the respective fifth intersection points "P7-7", "P7-8", sixth tangents 764-1, 764-2 at the respective sixth intersection points "P7-9", "P7-10", and seventh tangents 766-1, 766-2 at the respective seventh intersection points "P7-11", "P7-12".

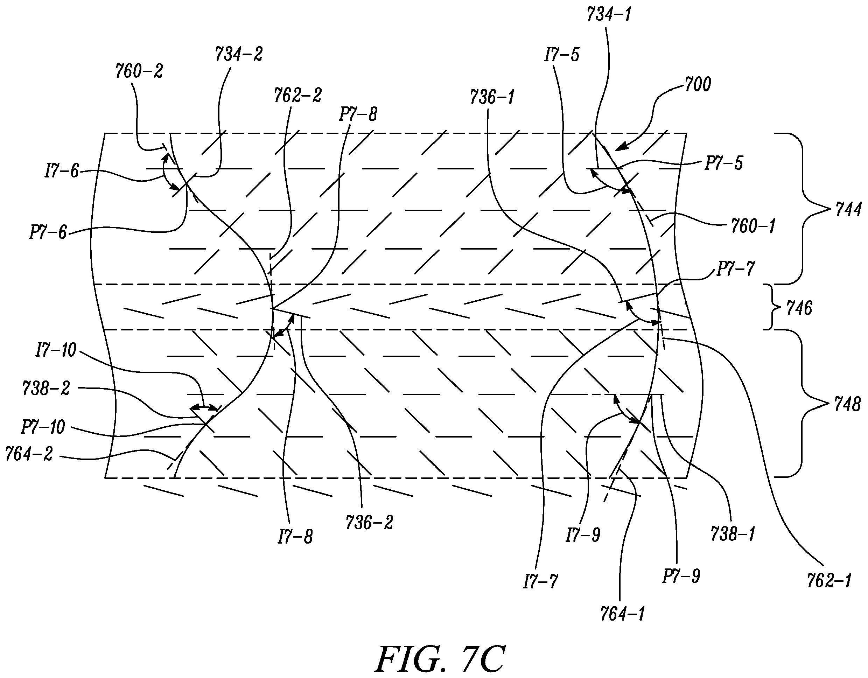

The angles "17-1", "I7-2", "I7-3", "I7-4", "I7-5", "I7-6", "I7-7", "I7-8", "I7-9", "17-10", "I7-11", "I7-12" may include a first intersection angle "17-1", a second intersection angle "I7-2", third intersection angles "I7-3", "I7-4", fourth intersection angles "I7-5", "I7-6", fifth intersection angles "I7-7", "I7-8", sixth intersection angles "I7-9", "I7-10", and seventh intersection angles "17-11", "I7-12". Further, as shown in FIG. 7B, the first intersection angle "I7-1" may be defined between each first slit 720 and the first tangent 754. As shown in FIG. 7D, the second intersection angle "I7-2" may be defined between each second slit 726 and the second tangent 756. As shown in FIG. 7B, the third intersection angle "I7-3" may be defined between each third slit 732-1 and the third tangent 758-1, and the third intersection angle "I7-4" may be defined between each third slit 732-2 and the third tangent 758-2. As shown in FIG. 7C, the fourth intersection angle "I7-5" may be defined between each third slit 734-1 and the fourth tangent 760-1, the fourth intersection angle "I7-6" may be defined between each third slit 734-2 and the fourth tangent 760-2, the fifth intersection angle "I7-7" may be defined between each third slit 736-1 and the fifth tangent 762-1, the fifth intersection angle "I7-8" may be defined between each third slit 736-2 and the fifth tangent 762-2, the sixth intersection angle "I7-9" may be defined between each third slit 738-1 and the sixth tangent 764-1, and the sixth intersection angle "I7-10" may be defined between each third slit 738-2 and the sixth tangent 764-2. As shown in FIG. 7D, the seventh intersection angle "I7-11" may be defined between each third slit 740-1 and the seventh tangent 766-1, and the seventh intersection angle "I7-12" may be defined between each third slit 740-2 and the seventh tangent 766-2. Each of the first, second, third, fourth, fifth, sixth, and seventh intersection angles "I7-1", "I7-2", "I7-3", "I7-4", "I7-5", "I7-6", "I7-7", "I7-8", "I7-9", "I7-10", "I7-11", "I7-12" may be greater than four degrees.

In some embodiments, the first intersection angle "I7-1" is at least 10 degrees, at least 20 degrees, at least 30 degrees, or at least 45 degrees, or at least 60 degrees. In some embodiments, the second intersection angle "I7-2" is at least 10 degrees, at least 20 degrees, at least 30 degrees, at least 45 degrees, or at least 60 degrees. In some embodiments, each third intersection angle "I7-3", "I7-4", "I7-5", "I7-6", "I7-7", "I7-8", "I7-9", "I7-10", "I7-11", "I7-12" is at least 10 degrees, at least 20 degrees, at least 30 degrees, at least 45 degrees, or at least 60 degrees.

As shown in FIG. 7A, the first slits 720, the second slits 726, and the third slits 732-1, 732-2, 734-1, 734-2, 736-1, 736-2, 738-1, 738-2, 740-1, 740-2 may be disposed in such a way relative to the closed perimeter 752, such that none of the first, second, and third slits 720, 726, 732-1, 732-2, 734-1, 734-2, 736-1, 736-2, 738-1, 738-2, 740-1, 740-2 are tangential to or aligned with the closed perimeter 752 at the first intersection points "P7-1", the second intersection points "P7-2", and the third intersection points "P7-3", "P7-4", "P7-5", "P7-6", "P7-7", "P7-8", "P7-9", "P7-10", "P7-11", "P7-12", respectively. In the illustrated embodiment of FIG. 7A, a shape of the closed perimeter 752 of the absorbent article 700 includes a kidney shape. The absorbent article 700 is formed from a portion of the absorbent web 702. The absorbent article 700 includes an article body 768. The article body 768 includes the first major surface 706 and the second major surface opposing the first major surface 706. The article body 768 also includes the closed perimeter 752 bounding the first major surface 706 and the second major surface. The article body 768 further includes the plurality of slits 720, 726, 732-1, 732-2, 734-1, 734-2, 736-1, 736-2, 738-1, 738-2, 740-1, 740-2 extending from the first major surface 706 to the second major surface. At least some slits 720, 726, 732-1, 732-2, 734-1, 734-2, 736-1, 736-2, 738-1, 738-2, 740-1, 740-2 from the plurality of slits 720, 726, 732-1, 732-2, 734-1, 734-2, 736-1, 736-2, 738-1, 738-2, 740-1, 740-2 intersect the closed perimeter 752 at corresponding intersection points "P7-1", "P7-2", "P7-3", "P7-4", "P7-5", "P7-6", "P7-7", "P7-8", "P7-9", "P7-10", "P7-11", "P7-12". The tangent 754, 756, 758-1, 758-2, 760-1, 760-2, 762-1, 762-2, 764-1, 764-2, 766-1, 766-2 to the closed perimeter 752 at each intersection point "P7-1", "P7-2", "P7-3", "P7-4", "P7-5", "P7-6", "P7-7", "P7-8", "P7-9", "P7-10", "P7-11", "P7-12" forms the angle "17-1", "I7-2", "I7-3 ", "I7-4", "I7-5", "I7-6", "I7-7", "I7-8", "I7-9", "I7-10", "I7-11", "I7-12" of at least about four degrees with the corresponding slit 720, 726, 732-1, 732-2, 734-1, 734-2, 736-1, 736-2, 738-1, 738-2, 740-1, 740-2.

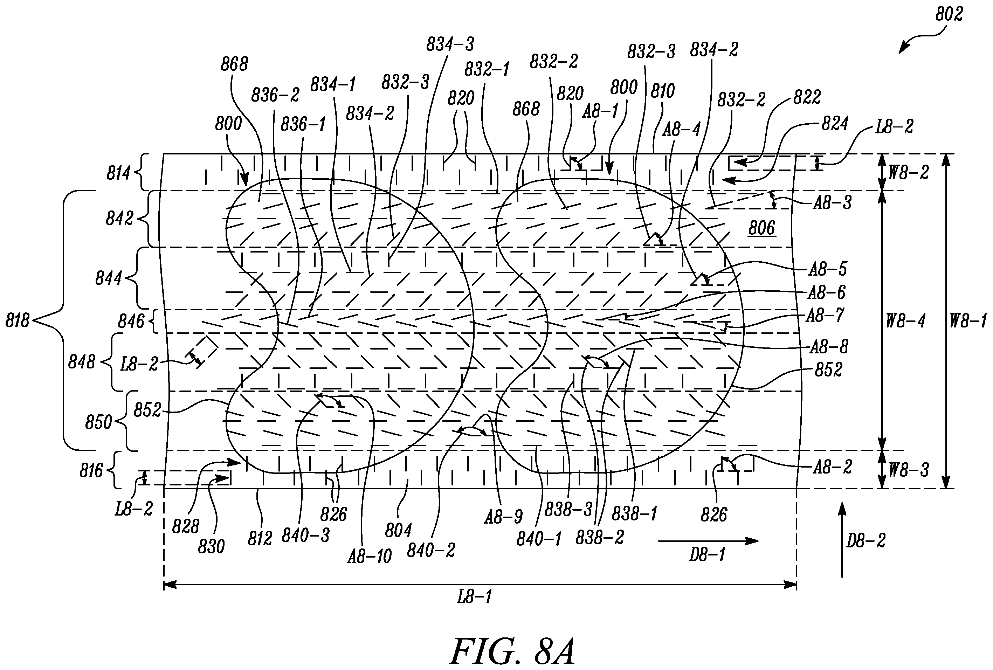

FIG. 8A illustrates a third absorbent web 802, according to another embodiment of the present disclosure. The absorbent web 802 defines a web body 804, a machine direction "D8-1", a transverse direction "D8-2", and a length "L8-1" similar to the web body 204, the machine direction "D1-1", the transverse direction "D1-2", and the length "L1-1" of the absorbent web 202 of FIG. 2A, respectively. Further, the absorbent web 802 defines a first major surface 806, a second major surface (not shown), a first longitudinal edge 810, and a second longitudinal edge 812 similar to the first major surface 206, the second major surface 208, the first longitudinal edge 210, and the second longitudinal edge 212 of the absorbent web 202 of FIG. 2A, respectively. The first longitudinal edge 810 and the second longitudinal edge 812 define a total width "W8-1" of the web body 804 therebetween along the transverse direction "D8-2".

The absorbent web 802 includes a first region 814 extending from the first longitudinal edge 810 and having a first width "W8-2" along the transverse direction "D8-2" less than the total width "W8-1" of the web body 804, a second region 816 spaced apart from the first region 814 and extending from the second longitudinal edge 812 and having a second width "W8-3" along the transverse direction "D8-2" less than the total width "W8-1" of the web body 804, and a third region 818 disposed between the first and second regions 814, 816 and having a third width "W8-4" along the transverse direction "D8-2" less than the total width "W8-1" of the web body 804. In the illustrated embodiment of FIG. 8A, each of the first, second, and third regions 814, 816, 818 is a substantially rectangular region of the web body 804 extending from the first major surface 806 to the second major surface. Further, in the illustrated embodiment of FIG. 8A, the first and second widths "W8-2", "W8-3" may be substantially equal to each other. In other embodiments, the first and second widths "W8-2", "W8-3" may be different from each other. Moreover, in the illustrated embodiment of FIG. 8A, the third width "W8-4" may be greater than each of the first and second widths "W8-2", "W8-3". Further, in the illustrated embodiment of FIG. 8A, the third width "W8-4" may be greater than each of the first and second widths "W8-2", "W8-3" by a factor of at least four. In other embodiments, the third width "W8-4" may be greater than each of the first and second widths "W8-2", "W8-3" by a factor of at least 1.25, 1.5, 2.5, 3, 3.5, 4, 5, and the like, without any limitations. Alternatively, the third width "W8-4" may be lesser than each of the first and second widths "W8-2", "W8-3". In another embodiment, the third width "W8-4" may be equal to the first and/or second widths "W8-2", "W8-3".

The web body 804 includes a plurality of first slits 820 extending from the first major surface 806 to the second major surface and disposed in the first region 814 of the web body 804. More particularly, the first slits 820 extend along a thickness (not shown) of the web body 804. Therefore, each first slit 820 is a through slit. Each first slit 820 is inclined from about 65 degrees to about 115 degrees relative to the machine direction "D8-1". Specifically, a first angle "A8-1" may be defined between the each first slit 820 and the machine direction "D8-1", such that the first angle "A8-1" may lie between 65 degrees and 115 degrees. Further, in some embodiments, at least some of the first slits 820 may be orthogonal to the machine direction "D8-1". In the illustrated embodiment of FIG. 8A, the first angle "A8-1" may be substantially equal to 90 degrees, i.e., each first slit 820 may be orthogonal to the machine direction "D8-1". Further, in the illustrated embodiment of FIG. 8A, each first slit 820 may be substantially parallel to the transverse direction "D8-2". Moreover, the first slits 820 may be substantially parallel to each other. The first region 814 may define a first row of slits 822 including multiple first slits 820 and a second row of slits 824 including multiple first slits 820, such that each first slit 820 in the first row of slits 822 may be misaligned from each first slit 820 in the second row of slits 824. Specifically, the first row of slits 822 is offset from the second row of slits 824 relative to the machine direction "D8-1".

The web body 804 also includes a plurality of second slits 826 extending from the first major surface 806 to the second major surface and disposed in the second region 816 of the web body 804. More particularly, the second slits 826 extend along the thickness of the web body 804. Therefore, each second slit 826 is a through slit. Each second slit 826 is inclined from about 65 degrees to about 115 degrees relative to the machine direction "D8-1". Specifically, a second angle "A8-2" may be defined between the each second slit 826 and the machine direction "D8-1", such that the second angle "A8-2" may lie between 65 degrees and 115 degrees. Further, in some embodiments, at least some of the second slits 826 may be orthogonal to the machine direction "D8-1". In the illustrated embodiment of FIG. 8A, the second angle "A8-2" may be substantially equal to 90 degrees, i.e., each second slit 826 may be orthogonal to the machine direction "D8-1". Further, in the illustrated embodiment of FIG. 8A, each second slit 826 may be substantially parallel to the transverse direction "D8-2". Moreover, the second slits 826 may be substantially parallel to each other. The second region 816 may define a first row of slits 828 including multiple second slits 826 and a second row of slits 830 including multiple second slits 826, such that each second slit 826 in the first row of slits 828 may be misaligned from each second slit 826 in the second row of slits 830. Specifically, the first row of slits 828 is offset from the second row of slits 830 relative to the machine direction "D8-1".

The web body 804 further includes a plurality of third slits 832-1, 832-2, 832-3, 834-1, 834-2, 834-3, 836-1, 836-2, 838-1, 838-2, 838-3, 840-1, 840-2, 840-3 extending from the first major surface 806 to the second major surface and disposed in the third region 818 of the web body 804. More particularly, the third slits 832-1, 832-2, 832-3, 834-1, 834-2, 834-3, 836-1, 836-2, 838-1, 838-2, 838-3, 840-1, 840-2, 840-3 extend along the thickness of the web body 804. Therefore, each third slit 832-1, 832-2, 832-3, 834-1, 834-2, 834-3, 836-1, 836-2, 838-1, 838-2, 838-3, 840-1, 840-2, 840-3 is a through slit. Further, at least some third slits 832-1, 832-2, 832-3, 834-1, 834-2, 834-3, 836-1, 836-2, 838-1, 838-2, 838-3, 840-1, 840-2, 840-3 from the plurality of third slits 832-1, 832-2, 832-3, 834-1, 834-2, 834-3, 836-1, 836-2, 838-1, 838-2, 838-3, 840-1, 840-2, 840-3 are substantially parallel to or inclined obliquely to the machine direction "D8-1". In the illustrated embodiment of FIG. 8A, the third region 818 includes a first subregion 842 disposed adjacent to the first region 814. At least some third slits 832-1 from the plurality of third slits 832-1, 832-2, 832-3 disposed in the first subregion 842 may be substantially parallel to the machine direction "D8-1". Further, at least some third slits 832-2 from the plurality of third slits 832-1, 832-2, 832-3 disposed in the first subregion 842 may be inclined from about 10 degrees to about 20 degrees relative to the machine direction "D8-1". Specifically, a third angle "A8-3" may be defined between each third slit 832-2 and the machine direction "D8-1", such that the third angle "A8-3" may lie between 10 degrees and 20 degrees. Further, at least some third slits 832-3 from the plurality of third slits 832-1, 832-2, 832-3 disposed in the first subregion 842 may be inclined from about 65 degrees to about 115 degrees relative to the machine direction "D8-1". Specifically, a fourth angle "A8-4" may be defined between each third slit 832-3 and the machine direction "D8-1", such that the fourth angle "A8-4" may lie between 65 degrees and 115 degrees.

The third region 818 also includes a second subregion 844 disposed adjacent to the first subregion 842 opposite to the first region 814. At least some third slits 834-1 from the plurality of third slits 834-1, 834-2, 834-3 disposed in the second subregion 844 may be substantially parallel to the machine direction "D8-1". The third slits 834-1 may be substantially orthogonal to the transverse direction "D8-2". Further, at least some third slits 834-2 from the plurality of third slits 834-1, 834-2, 834-3 may be inclined from about 40 degrees to about 70 degrees relative to the machine direction "D8-1". Specifically, a fifth angle "A8-5" may be defined between each third slit 834-2 and the machine direction "D8-1", such that the fifth angle "A8-5" may lie between 40 degrees and 70 degrees. In some embodiments, at least some third slits 834-3 from the plurality of third slits 834-1, 834-2, 834-3 may be orthogonal to the machine direction "D8-1". Specifically, the third slits 834-3 may be orthogonal to the machine direction "D8-1" and parallel to the transverse direction "D8-2". Further, at least some third slits 834-1 are disposed between adjacent third slits 834-3.

The third region 818 further includes a third subregion 846 disposed adjacent to the second subregion 844 opposite to the first subregion 842. At least some third slits 836-1 from the plurality of third slits 836-1, 836-2 disposed in the third subregion 846 may be inclined from about 10 degrees to about 20 degrees relative to the machine direction "D8-1". Specifically, a sixth angle "A8-6" may be defined between each third slit 836-1 and the machine direction "D8-1", such that the sixth angle "A8-6" may lie between 10 degrees and 20 degrees. Further, at least some third slits 836-2 from the plurality of third slits 836-1, 836-2 disposed in the third subregion 846 may be inclined from about 85 degrees to about 265 degrees relative to the machine direction "D8-1". Specifically, a seventh angle "A8-7" may be defined between each third slit 836-2 and the machine direction "D8-1", such that the seventh angle "A8-7" may lie between 85 degrees and 265 degrees.

The third region 818 includes a fourth subregion 848 disposed adjacent to the third subregion 846 opposite to the second region 816. At least some third slits 838-1 from the plurality of third slits 838-1, 838-2, 838-3 disposed in the fourth subregion 848 may be substantially parallel to the machine direction "D8-1". The third slits 838-1 may be substantially orthogonal to the transverse direction "D8-2". Further, at least some third slits 838-2 from the plurality of third slits 838-1, 838-2, 838-3 may be inclined from about 110 degrees to about 140 degrees relative to the machine direction "D8-1". Specifically, an eighth angle "A8-8" may be defined between each third slit 838-2 and the machine direction "D8-1", such that the eighth angle "A8-8" may lie between 110 degrees and 140 degrees. In some embodiments, at least some third slits 838-3 from the plurality of third slits 838-1, 838-2, 838-3 may be orthogonal to the machine direction "D8-1". Specifically, the third slits 838-3 may be orthogonal to the machine direction "D8-1" and parallel to the transverse direction "D8-2". Further, at least some third slits 838-1 are disposed between adjacent third slits 838-3.

The third region 818 also includes a fifth subregion 850 disposed between the fourth subregion 848 and the second region 816. At least some third slits 840-1 from the plurality of third slits 840-1, 840-2, 840-3 disposed in the fifth subregion 850 may be substantially parallel to the machine direction "D8-1". Further, at least some third slits 840-2 from the plurality of third slits 840-1, 840-2, 840-3 disposed in the fifth subregion 850 may be inclined from about 160 degrees to about 170 degrees relative to the machine direction "D8-1". Specifically, a ninth angle "A8-9" may be defined between each third slit 840-2 and the machine direction "D8-1", such that the ninth angle "A8-9" may lie between 160 degrees and 170 degrees. Further, at least some third slits 840-3 from the plurality of third slits 840-1, 840-2, 840-3 disposed in the fifth subregion 850 may be inclined from about 90 degrees to about 160 degrees relative to the machine direction "D8-1". Specifically, a tenth angle "A8-10" may be defined between each third slit 840-3 and the machine direction "D8-1", such that the tenth angle "A8-10" may lie between 90 degrees and 160 degrees.

It should be noted that some third slits 832-1, 832-2, 832-3, 834-1, 834-2, 834-3, 836-1, 836-2, 838-1, 838-2, 838-3, 840-1, 840-2, 840-3 may be inclined at an acute angle relative to the machine direction "D8-1". It should be further noted that some third slits 832-1, 832-2, 832-3, 834-1, 834-2, 834-3, 836-1, 836-2, 838-1, 838-2, 838-3, 840-1, 840-2, 840-3 may be inclined at an obtuse angle relative to the machine direction "D8-1". Further, at least some third slits 832-1, 832-2, 832-3, 834-1, 834-2, 836-1, 836-2, 838-1, 838-2, 840-1, 840-2, 840-3 from the plurality of third slits 832-1, 832-2, 832-3, 834-1, 834-2, 834-3, 836-1, 836-2, 838-1, 838-2, 838-3, 840-1, 840-2, 840-3 may be inclined to each first slit 820 and each second slit 826. In the illustrated embodiment of FIG. 8A, each third slit 832-1, 832-2, 832-3, 834-1, 834-2, 836-1, 836-2, 838-1, 838-2, 840-1, 840-2, 840-3 may be inclined to each first slit 820 and each second slit 826. Moreover, at least some third slits 832-1, 834-1, 838-1, 840-1 from the plurality of third slits 832-1, 832-2, 832-3, 834-1, 834-2, 834-3, 836-1, 836-2, 838-1, 838-2, 838-3, 840-1, 840-2, 840-3 may be orthogonal to each first slit 820 and each second slit 826. In the illustrated embodiment of FIG. 8A, each third slit 832-1, 834-1, 838-1, 840-1 may be orthogonal to each first slit 820 and each second slit 826. In some embodiments, at least some third slits 834-3, 838-3 from the plurality of third slits 832-1, 832-2, 832-3, 834-1, 834-2, 834-3, 836-1, 836-2, 838-1, 838-2, 838-3, 840-1, 840-2, 840-3 may be parallel to each first slit 820 and each second slit 826. In the illustrated embodiment of FIG. 8A, each third slit 834-3, 838-3 may be parallel to each first slit 820 and each second slit 826.

The first slits 820, the second slits 826, and the third slits 832-1, 832-2, 832-3, 834-1, 834-2, 834-3, 836-1, 836-2, 838-1, 838-2, 838-3, 840-1, 840-2, 840-3 may be spatially arranged in at least three different directions. Further, the first slits 820, the second slits 826, and the third slits 832-1, 832-2, 832-3, 834-1, 834-2, 834-3, 836-1, 836-2, 838-1, 838-2, 838-3, 840-1, 840-2, 840-3 may include different angular orientations with respect to each other. The angular orientation may differ by 90 degrees or more. Each of the pluralities of first, second, and third slits 820, 826, 832-1, 832-2, 832-3, 834-1, 834-2, 834-3, 836-1, 836-2, 838-1, 838-2, 838-3, 840-1, 840-2, 840-3 may be substantially linear. In some embodiments, each of the pluralities of first, second, and third slits 820, 826, 832-1, 832-2, 832-3, 834-1, 834-2, 834-3, 836-1, 836-2, 838-1, 838-2, 838-3, 840-1, 840-2, 840-3 may include a non-linear profile. In some embodiments, each of the pluralities of first, second, and third slits 820, 826, 832-1, 832-2, 832-3, 834-1, 834-2, 834-3, 836-1, 836-2, 838-1, 838-2, 838-3, 840-1, 840-2, 840-3 may include a combination of a linear profile and a non-linear profile. Each of the plurality of first, second, and third slits 820, 826, 832-1, 832-2, 832-3, 834-1, 834-2, 834-3, 836-1, 836-2, 838-1, 838-2, 838-3, 840-1, 840-2, 840-3 may include a slit length "L8-2" from about 3 mm to about 25 mm. In some embodiments, the slit length "L8-2" of the first, second, and third slits 820, 826, 832-1, 832-2, 832-3, 834-1, 834-2, 834-3, 836-1, 836-2, 838-1, 838-2, 838-3, 840-1, 840-2, 840-3 may be similar to each other. Alternatively, the slit length "L8-2" of the first, second, and third slits 820, 826, 832-1, 832-2, 832-3, 834-1, 834-2, 834-3, 836-1, 836-2, 838-1, 838-2, 838-3, 840-1, 840-2, 840-3 may be different from each other, without any limitations.

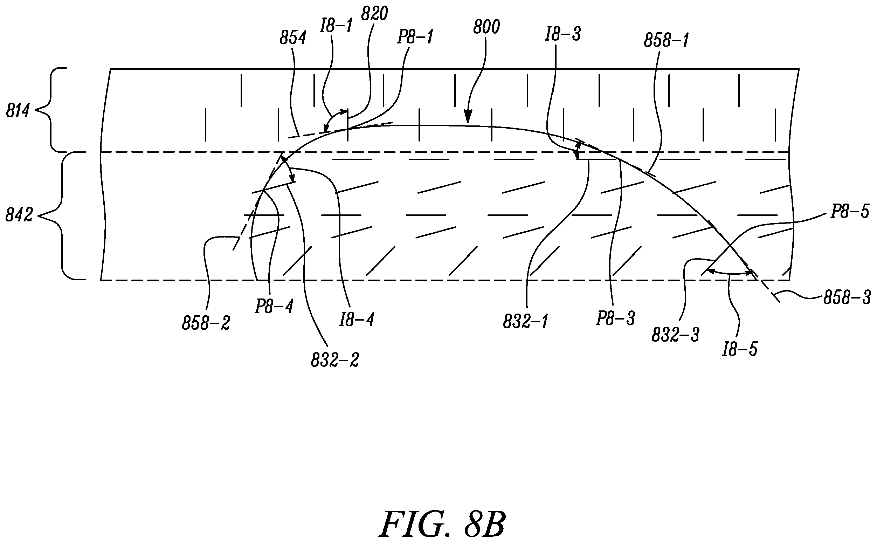

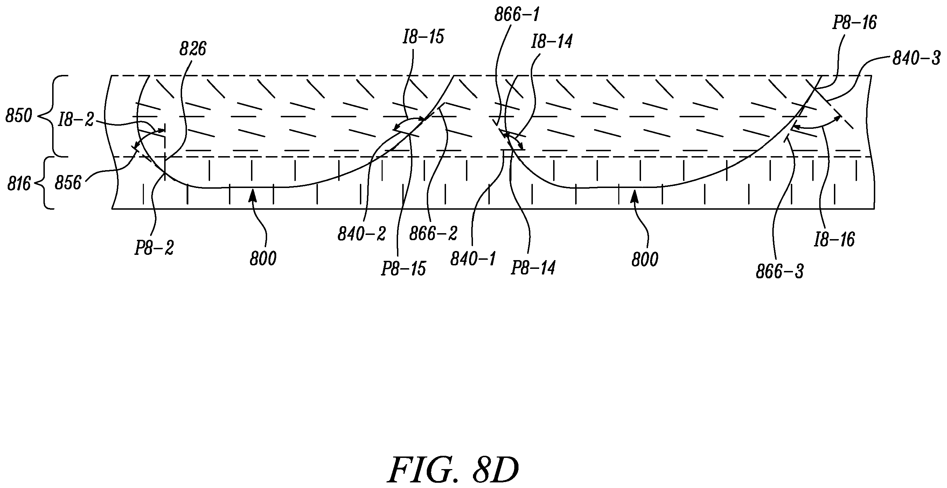

Further, multiple absorbent articles 800 may be cut from the absorbent web 802. For exemplary purposes, only two absorbent articles 800 are illustrated herein. The absorbent article 800 includes a closed perimeter 852 disposed along the third region 818 and extending at least partially into each of the first and second regions 814, 816. The closed perimeter 852 intersects at least some first slits 820 from the plurality of first slits 820 at corresponding first intersection points "P8-1" (shown in FIG. 8B), at least some second slits 826 from the plurality of second slits 826 at corresponding second intersection points "P8-2" (shown in FIG.8D), and at least some third slits 832-1, 832-2, 832-3, 834-1, 834-2, 834-3, 836-1, 836-2, 838-1, 838-2, 838-3, 840-1, 840-2, 840-3 from the plurality of third slits 832-1, 832-2, 832-3, 834-1, 834-2, 834-3, 836-1, 836-2, 838-1, 838-2, 838-3, 840-1, 840-2, 840-3 at corresponding third intersection points "P8-3", "P8-4", "P8-5", "P8-6", "P8-7", "P8-8", "P8-9", "P8-10", "P8-11", "P8-12", "P8-13", "P8-14", "P8-15", "P8-16" (shown in FIGS. 8B to 8D). Therefore, the closed perimeter 834 may cut through at least some of the first slits 820, at least some of the second slits 826, and at least some of the third slits 832-1, 832-2, 832-3, 834-1, 834-2, 834-3, 836-1, 836-2, 838-1, 838-2, 838-3, 840-1, 840-2, 840-3, such that the at least some first slits 820, at least some second slits 826, and at least some third slits 832-1, 832-2, 832-3, 834-1, 834-2, 834-3, 836-1, 836-2, 838-1, 838-2, 838-3, 840-1, 840-2, 840-3 are at partially bounded by the closed perimeter 834. A tangent 854, 856, 858-1, 858-2, 858-3, 860-1, 860-2, 860-3, 862-1, 862-2, 864-1, 864-2, 864-3, 866-1, 866-2, 866-3 (shown in FIGS. 8B to 8D) to the closed perimeter 852 at each of the first, second, and third intersection points "P8-1", "P8-2", "P8-3", "P8-4", "P8-5", "P8-6", "P8-7", "P8-8", "P8-9", "P8-10", "P8-11", "P8-12", "P8-13", "P8-14", "P8-15", "P8-16" forms an angle "I8-1", "I8-2", "I8-3", "I8-4", "I8-5", "I8-6", "I8-7", "18-8", "I8-9", "I8-10", "I8-11", "I8-12", "18-13", "18-14", "I8-15", "18-16" of at least about four degrees with the corresponding first, second, or third slits 820, 826, 832-1, 832-2, 832-3, 834-1, 834-2, 834-3, 836-1, 836-2, 838-1, 838-2, 838-3, 840-1, 840-2, 840-3. Specifically, the tangents 854, 856, 858-1, 858-2, 858-3, 860-1, 860-2, 860-3, 862-1, 862-2, 864-1, 864-2, 864-3, 866-1, 866-2, 866-3 may include a first tangent 854 at the first intersection point "P8-1", a second tangent 856 at the second intersection point "P8-2", third tangents 858-1, 858-2, 858-3 at the respective third intersection points "P8-3", "P8-4", "P8-5", fourth tangents 860-1, 860-2, 860-3 at the respective fourth intersection points "P8-6", "P8-7", "P8-8", fifth tangents 862-1, 862-2 at the respective fifth intersection points "P8-9", "P8-10", sixth tangents 864-1, 864-2, 864-3 at the respective sixth intersection points "P8-11", "P8-12", "P8-13", and seventh tangents 866-1, 866-2, 866-3 at the respective seventh intersection points "P8-14", "P8-15", "P8-16".

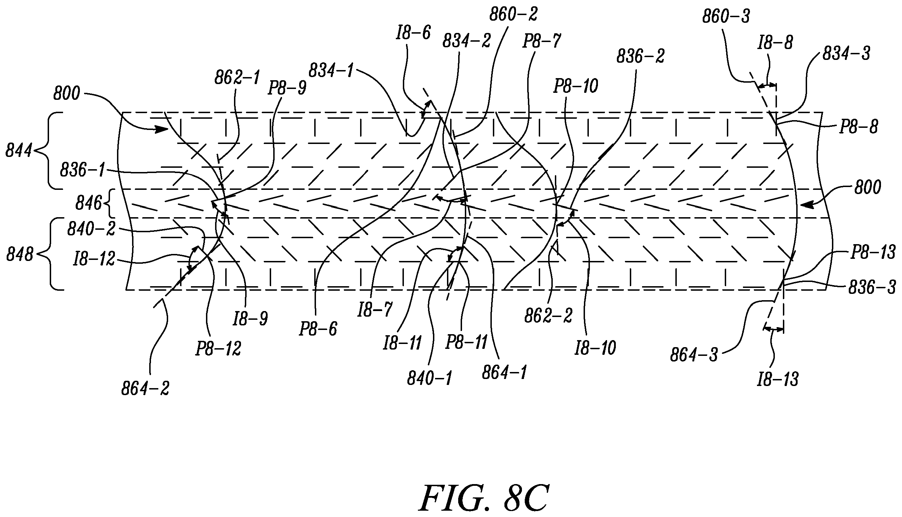

The angles "I8-1", "I8-2", "I8-3", "I8-4", "I8-5", "I8-6", "I8-7", "I8-8", "I8-9", "18-10", "I8-11", "I8-12", "I8-13", "18-14", "I8-15", "18-16" may include a first intersection angle "I8-1", a second intersection angle "I8-2", third intersection angles "I8-3", "I8-4", "I8-5", fourth intersection angles "I8-6", "I8-7", "I8-8", fifth intersection angles "I8-9", "I8-10", sixth intersection angles "18-11", "I8-12", "I8-13", and seventh intersection angles "I8-14", "I8-15", "I8-16". Further, as shown in FIG. 8B, the first intersection angle "I8-1" may be defined between each first slit 820 and the first tangent 854. As shown in FIG. 8D, the second intersection angle "I8-2" may be defined between each second slit 826 and the second tangent 856. As shown in FIG. 8B, the third intersection angle "I8-3" may be defined between each third slit 832-1 and the third tangent 858-1, the third intersection angle "I8-4" may be defined between each third slit 832-2 and the third tangent 858-2, and the third intersection angle "I8-5" may be defined between each third slit 832-3 and the third tangent 858-3. As shown in FIG. 8C, the fourth intersection angle "I8-6" may be defined between each third slit 834-1 and the fourth tangent 860-1, the fourth intersection angle "I8-7" may be defined between each third slit 834-2 and the fourth tangent 860-2, the fourth intersection angle "I8-8" may be defined between each third slit 834-3 and the fourth tangent 860-3, the fifth intersection angle "I8-9" may be defined between each third slit 836-1 and the fifth tangent 862-1, the fifth intersection angle "I8-10" may be defined between each third slit 836-2 and the fifth tangent 862-2, the sixth intersection angle "I8-11" may be defined between each third slit 838-1 and the sixth tangent 864-1, the sixth intersection angle "I8-12" may be defined between each third slit 838-2 and the sixth tangent 864-2, and the sixth intersection angle "I8-13" may be defined between each third slit 838-3 and the sixth tangent 864-3. As shown in FIG. 8D, the seventh intersection angle "I8-14" may be defined between each third slit 840-1 and the seventh tangent 866-1, the seventh intersection angle "I8-15" may be defined between each third slit 840-2 and the seventh tangent 866-2, and the seventh intersection angle "I8-16" may be defined between each third slit 840-3 and the seventh tangent 866-3. Each of the first, second, third, fourth, fifth, sixth, and seventh intersection angles "I8-1", "I8-2", "I8-3", "I8-4", "I8-5", "I8-6", "I8-7", "I8-8", "I8-9", "I8-10", "I8-11", "I8-12", "I8-13", "I8-14", "I8-15", "I8-16" may be greater than four degrees.

In some embodiments, the first intersection angle "I8-1" is at least 10 degrees, at least 20 degrees, at least 30 degrees, or at least 45 degrees, or at least 60 degrees. In some embodiments, the second intersection angle "I8-2" is at least 10 degrees, at least 20 degrees, at least 30 degrees, at least 45 degrees, or at least 60 degrees. In some embodiments, each third intersection angle "I8-3", "I8-4", "I8-5", "I8-6", "I8-7", "I8-8", "I8-9", "18-10", "18-11", "18-12", "18-13", "18-14", "18-15", "18-16" is at least 10 degrees, at least 20 degrees, at least 30 degrees, at least 45 degrees, or at least 60 degrees.

As shown in FIG. 8A, the first slits 820, the second slits 826, and the third slits 832-1, 832-2, 832-3, 834-1, 834-2, 834-3, 836-1, 836-2, 838-1, 838-2, 838-3, 840-1, 840-2, 840-3 may be disposed in such a way relative to the closed perimeter 852, such that none of the first, second, and third slits 820, 826, 832-1, 832-2, 832-3, 834-1, 834-2, 834-3, 836-1, 836-2, 838-1, 838-2, 838-3, 840-1, 840-2, 840-3 are tangential to or aligned with the closed perimeter 852 at the first intersection points "P8-1", the second intersection points "P8-2", and the third intersection points "P8-3", "P8-4", "P8-5", "P8-6", "P8-7", "P8-8", "P8-9", "P8-10", "P8-11", "P8-12", "P8-13", "P8-14", "P8-15", "P8-16", respectively. In the illustrated embodiment of FIG. 8A, the shape of the closed perimeter 852 of the absorbent article 800 includes a kidney shape. The absorbent article 800 is formed from a portion of the absorbent web 802. The absorbent article 800 includes an article body 868. The article body 868 includes the first major surface 806 and the second major surface opposing the first major surface 806. The article body 868 also includes the closed perimeter 852 bounding the first major surface 806 and the second major surface. The article body 868 further includes the plurality of slits 820, 826, 832-1, 832-2, 832-3, 834-1, 834-2, 834-3, 836-1, 836-2, 838-1, 838-2, 838-3, 840-1, 840-2, 840-3 extending from the first major surface 806 to the second major surface. At least some slits 820, 826, 832-1, 832-2, 832-3, 834-1, 834-2, 834-3, 836-1, 836-2, 838-1, 838-2, 838-3, 840-1, 840-2, 840-3 from the plurality of slits 820, 826, 832-1, 832-2, 832-3, 834-1, 834-2, 834-3, 836-1, 836-2, 838-1, 838-2, 838-3, 840-1, 840-2, 840-3 intersect the closed perimeter 852 at corresponding intersection points "P8-1", "P8-2", "P8-3", "P8-4", "P8-5", "P8-6", "P8-7", "P8-8", "P8-9", "P8-10", "P8-11", "P8-12", "P8-13", "P8-14", "P8-15", "P8-16". The tangent 854, 856, 858-1, 858-2, 858-3, 860-1, 860-2, 860-3, 862-1, 862-2, 864-1, 864-2, 864-3, 866-1, 866-2, 866-3 to the closed perimeter 852 at each intersection point "P8-1", "P8-2", "P8-3", "P8-4", "P8-5", "P8-6", "P8-7", "P8-8", "P8-9", "P8-10", "P8-11", "P8-12", "P8-13", "P8-14", "P8-15", "P8-16" forms the angle "I8-1", "I8-2", "I8-3", "I8-4", "I8-5", "I8-6", "I8-7", "I8-8", "I8-9", "I8-10", "I8-11", "I8-12", "I8-13", "I8-14", "18-15", "18-16" of at least about four degrees with the corresponding slit 820, 826, 832-1, 832-2, 832-3, 834-1, 834-2, 834-3, 836-1, 836-2, 838-1, 838-2, 838-3, 840-1, 840-2, 840-3.

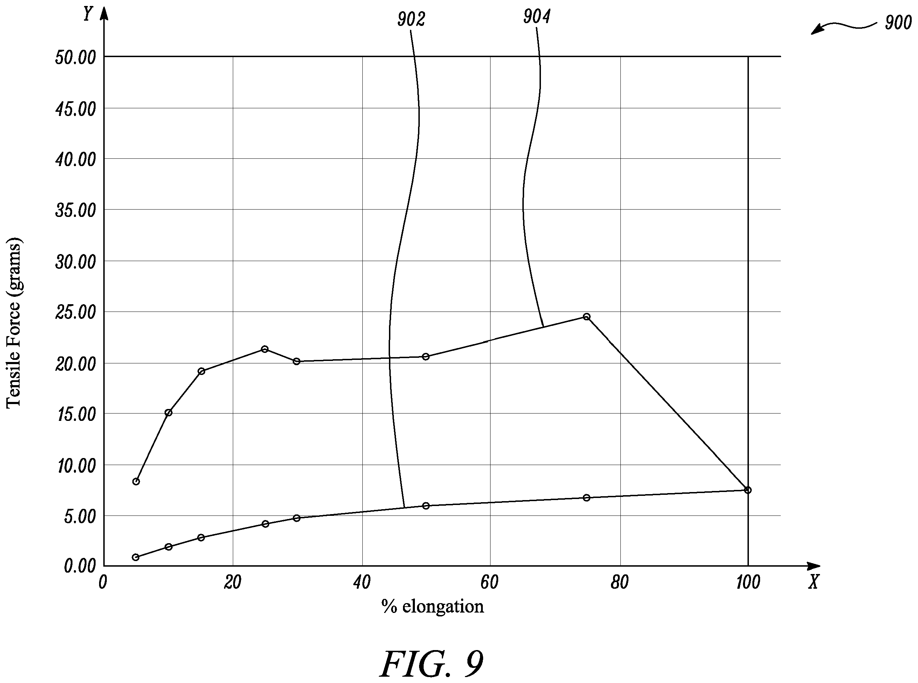

FIG. 9 illustrates an exemplary plot 900. The plot 900 was created based on a stretchability test that was performed on the absorbent article 200 illustrated in FIG. 2B including the slits 220, 226, 232-1, 232-2 (see FIG. 2B) and an absorbent article (not shown). The length "L1-2" (see FIG. 2B) of the slits 220, 226, 232-1, 232-2 was approximately equal to 16 mm. In FIG. 9, various values for elongation in terms of percentage were marked on an X-axis. Further, various values for a tensile force in grams (g) applied on the absorbent article 200 and the absorbent article without slits were marked on the Y-axis. A first curve 902 represents an elongation of the absorbent article 200. A second curve 904 represents an elongation of the absorbent article without slits.

As illustrated from the plot 900, at 20% elongation, the absorbent article 200 may require approximately 4 g of tensile force, whereas the absorbent article without slits may require approximately 20 g of tensile force. At 40% elongation, the absorbent article 200 may require approximately 5 g of tensile force, whereas the absorbent article without slits requires 20 g of tensile force. At 60% elongation, the absorbent article 200 may require approximately 6 g of tensile force, whereas the absorbent article without slits may require approximately 22 g of tensile force. At 80% elongation, the absorbent article 200 may require approximately 7 g of tensile force, whereas the absorbent article without slits may require approximately 21 g of tensile force.

It may be concluded from the plot 900 that the tensile forces required to elongate the absorbent article without slits may be greater than the tensile forces required to elongate the absorbent article 200 with slits 220, 226, 232-1, 232-2. For example, the tensile forces required for 20% elongation of the absorbent article without slits may be 20% to 600% greater than the tensile forces required to elongate the absorbent article 200. The slits 220, 226, 232-1, 232-2 in the absorbent article 200 may also increase the elongation of the absorbent article 200 before breakage by over 15%, and preferably over 50%.

Further, another test was performed to determine an absorbency and a moisture vapor transmission rate (MVTR) of the absorbent article 200 and the absorbent article without slits. The test results demonstrated an increase of about 5% to about 40% in a fluid handling capacity of the absorbent article 200 as compared to the absorbent article without slits. The fluid handling capacity may include absorbency and MVTR.

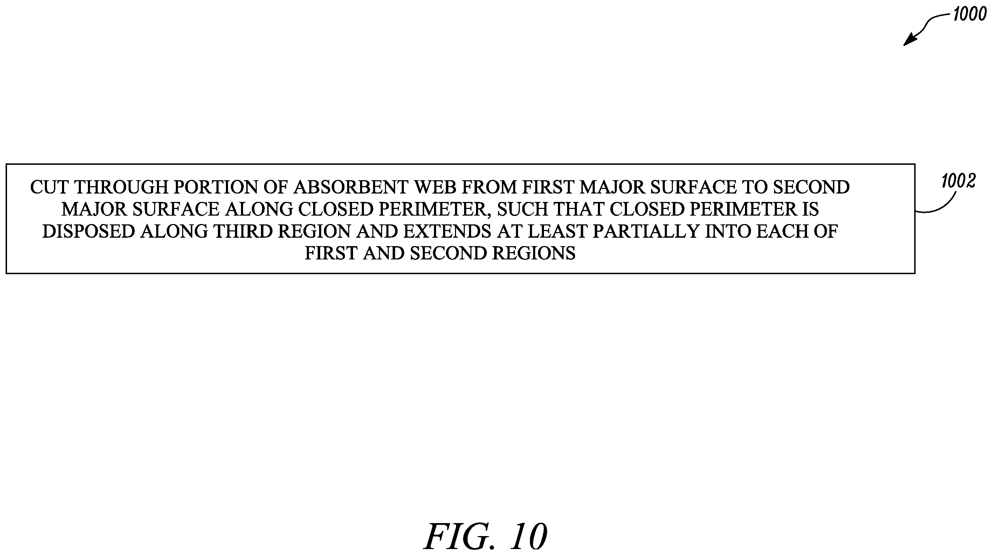

FIG. 10 illustrates a flowchart for a method 1000 of manufacturing the absorbent article 200 from the absorbent web 202. The method 1000 will be explained in relation to the absorbent web 202 and the absorbent article 200 described in relation to FIGS. 1, 2A, and 2B. However, the method 1000 is equally applicable to each absorbent web 302, 402, 502, 602, 702, 802 and each absorbent article 300, 400, 500, 600, 700, 800 described in relation to FIGS. 3 to 8D. At step 1002, a portion of the absorbent web 202 is cut through from the first major surface 206 to the second major surface 208 along the closed perimeter 234, such that the closed perimeter 234 is disposed along the third region 218 and extends at least partially into each of the first and second regions 214, 216. The closed perimeter 234 intersects at least some first slits 220 from the plurality of first slits 220 at corresponding first intersection points "P1-1", at least some second slits 226 from the plurality of second slits 226 at corresponding second intersection points "P1-2", and at least some third slits 232-1, 232-2 from the plurality of third slits 232-1, 232-2 at corresponding third intersection points "P1-3", "P1-4". Further, the tangent 236, 238, 240-1, 240-2 to the closed perimeter 234 at each of the first, second, and third intersection points "P1-1", "P1-2", "P1-3", "P1-4" forms the angle "I1-1", "I1-2", "I1-3", "I1-4" of at least about four degrees with the corresponding first, second, or third slits 220, 226, 232-1, 232-2.

Referring to FIGS. 1, 2A, and 2B, the wound dressing 100 of the present disclosure may include the absorbent article 200 that may be conformable, resilient, and easy to use. Further, the wound dressing 100 may be comfortable to wear and may exhibit improved elongation properties. Furthermore, the slits 220, 226, 232-1, 232-2 of the absorbent web 202 may be oriented in such a way that, when the absorbent article 200 is cut from the absorbent web 202, the closed perimeter 234 of the absorbent article 200 does not align with any of the slits 220, 226, 232-1, 232-2. Specifically, the slits 220, 226, 232-1, 232-2 of the absorbent web 202 may be disposed such that none of the slits 220, 226, 232-1, 232-2 are tangential to the closed perimeter 234 of the absorbent article 200 which may eliminate debris formation in the absorbent article 200, may improve conformability, and may increase elongation of the absorbent article 200 in all directions.

Further, the wound dressing 100 may be used at a contoured sacrum and coccyx area, or at an articulating joint, such as, knee, elbow, and the like. The wound dressing 100 may be used for long injuries, such as, surgical incisions or for non-linear skin traumas as the wound dressing 100 can flex, stretch, or bend adequately during application. When used on skin traumas that traverse non-planar surfaces, such as, long and/or nonlinear skin traumas over bendable surfaces, the wound dressing 100 including the improved absorbent article 200 may exhibit adequate wound dressing conformability. Furthermore, the wound dressing 100 including the improved absorbent article 200 may eliminate a requirement of multiple wound dressings or customized wound dressings as the absorbent article 200 may exhibit improved stretchability and elongation. Moreover, the absorbent article 200 may provide improved coverage and sealing of the injury from contaminants. It should be noted that the description provided above is equally applicable to each absorbent web 302, 402, 502, 602, 702, 802 and each absorbent article 300, 400, 500, 600, 700, 800 described in relation to FIGS. 3 to 8D.

Unless otherwise indicated, all numbers expressing feature sizes, amounts, and physical properties used in the specification and claims are to be understood as being modified by the term "about". Accordingly, unless indicated to the contrary, the numerical parameters set forth in the foregoing specification and attached claims are approximations that can vary depending upon the desired properties sought to be obtained by those skilled in the art utilizing the teachings disclosed herein.

Although specific embodiments have been illustrated and described herein, it will be appreciated by those of ordinary skill in the art that a variety of alternate and/or equivalent implementations can be substituted for the specific embodiments shown and described without departing from the scope of the present disclosure. This application is intended to cover any adaptations or variations of the specific embodiments discussed herein. Therefore, it is intended that this disclosure be limited only by the claims.

## Claims

1. An absorbent web comprising:
a web body (204) defining a machine direction (D1-1)_along a length (L1-1) of the web body (204) and a transverse direction (D1-2) orthogonal to the machine direction (D1-1), the web body (204) comprising:
a first major surface (206) and a second major surface (208) opposing the first major surface (206);
a first longitudinal edge (210) extending along the machine direction (D1-1) and a second longitudinal edge (212) opposing the first longitudinal edge (210), such that each of the first and second major surfaces (206, 208) is disposed between the first and second longitudinal edges (210, 212), the first longitudinal edge (210) and the second longitudinal edge (212) defining a total width of the web body therebetween along the transverse direction (D1-2);
a first region (214) extending from the first longitudinal edge (210) and having a first width (W1-2) along the transverse direction(D1-2) less than the total width of the web body (204), a second region (216) spaced apart from the first region (214) and extending from the second longitudinal edge (212) and having a second width (W1-3) along the transverse direction (D1-2) less than the total width of the web body, and a third region (218) disposed between the first and second regions (214, 216 )and having a third width (W1-4) along the transverse direction (D1-2) less than the total width of the web body (204);
a plurality of first slits (220) extending from the first major surface (206) to the second major surface (208) and disposed in the first region (214) of the web body (204), wherein each first slit (220) is inclined from about 65 degrees to about 115 degrees relative to the machine direction (D1-1);
a plurality of second slits (226) extending from the first major surface (206) to the second major surface (208) and disposed in the second region (216) of the web body (204), wherein each second slit (226) is inclined from about 65 degrees to about 115 degrees relative to the machine direction (D1-1); and
a plurality of third slits (231-1, 232-2) extending from the first major surface (206) to the second major surface (208) and disposed in the third region (218) of the web body (204), wherein at least some third slits from the plurality of third slits (231-1, 232-2) are substantially parallel to or inclined obliquely to the machine direction (D1-1), and wherein at least some third slits from the plurality of third slits (231-1, 232-2) are inclined to each first slit (220) and each second slit (226).

2. The absorbent web of claim 1, wherein each third slit (231-1, 232-2) is substantially parallel to or inclined obliquely to the machine direction (D1-1).

3. The absorbent web of claim 1, wherein at least some other third slits from the plurality of third slits (231-1, 232-2) are substantially parallel to each first slit (220) and each second slit (226).

4. The absorbent web of claim 1, wherein each third slit (231-1, 232-2) is inclined to each first slit (220) and each second slit (226).

5. The absorbent web of claim 1, wherein each first slit (220) and/or each second slit (226) is substantially parallel to the transverse direction (D1-2).

6. The absorbent web of claim 1, wherein the first slits (220) are substantially parallel to each other, and/or the second slits (226) are substantially parallel to each other.

7. The absorbent web of claim 1, wherein at least some third slits from the plurality of third slits (231-1, 232-2) are substantially parallel to the machine direction (D1-1), inclined from about 85 degrees to about 265 degrees relative to the machine direction (D1-1), substantially orthogonal to at least some other third slits from the plurality of third slits (231-1, 232-2), or inclined obliquely to at least some other third slits from the plurality of third slits (231-1, 232-2).

8. The absorbent web of claim 1, wherein at least some third slits from the plurality of third slits (231-1, 232-2) are substantially parallel to the machine direction (D1-1), wherein at least some third slits from the plurality of third slits (231-1, 232-2) are inclined at an acute angle relative to the machine direction (D1-1), and wherein at least some third slits from the plurality of third slits (231-1, 232-2) are inclined at an obtuse angle relative to the machine direction (D1-1).

9. The absorbent web of claim 1, wherein the third region comprises:
a first subregion (742) disposed adjacent to the first region (214), wherein at least some third slits from the plurality of third slits (231-1, 232-2) disposed in the first subregion (742) are substantially parallel to the machine direction (D1-1), and wherein at least some third slits from the plurality of third slits (231-1, 232-2) disposed in the first subregion (742) are inclined from about 10 degrees to about 20 degrees relative to the machine direction (D1-1);
a second subregion (744) disposed adjacent to the first subregion (742) opposite to the first region (214), wherein at least some third slits from the plurality of third slits (231-1, 232-2) disposed in the second subregion (744) are substantially parallel to the machine direction (D1-1), and wherein at least some third slits from the plurality of third slits (231-1, 232-2) disposed in the second subregion (744) are inclined from about 40 degrees to about 70 degrees relative to the machine direction (D1-1);
a third subregion (746) disposed adjacent to the second subregion (744) opposite to the first subregion (742), wherein at least some third slits from the plurality of third slits (231-1, 232-2) disposed in the third subregion (746) are inclined from about 10 degrees to about 20 degrees relative to the machine direction (D1-1), and wherein at least some third slits from the plurality of third slits (231-1, 232-2) disposed in the third subregion (746) are inclined from about 160 degrees to about 170 degrees relative to the machine direction (D1-1);
a fourth subregion (748) disposed adjacent to the third subregion (746) opposite to the second region (216), wherein at least some third slits from the plurality of third slits (231-1, 232-2) disposed in the fourth subregion (748) are substantially parallel to the machine direction (D1-1), and wherein at least some third slits from the plurality of third slits (231-1, 232-2) disposed in the fourth subregion (748) are inclined from about 110 degrees to about 140 degrees relative to the machine direction (D1-1); and
a fifth subregion (750) disposed between the fourth subregion (748) and the second region (744), wherein at least some third slits from the plurality of third slits (231-1, 232-2) disposed in the fifth subregion (750) are substantially parallel to the machine direction (D1-1), and wherein at least some third slits from the plurality of third slits (231-1, 232-2) disposed in the fifth subregion (750) are inclined from about 160 degrees to about 170 degrees relative to the machine direction (D1-1).

10. The absorbent web of claim 1, wherein third width is greater than each of the first and second widths by a factor of at least two, or the third width is less than each of the first and second widths.

11. The absorbent web of claim 1, wherein each of the plurality of first (220), second (226), and third slits (231-1, 232-2) comprises a slit length from about 3 mm to about 25 mm.

12. An absorbent article formed from a portion of the absorbent web of claim 1, the absorbent article (200) extending from the first major surface (206) to the second major surface (208), the absorbent article (200) comprising a closed perimeter (234) disposed along the third region (218) and extending at least partially into each of the first (214) and second regions (216), wherein the closed perimeter (234) intersects at least some first slits from the plurality of first slits (220) at corresponding first intersection points (P1-1), at least some second slits from the plurality of second slits (226) at corresponding second intersection points (P1-2), and at least some third slits from the plurality of third slits (232-1, 232-2) at corresponding third intersections points (P1-3, P1-4), and wherein a tangent (236, 238, 240-1, 240-2) to the closed perimeter (234) at each of the first, second, and third intersection points (P1-1, P1-2, P1-3, P1-4) forms an angle (I1-1, I1-2, I1-3, I1-4) of at least about four degrees with the corresponding first, second, or third slits (220, 226, 232-1, 232-2).

13. A wound dressing comprising the absorbent article of claim 12.

14. A method of manufacturing an absorbent article from the absorbent web (202) of claim 1, the method comprising cutting through a portion of the absorbent web (202) from the first major surface (206) to the second major surface (208) along a closed perimeter (234), such that the closed perimeter (234) is disposed along the third region (218) and extends at least partially into each of the first (214) and second regions (216), wherein the closed perimeter (234) intersects at least some first slits from the plurality of first slits (220) at corresponding first intersection points (P1-1), at least some second slits from the plurality of second slits (226) at corresponding second intersection points (P1-2), and at least some third slits from the plurality of third slits (232-1, 232-2) at corresponding third intersections points (P1-2, P1-4), and wherein a tangent (236, 238, 240-1, 240-2) to the closed perimeter (234) at each of the first, second, and third intersection points (P1-1, P1-2, P1-3, P1-4) forms an angle of at least about four degrees with the corresponding first, second, or third slits (220, 226, 232-1, 232-2).

15. An absorbent article comprising:
an article body (242) comprising:
a first major surface (206) and a second major surface (208) opposing the first major surface (206);
a closed perimeter (234) bounding the first and second major surfaces (206, 208); and
a plurality of slits (220, 226, 232-1, 232-2) extending from the first major surface (206) to the second major surface (208), wherein at least some slits from the plurality of slits (220, 226, 232-1, 232-2) intersect the closed perimeter (234) at corresponding intersection points (P1-1, P1-2, P1-3, P1-4), and wherein a tangent (236, 238, 240-1, 240-2) to the closed perimeter (2340 at each intersection point (P1-1, P1-2, P1-3, P1-4) forms an angle of at least about four degrees with the corresponding slit.

## Patentansprüche

1. Eine absorbierende Bahn, aufweisend:
ein Bahnkörper (204), der eine Maschinenrichtung (D1-1) entlang einer Länge (L1-1) des Bahnkörpers (204) und einer Querrichtung (D1-2) orthogonal zu der Maschinenrichtung (D1-1) definiert, der Bahnkörper (204) aufweisend:
eine erste Hauptoberfläche (206) und eine zweite Hauptoberfläche (208), die der ersten Hauptoberfläche (206) gegenüberliegt;
eine erste Längskante (210), die sich entlang der Maschinenrichtung (D1-1) erstreckt, und eine zweite Längskante (212), die der ersten Längskante (210) gegenüberliegt, derart, dass jede der ersten und der zweiten Hauptoberfläche (206, 208) zwischen der ersten und der zweiten Längskante (210, 212) angeordnet ist, wobei die erste Längskante (210) und die zweite Längskante (212) eine Gesamtbreite des Bahnkörpers dazwischen entlang der Querrichtung (D1-2) definieren;
einen ersten Bereich (214), der sich von der ersten Längskante (210) erstreckt und eine erste Breite (W1-2) entlang der Querrichtung (D1-2) aufweist, die geringer als die Gesamtbreite des Bahnkörpers (204) ist, einen zweiten Bereich (216), der von dem ersten Bereich (214) beabstandet ist und sich von der zweiten Längskante (212) erstreckt und eine zweite Breite (W1-3) entlang der Querrichtung (D1-2) aufweist, die geringer als die Gesamtbreite des Bahnkörpers ist, und einen dritten Bereich (218), der zwischen dem ersten und dem zweiten Bereich (214, 216) angeordnet ist und eine dritte Breite (W1-4) entlang der Querrichtung (D1-2) aufweist, die geringer als die Gesamtbreite des Bahnkörpers (204) ist;
eine Mehrzahl von ersten Schlitzen (220), die sich von der ersten Hauptoberfläche (206) zu der zweiten Hauptoberfläche (208) erstrecken und in dem ersten Bereich (214) des Bahnkörpers (204) angeordnet sind, wobei jeder erste Schlitz (220) von etwa 65 Grad bis etwa 115 Grad relativ zu der Maschinenrichtung (D1-1) geneigt ist;
eine Mehrzahl von zweiten Schlitzen (226), die sich von der ersten Hauptoberfläche (206) zu der zweiten Hauptoberfläche (208) erstrecken und in dem zweiten Bereich (216) des Bahnkörpers (204) angeordnet sind, wobei jeder zweite Schlitz (226) von etwa 65 Grad bis etwa 115 Grad relativ zu der Maschinenrichtung (D1-1) geneigt ist; und
eine Mehrzahl von dritten Schlitzen (231-1, 232-2), die sich von der ersten Hauptoberfläche (206) zu der zweiten Hauptoberfläche (208) erstrecken und in dem dritten Bereich (218) des Bahnkörpers (204) angeordnet sind, wobei mindestens einige dritte Schlitze aus der Mehrzahl von dritten Schlitzen (231-1, 232-2) im Wesentlichen parallel zu oder schräg geneigt zu der Maschinenrichtung (D1-1) sind, und wobei mindestens einige dritte Schlitze aus der Mehrzahl von dritten Schlitzen (231-1, 232-2) zu jedem ersten Schlitz (220) und jedem zweiten Schlitz (226) geneigt sind.

2. Die absorbierende Bahn nach Anspruch 1, wobei jeder dritte Schlitz (231-1, 232-2) im Wesentlichen parallel zu oder schräg geneigt zu der Maschinenrichtung (D1-1) ist.

3. Die absorbierende Bahn nach Anspruch 1, wobei mindestens einige andere dritte Schlitze aus der Mehrzahl von dritten Schlitzen (231-1, 232-2) im Wesentlichen parallel zu jedem ersten Schlitz (220) und jedem zweiten Schlitz (226) sind.

4. Die absorbierende Bahn nach Anspruch 1, wobei jeder dritte Schlitz (231-1, 232-2) zu jedem ersten Schlitz (220) und jedem zweiten Schlitz (226) geneigt ist.

5. Die absorbierende Bahn nach Anspruch 1, wobei jeder erste Schlitz (220) und/oder jeder zweite Schlitz (226) im Wesentlichen parallel zu der Querrichtung (D1-2) ist.

6. Die absorbierende Bahn nach Anspruch 1, wobei die ersten Schlitze (220) im Wesentlichen parallel zueinander sind, und/oder die zweiten Schlitze (226) im Wesentlichen parallel zueinander sind.

7. Die absorbierende Bahn nach Anspruch 1, wobei mindestens einige dritte Schlitze aus der Mehrzahl von dritten Schlitzen (231-1, 232-2) im Wesentlichen parallel zu der Maschinenrichtung (D1-1) sind, von etwa 85 Grad bis etwa 265 Grad relativ zu der Maschinenrichtung (D1-1) geneigt sind, im Wesentlichen orthogonal zu mindestens einigen anderen dritten Schlitzen aus der Mehrzahl von dritten Schlitzen (231-1, 232-2) sind, oder zu mindestens einigen anderen dritten Schlitzen aus der Mehrzahl von dritten Schlitzen (231-1, 232-2) schräg geneigt sind.

8. Die absorbierende Bahn nach Anspruch 1, wobei mindestens einige dritte Schlitze aus der Mehrzahl von dritten Schlitzen (231-1, 232-2) im Wesentlichen parallel zu der Maschinenrichtung (D1-1) sind, wobei mindestens einige dritte Schlitze aus der Mehrzahl von dritten Schlitzen (231-1, 232-2) in einem spitzen Winkel relativ zu der Maschinenrichtung (D1-1) geneigt sind, und wobei mindestens einige dritte Schlitze aus der Mehrzahl von dritten Schlitzen (231-1, 232-2) in einem stumpfen Winkel relativ zu der Maschinenrichtung (D1-1) geneigt sind.

9. Die absorbierende Bahn nach Anspruch 1, wobei der dritte Bereich aufweist:
einen ersten Teilbereich (742), der benachbart zu dem ersten Bereich (214) angeordnet ist, wobei mindestens einige dritte Schlitze aus der Mehrzahl von dritten Schlitzen (231-1, 232-2), die in dem ersten Teilbereich (742) angeordnet sind, im Wesentlichen parallel zu der Maschinenrichtung (D1-1) sind, und wobei mindestens einige dritte Schlitze aus der Mehrzahl von dritten Schlitzen (231-1, 232-2), die in dem ersten Teilbereich (742) angeordnet sind, von etwa 10 Grad bis etwa 20 Grad relativ zu der Maschinenrichtung (D1-1) geneigt sind;
einen zweiten Teilbereich (744), der benachbart zu dem ersten Teilbereich (742) gegenüber dem ersten Bereich (214) angeordnet ist, wobei mindestens einige dritte Schlitze aus der Mehrzahl von dritten Schlitzen (231-1, 232-2), die in dem zweiten Teilbereich (744) angeordnet sind, im Wesentlichen parallel zu der Maschinenrichtung (D1-1) sind, und wobei mindestens einige dritte Schlitze aus der Mehrzahl von dritten Schlitzen (231-1, 232-2), die in dem zweiten Teilbereich (744) angeordnet sind, von etwa 40 Grad bis etwa 70 Grad relativ zu der Maschinenrichtung (D1-1) geneigt sind;
einen dritten Teilbereich (746), der benachbart zu dem zweiten Teilbereich (744) gegenüber dem ersten Teilbereich (742) angeordnet ist, wobei mindestens einige dritte Schlitze aus der Mehrzahl von dritten Schlitzen (231-1, 232-2), die in dem dritten Teilbereich (746) angeordnet sind, von etwa 10 Grad bis etwa 20 Grad relativ zu der Maschinenrichtung (D1-1) geneigt sind, und wobei mindestens einige dritte Schlitze aus der Mehrzahl von dritten Schlitzen (231-1, 232-2), die in dem dritten Teilbereich (746) angeordnet sind, von etwa 160 Grad bis etwa 170 Grad relativ zu der Maschinenrichtung (D1-1) geneigt sind;
einen vierten Teilbereich (748), der angrenzend an den dritten Teilbereich (746) gegenüber dem zweiten Bereich (216) angeordnet ist, wobei mindestens einige dritte Schlitze aus der Mehrzahl von dritten Schlitzen (231-1, 232-2), die in dem vierten Teilbereich (748) angeordnet sind, im Wesentlichen parallel zu der Maschinenrichtung (D1-1) sind, und
wobei mindestens einige dritte Schlitze aus der Mehrzahl von dritten Schlitzen (231-1, 232-2), die in dem vierten Teilbereich (748) angeordnet sind, von etwa 110 Grad bis etwa 140 Grad relativ zu der Maschinenrichtung (D1-1) geneigt sind; und
einen fünften Teilbereich (750), der zwischen dem vierten Teilbereich (748) und dem zweiten Bereich (744) angeordnet ist, wobei mindestens einige dritte Schlitze aus der Mehrzahl von dritten Schlitzen (231-1, 232-2), die in dem fünften Teilbereich (750) angeordnet sind, im Wesentlichen parallel zu der Maschinenrichtung (D1-1) sind, und wobei mindestens einige dritte Schlitze aus der Mehrzahl von dritten Schlitzen (231-1, 232-2), die in dem fünften Teilbereich (750) angeordnet sind, von etwa 160 Grad bis etwa 170 Grad relativ zu der Maschinenrichtung (D1-1) geneigt sind.

10. Die absorbierende Bahn nach Anspruch 1, wobei die dritte Breite größer als jede der ersten und der zweiten Breite um einen Faktor von mindestens zwei ist, oder die dritte Breite kleiner als jede der ersten und der zweiten Breite ist.

11. Die absorbierende Bahn nach Anspruch 1, wobei jeder der Mehrzahl von ersten (220), zweiten (226) und dritten Schlitzen (231-1, 232-2) eine Schlitzlänge von etwa 3 mm bis etwa 25 mm aufweist.

12. Ein absorbierender Artikel, der aus einem Abschnitt der absorbierenden Bahn nach Anspruch 1 ausgebildet ist, wobei sich der absorbierende Artikel (200) von der ersten Hauptoberfläche (206) zu der zweiten Hauptoberfläche (208) erstreckt, der absorbierende Artikel (200) aufweisend einen geschlossenen Umfang (234), der entlang des dritten Bereichs (218) angeordnet ist und sich mindestens teilweise in jedem des ersten (214) und des zweiten Bereichs (216) erstreckt, wobei der geschlossene Umfang (234) mindestens einige erste Schlitze aus der Mehrzahl von ersten Schlitzen (220) an entsprechenden ersten Schnittpunkten (P1-1), mindestens einige zweite Schlitze aus der Mehrzahl von zweiten Schlitzen (226) an entsprechenden zweiten Schnittpunkten (P1-2) und mindestens einige dritte Schlitze aus der Mehrzahl von dritten Schlitzen (232-1, 232-2) an entsprechenden dritten Schnittpunkten (P1-3, P1-4) schneidet, und wobei eine Tangente (236, 238, 240-1, 240-2) an dem geschlossenen Umfang (234) an jedem der ersten, zweiten und dritten Schnittpunkte (P1-1, P1-2, P1-3, P1-4) einen Winkel (11-1, 11-2, 11-3, 11-4) von mindestens etwa vier Grad mit den entsprechenden ersten, zweiten oder dritten Schlitzen (220, 226, 232-1, 232-2) ausbildet.

13. Ein Wundverband, aufweisend den absorbierenden Artikel nach Anspruch 12.

14. Ein Verfahren zum Herstellen eines absorbierenden Artikels aus der absorbierenden Bahn (202) von Anspruch 1, das Verfahren aufweisend ein Schneiden durch einen Abschnitt der absorbierenden Bahn (202) von der ersten Hauptoberfläche (206) zu der zweiten Hauptoberfläche (208) entlang eines geschlossenen Umfangs (234), derart, dass der geschlossene Umfang (234) entlang des dritten Bereichs (218) angeordnet ist und sich mindestens teilweise in jeden des ersten (214) und des zweiten Bereichs (216) erstreckt, wobei der geschlossene Umfang (234) mindestens einige erste Schlitze aus der Mehrzahl von ersten Schlitzen (220) an entsprechenden ersten Schnittpunkten (P1-1), mindestens einige zweite Schlitze aus der Mehrzahl von zweiten Schlitzen (226) an entsprechenden zweiten Schnittpunkten (P1-2) und mindestens einige dritte Schlitze aus der Mehrzahl von dritten Schlitzen (232-1, 232-2) an entsprechenden dritten Schnittpunkten (P1-2, P1-4) schneidet, und wobei eine Tangente (236, 238, 240-1, 240-2) an dem geschlossenen Umfang (234) an jedem der ersten, zweiten und dritten Schnittpunkte (P1-1, P1-2, P1-3, P1-4) einen Winkel von mindestens etwa vier Grad mit den entsprechenden ersten, zweiten oder dritten Schlitzen (220, 226, 232-1, 232-2) ausbildet.

15. Ein absorbierender Artikel, aufweisend:
einen Artikelkörper (242), aufweisend:
eine erste Hauptoberfläche (206) und eine zweite Hauptoberfläche (208), die der ersten Hauptoberfläche (206) gegenüberliegt;
einen geschlossenen Umfang (234), der die erste und die zweite Hauptoberfläche (206, 208) begrenzt; und
eine Mehrzahl von Schlitzen (220, 226, 232-1, 232-2), die sich von der ersten Hauptoberfläche (206) zu der zweiten Hauptoberfläche (208) erstrecken, wobei mindestens einige Schlitze aus der Mehrzahl von Schlitzen (220, 226, 232-1, 232-2) den geschlossenen Umfang (234) an entsprechenden Schnittpunkten (P1-1, P1-2, P1-3, P1-4) schneiden, und wobei eine Tangente (236, 238, 240-1, 240-2) an dem geschlossenen Umfang (2340 an jedem Schnittpunkt (P1-1, P1-2, P1-3, P1-4) einen Winkel von mindestens etwa vier Grad mit dem entsprechenden Schlitz ausbildet.

## Revendications

1. Bande absorbante comprenant :
un corps de bande (204) définissant une direction machine (D1-1) le long d'une longueur (L1-1) du corps de bande (204) et une direction transversale (D1-2) orthogonale à la direction machine (D1-1), le corps de bande (204) comprenant :
une première surface principale (206) et une seconde surface principale (208) à l'opposée de la première surface principale (206) ;
un premier bord longitudinal (210) s'étendant le long de la direction machine (D1-1) et un second bord longitudinal (212) à l'opposé du premier bord longitudinal (210), de telle sorte que chacune des première et seconde surfaces principales (206, 208) est disposée entre les premier et second bords longitudinaux (210, 212), le premier bord longitudinal (210) et le second bord longitudinal (212) définissant une largeur totale du corps de bande entre eux le long de la direction transversale (D1-2) ;
une première région (214) s'étendant à partir du premier bord longitudinal (210) et ayant une première largeur (W1-2) le long de la direction transversale (D1-2) inférieure à la largeur totale du corps de bande (204), une deuxième région (216) espacée de la première région (214) et s'étendant à partir du second bord longitudinal (212) et ayant une deuxième largeur (W1-3) le long de la direction transversale (D1-2) inférieure à la largeur totale du corps de bande, et une troisième région (218) disposée entre les première et deuxième régions (214, 216) et ayant une troisième largeur (W1-4) le long de la direction transversale (D1-2) inférieure à la largeur totale du corps de bande (204) ;
une pluralité de premières fentes (220) s'étendant à partir de la première surface principale (206) jusqu'à la seconde surface principale (208) et disposées dans la première région (214) du corps de bande (204), dans laquelle chaque première fente (220) est inclinée d'environ 65 degrés à environ 115 degrés par rapport à la direction machine (D1-1) ;
une pluralité de deuxièmes fentes (226) s'étendant à partir de la première surface principale (206) jusqu'à la seconde surface principale (208) et disposées dans la deuxième région (216) du corps de bande (204), dans laquelle chaque deuxième fente (226) est inclinée d'environ 65 degrés à environ 115 degrés par rapport à la direction machine (D1-1) ; et
une pluralité de troisièmes fentes (231-1, 232-2) s'étendant à partir de la première surface principale (206) jusqu'à la seconde surface principale (208) et disposées dans la troisième région (218) du corps de bande (204), dans laquelle au moins certaines des troisièmes fentes de la pluralité de troisièmes fentes (231-1, 232-2) sont sensiblement parallèles à ou inclinées obliquement par rapport à la direction machine (DI-1), et dans laquelle au moins certaines des troisièmes fentes de la pluralité de troisièmes fentes (231-1, 232-2) sont inclinées par rapport à chaque première fente (220) et à chaque deuxième fente (226).

2. Bande absorbante selon la revendication 1, dans laquelle chaque troisième fente (231-1, 232-2) est sensiblement parallèle à ou inclinée obliquement par rapport à la direction machine (DI-1).

3. Bande absorbante selon la revendication 1, dans laquelle au moins certaines des autres troisièmes fentes de la pluralité de troisièmes fentes (231-1, 232-2) sont sensiblement parallèles à chaque première fente (220) et à chaque deuxième fente (226).

4. Bande absorbante selon la revendication 1, dans laquelle chaque troisième fente (231-1, 232-2) est inclinée par rapport à chaque première fente (220) et à chaque deuxième fente (226).

5. Bande absorbante selon la revendication 1, dans laquelle chaque première fente (220) et/ou chaque deuxième fente (226) sont sensiblement parallèles à la direction transversale (D1-2).

6. Bande absorbante selon la revendication 1, dans laquelle les premières fentes (220) sont sensiblement parallèles les unes aux autres, et/ou les deuxièmes fentes (226) sont sensiblement parallèles les unes aux autres.

7. Bande absorbante selon la revendication 1, dans laquelle au moins certaines des troisièmes fentes de la pluralité de troisièmes fentes (231-1, 232-2) sont sensiblement parallèles à la direction machine (D1-1), inclinées d'environ 85 degrés à environ 265 degrés par rapport à la direction machine (D1-1), sensiblement orthogonales à au moins certaines des autres troisièmes fentes de la pluralité de troisièmes fentes (231-1, 232-2), ou inclinées obliquement par rapport à au moins certaines des autres troisièmes fentes parmi la pluralité de troisièmes fentes (231-1, 232-2).

8. Bande absorbante selon la revendication 1, dans laquelle au moins certaines des troisièmes fentes de la pluralité de troisièmes fentes (231-1, 232-2) sont sensiblement parallèles à la direction machine (D1-1), dans laquelle au moins certaines des troisièmes fentes de la pluralité de troisièmes fentes (231-1, 232-2) sont inclinées selon un angle aigu par rapport à la direction machine (D1-1), et dans laquelle au moins certaines des troisièmes fentes de la pluralité de troisièmes fentes (231-1, 232-2) sont inclinées selon un angle obtus par rapport à la direction machine (D1-1).

9. Bande absorbante selon la revendication 1, dans laquelle la troisième région comprend :
une première sous-région (742) disposée à côté de la première région (214), dans laquelle au moins certaines des troisièmes fentes de la pluralité de troisièmes fentes (231-1, 232-2) disposées dans la première sous-région (742) sont sensiblement parallèles à la direction machine (D1-1), et dans laquelle au moins certaines des troisièmes fentes de la pluralité de troisièmes fentes (231-1, 232-2) disposées dans la première sous-région (742) sont inclinées d'environ 10 degrés à environ 20 degrés par rapport à la direction machine (D1-1) ;
une deuxième sous-région (744) disposée à côté de la première sous-région (742) à l'opposée de la première région (214), dans laquelle au moins certaines des troisièmes fentes de la pluralité de troisièmes fentes (231-1, 232-2) disposées dans la deuxième sous-région (744) sont sensiblement parallèles à la direction machine (D1-1), et dans laquelle au moins certaines des troisièmes fentes de la pluralité de troisièmes fentes (231-1, 232-2) disposées dans la deuxième sous-région (744) sont inclinées d'environ 40 degrés à environ 70 degrés par rapport à la direction machine (D1-1) ;
une troisième sous-région (746) disposée à côté de la deuxième sous-région (744) à l'opposée de la première sous-région (742), dans laquelle au moins certaines des troisièmes fentes de la pluralité de troisièmes fentes (231-1, 232-2) disposées dans la troisième sous-région (746) sont inclinées d'environ 10 degrés à environ 20 degrés par rapport à la direction machine (D1-1), et dans laquelle au moins certaines des troisièmes fentes de la pluralité de troisièmes fentes (231-1, 232-2) disposées dans la troisième sous-région (746) sont inclinées d'environ 160 degrés à environ 170 degrés par rapport à la direction machine (D1-1) ;
une quatrième sous-région (748) disposée à côté de la troisième sous-région (746) à l'opposé de la deuxième région (216), dans laquelle au moins certaines des troisièmes fentes de la pluralité de troisièmes fentes (231-1, 232-2) disposées dans la quatrième sous-région (748) sont sensiblement parallèles à la direction machine (D1-1), et
dans laquelle au moins certaines des troisièmes fentes de la pluralité de troisièmes fentes (231-1, 232-2) disposées dans la quatrième sous-région (748) sont inclinées d'environ 110 degrés à environ 140 degrés par rapport à la direction machine (D1-1) ; et
une cinquième sous-région (750) disposée entre la quatrième sous-région (748) et la deuxième région (744), dans laquelle au moins certaines des troisièmes fentes de la pluralité de troisièmes fentes (231-1, 232-2) disposées dans la cinquième sous-région (750) sont sensiblement parallèles à la direction machine (D1-1), et dans laquelle au moins certaines des troisièmes fentes de la pluralité de troisièmes fentes (231-1, 232-2) disposées dans la cinquième sous-région (750) sont inclinées d'environ 160 degrés à environ 170 degrés par rapport à la direction machine (D1-1).

10. Bande absorbante selon la revendication 1, dans laquelle la troisième largeur est supérieure à chacune des première et deuxième largeurs d'un facteur d'au moins deux, ou la troisième largeur est inférieure à chacune des première et deuxième largeurs.

11. Bande absorbante selon la revendication 1, dans laquelle chacune de la pluralité de premières (220), deuxièmes (226) et troisièmes fentes (231-1, 232-2) comprend une longueur de fente d'environ 3 mm à environ 25 mm.

12. Article absorbant formé à partir d'une partie de la bande absorbante selon la revendication 1, l'article absorbant (200) s'étendant à partir de la première surface principale (206) jusqu'à la seconde surface principale (208), l'article absorbant (200) comprenant un périmètre fermé (234) disposé le long de la troisième région (218) et s'étendant au moins partiellement dans chacune des première (214) et deuxième régions (216), dans lequel le périmètre fermé (234) croise au moins certaines des premières fentes de la pluralité de premières fentes (220) au niveau de premiers points d'intersection (P1-1) correspondants, au moins certaines des deuxièmes fentes de la pluralité de deuxièmes fentes (226) au niveau de deuxièmes points d'intersection (P1-2) correspondants, et au moins certaines des troisièmes fentes de la pluralité de troisièmes fentes (232-1, 232-2) au niveau de troisièmes points d'intersection (P1-3, P1-4) correspondants, et dans lequel une tangente (236, 238, 240-1, 240-2) au périmètre fermé (234) au niveau de chacun des premiers, deuxièmes et troisièmes points d'intersection (P1-1, P1-2, P1-3, P1-4) forme un angle (11-1, 11-2, 11-3, 11-4) d'au moins environ quatre degrés avec les premières, deuxièmes ou troisièmes fentes (220, 226, 232-1, 232-2) correspondantes.

13. Pansement comprenant l'article absorbant selon la revendication 12.

14. Procédé de fabrication d'un article absorbant à partir de la bande absorbante (202) selon la revendication 1, le procédé comprenant la coupe à travers une partie de la bande absorbante (202) à partir de la première surface principale (206) jusqu'à la seconde surface principale (208) le long d'un périmètre fermé (234), de telle sorte que le périmètre fermé (234) est disposé le long de la troisième région (218) et s'étend au moins partiellement dans chacune des première (214) et deuxième régions (216), dans lequel le périmètre fermé (234) croise au moins certaines des premières fentes de la pluralité de premières fentes (220) au niveau de premiers points d'intersection (P1-1) correspondants, au moins certaines des deuxièmes fentes de la pluralité de deuxièmes fentes (226) au niveau de deuxièmes points d'intersection (P1-2) correspondants, et au moins certaines des troisièmes fentes de la pluralité de troisièmes fentes (232-1, 232-2) au niveau de troisièmes points d'intersection (P1-2, P1-4) correspondants, et dans lequel une tangente (236, 238, 240-1, 240-2) au périmètre fermé (234) au niveau de chacun des premiers, deuxièmes et troisièmes points d'intersection (P1-1, P1-2, P1-3, P1-4) forme un angle d'au moins environ quatre degrés avec les premières, deuxièmes ou troisièmes fentes (220, 226, 232-1, 232-2) correspondantes.

15. Article absorbant comprenant :
un corps d'article (242) comprenant :
une première surface principale (206) et une seconde surface principale (208) à l'opposée de la première surface principale (206) ;
un périmètre fermé (234) délimitant les première et seconde surfaces principales (206, 208) ; et
une pluralité de fentes (220, 226, 232-1, 232-2) s'étendant à partir de la première surface principale (206) jusqu'à la seconde surface principale (208), dans lequel au moins certaines fentes de la pluralité de fentes (220, 226, 232-1, 232-2) croisent le périmètre fermé (234) au niveau de points d'intersection (P1-1, P1-2, P1-3, P1-4) correspondants, et dans lequel une tangente (236, 238, 240-1, 240-2) au périmètre fermé (2340) au niveau de chaque point d'intersection (P1-1, P1-2, P1-3, P1-4) forme un angle d'au moins environ quatre degrés avec la fente correspondante.
